# EUROPEAN PATENT APPLICATION

(11) **EP 0 846 670 A1**
(43) Date of publication of application: **10.06.1998**
(21) Application number: 96926607.1
(22) Date of filing: 08.08.1996
(51) Int. Cl.: C07C 22/08, C07C 25/18, C07C 25/24, C07C 43/174, C07C 43/176, C07C 43/192, C07C 43/225, C07C 69/75, C07C 69/773, C07C 255/50, C07C 255/54

(54) **CYCLOHEXANE DERIVATIVES AND LIQUID-CRYSTAL COMPOSITIONS**

(30) Priority: 09.08.1995 JP 225820/95
(71) Applicant: CHISSO CORPORATION, Osaka-shi Osaka 530 (JP)
(72) Inventor: KATO, Takashi, Ichihara-shi, Chiba 290 (JP); MATSUI, Shuichi, Ichihara-shi, Chiba 290 (JP); MIYAZAWA, Kazutoshi, Ichihara-shi, Chiba 290 (JP); HACHIYA, Norihisa, Ichihara-shi, Chiba 299-01 (JP); NAKAGAWA, Etsuo, Ichihara-shi, Chiba 290 (JP)
(74) Representative: Ziebig, Marlene, Dr. Dipl.-Chem.
(86) International application number: JP9602253
(87) International publication number: WO9706124

(57) **Abstract**

Novel liquid crystalline compounds and liquid crystal compositions comprising the compound are provided which liquid crystalline compounds have a high dielectric anisotropy, high voltage holding ratio at temperatures in which the compounds are employed, low threshold voltage, and improved solubility with other liquid crystal materials at low temperatures; and the liquid crystalline compounds are cyclohexane derivatives expressed by the following general formula (1) 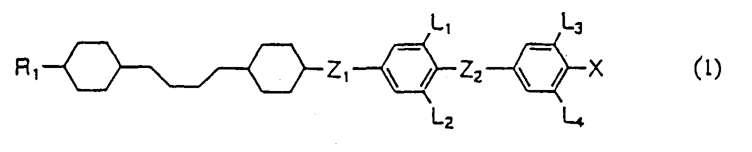 wherein R₁ represents an alkyl group having 1 to 15 carbon atoms, at least one non-adjacent methylene group (-CH₂-) in the alkyl group may be replaced by -O-, -S-, -CH=CH-, or -C≡C-; L₁, L₂, L₃, and L₄ independently represent H or F; X represent a halogen atom, CF₃, OCF₃, OCHF₂, CN, or NCS; and Z₁ and Z₂ independently represent -(CH₂)₂-, -CH=CH-, -COO-, or a covalent bond.

## Description

### TECHNICAL FIELD

The present invention relates to liquid crystalline compounds and liquid crystal compositions. More specifically, the invention relates to novel cyclohexane derivatives having butylene group as bonding group; to liquid crystal compositions comprising the liquid crystalline compound; and to liquid crystal display devices comprising the liquid crystal composition.

### BACKGROUND ART

Many display devices employing optical anisotropy and dielectric anisotropy which are characteristics of liquid crystalline compounds have heretofore been produced. The devices have widely been used for watches, tabletop calculators, word processors, TV sets, or the likes, and the demand for the display devices is increasing year by year.

Liquid crystal materials exhibit a liquid crystal phase, which is inherent to each of them, between a solid phase and liquid phase. The liquid crystal phase may be divided broadly into a nematic phase, smectic phase, and cholesteric phase from the aspect of its form. Among them, the nematic phase has most widely been employed for display devices. As the display mode applied for liquid crystal display, TN (twisted nematic) mode, DS (dynamic scattering) mode, guest-host mode, and DAP (deformation of aligned phases) mode have been developed all of which show electro-optic effects. Particularly, based on a fact that liquid crystal display in color is further progressing lately, devices of TFT (thin-film-transistor) mode and STN (super twisted nematic) mode both of which form a class of TN mode have now become a main current, and display devices using these modes are being produced in a large quantity.

Whereas many liquid crystalline compounds including ones which are currently at a stage of research are known up to now, compounds which can be filled in display devices as a single liquid crystal material to actually use as liquid crystal composition have not yet been known.

This is because whereas liquid crystalline compounds expected to be used as a material for display devices must exhibit a liquid crystal phase in a temperature range as wide as possible with room temperature (which naturally happens in the world and at which display devices are most often used) being its center, must be sufficiently stable against environmental factors to be used, and must have physical properties sufficient to drive display devices, no compounds which can satisfy these requirements by a single compound have yet been found.

Accordingly, several kind of liquid crystalline compounds (the term "liquid crystalline compounds" is used in this specification as a general term for the compounds exhibiting a liquid crystal phase and for the compounds which do not exhibit a liquid crystal phase but are useful as a component of liquid crystal compositions) are mixed to prepare liquid crystal compositions having the required characteristics described above and supplied as material for practical use at present.

It is considered to be necessary that these liquid crystal compositions are stable against moisture, light, heat, and air which are usually present under the conditions in which display devices are used; are stable against electric field and/or electromagnetic radiation; have an appropriate value of physical properties such as optical anisotropy (Δn), dielectric anisotropy (Δε), viscosity (η), electroconductivity, and ratio of elastic constants K₃₃/K₁₁ (K₃₃: bend elastic constant, K₁₁: splay elastic constant) depending on the display mode and the shape of display devices. Also, it is considered to be necessary that liquid crystalline compounds to be mixed are chemically stable in the environment in which the liquid crystalline compounds are used, and that components in liquid crystal compositions have a good solubility to one another.

In addition, reduction of electric power consumed in color displays is currently demanded. Under such circumstance, physical properties which are especially required to be improved are threshold voltage (Vₜₕ) and voltage holding ratio (VHR).

One of the factors which control the Vₜₕ is Δε, and the Vₜₕ can be lowered by increasing the value of Δε. On the other hand, VHR is a factor which affects on the electric resistance in liquid crystal panels, lowering of the former causes the lowering of the latter, and lowering of the latter (electric resistance) should be avoided since it lowers the contrast of displays and particularly shortens the utilization life of displays driven at a low voltage. Thus, it has become an extremely important subject to achieve a low Vₜₕ and a high VHR for improving various characteristics of color displays including the reduction of electric power consumed therein.

Heretofore, compounds expressed by the following formula (a) or (b) are known, as ones having a high Δε and a high VHR, through Laid-open Japanese Patent Publication Nos. Hei 2-233,626 and Hei 5-310,605. 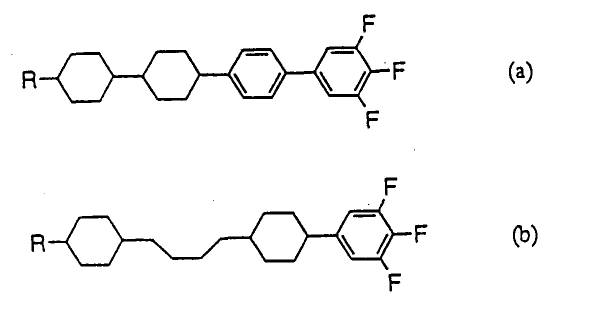 wherein R represents an alkyl group.

These compounds have 3,4,5-trifluoro group as a terminal group, and exhibit a comparatively high Δε. However, compounds expressed by the formula (a) are poor in mutual solubility with other known liquid crystalline compounds due to a fact, as a reason, that the compounds have a four ring structure in which rings are directly linked in a line. Further, not only the compounds of the formula (a) have a considerably lower VHR than a required value, particularly lower than 90 % at a temperature higher than 100°C, but also their temperature dependance can not be said to be good.

Compounds expressed by the formula (b) are expected to have a good mutual solubility by the effect of butylene group as a bonding group in the molecule. On the other hand, however, liquid crystal range of the compounds is very narrow due to the loss of molecular linearity caused by the insertion of the butylene group, and thus the compounds can not be said to be ones which satisfy the required characteristics.

### DISCLOSURE OF THE INVENTION

An object of the present invention is to solve the defects in the prior art described above. Another object of the present invention is to provide liquid crystalline compounds which exhibit a wide temperature range of a liquid crystal phase, have a high dielectric anisotropy and high voltage holding ratio, and exhibit a good mutual solubility; liquid crystal compositions comprising the compound; and liquid crystal display devices comprising the composition.

The present invention for achieving the objects described above is summarized as follows:
(1) A cyclohexane derivative expressed by the general formula (1) 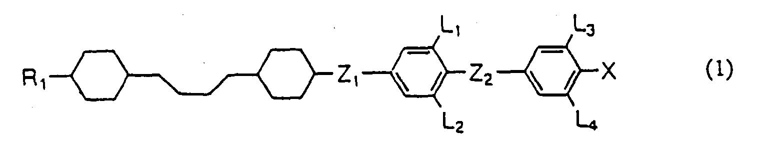 wherein R₁ represents an alkyl group having 1 to 15 carbon atoms, at least one non-adjacent methylene group (-CH₂-) in the alkyl group may be replaced by -O-, -S-, -CH=CH-, or -C≡C-; L₁, L₂, L₃, and L₄ independently represent H or F; X represent a halogen atom, CF₃, OCF₃, OCHF₂, CN, or NCS; and Z₁ and Z₂ independently represent -(CH₂)₂-, -CH=CH-, -COO-, or a covalent bond.
(2) The cyclohexane derivative recited in paragraph (1) above wherein both L₁ and L₂ are H.
(3) The cyclohexane derivative recited in paragraph (1) above wherein both L₁ and L₂ are F.
(4) The cyclohexane derivative recited in paragraph (2) above wherein R₁ is an alkyl group having 1 to 15 carbon atoms.
(5) The cyclohexane derivative recited in paragraph (3) above wherein R₁ is an alkyl group having 1 to 15 carbon atoms.
(6) A liquid crystal composition comprising at least two components and comprising at least one cyclohexane derivative recited in any one of paragraphs (1) to (5).
(7) A liquid crystal composition comprising, as a first component, at least one cyclohexane derivative recited in any one of paragraphs (1) to (5), and comprising, as a second component, at least one compound selected from the group consisting of the compounds expressed by any one of the general formulas (2), (3), and (4) 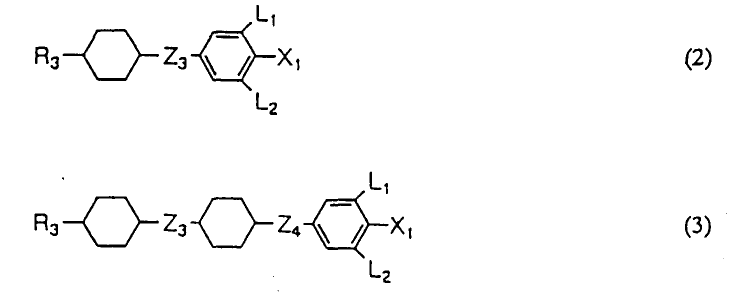 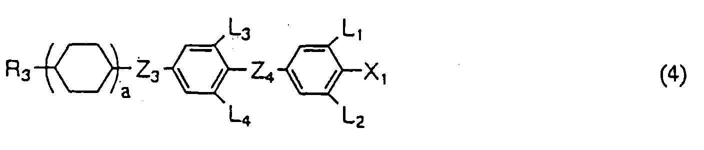 wherein R₃ represents an alkyl group having 1 to 10 carbon atoms; X₁ represents F, Cl, OCF₃, OCF₂H, CF₃, CF₂H, or CFH₂; L₁, L₂, L₃, and L₄ independently represent H or F; Z₃ and Z₄ independently represent -(CH₂)₂-, -CH=CH-, or a covalent bond; and a is 1 or 2.
(8) A liquid crystal composition comprising, as a first component, at least one cyclohexane derivative recited in any one of paragraphs (1) to (5) above, and comprising, as a second component, at least one compound selected from the group consisting of the compounds expressed by any one of the general formulas (5), (6), (7), (8), and (9) 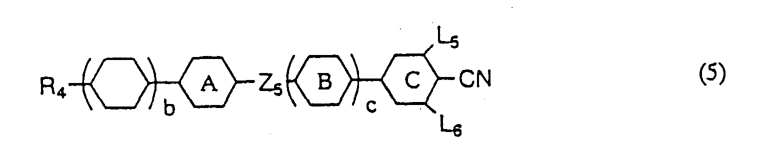 wherein R₄ represents F, an alkyl group having 1 to 10 carbon atoms, or an alkenyl group having 2 to 10 carbon atoms, at least one non-adjacent methylene group (-CH₂-) in the alkyl or alkenyl group may be replaced by oxygen atom (-O-); ring A represents trans-1,4-cyclohexylene group, 1,4-phenylene group, pyrimidine-2,5-diyl group, or 1,3-dioxane-2,5-diyl group; ring B represents trans-1,4-cyclohexylene group, 1,4-phenylene group, or pyrimidine-2,5-diyl group; ring C represents trans-1,4-cyclohexylene group or 1,4-phenylene group; Z₅ represents -(CH₂)₂-, -COO-, or a covalent bond; L₅ and L₆ independently represent H or F; and b and c are independently 0 or 1, 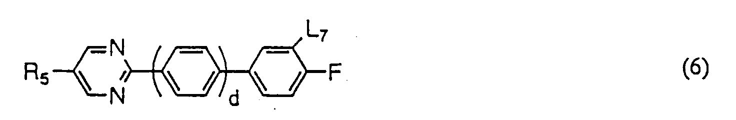 wherein R₅ represents an alkyl group having 1 to 10 carbon atoms; L₇ represents H or F; and d is 0 or 1, 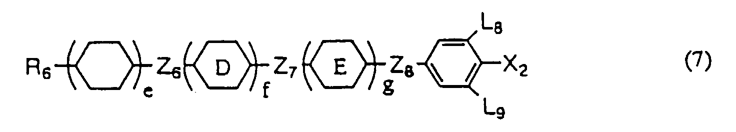 wherein R₆ represents an alkyl group having 1 to 10 carbon atoms; ring D and ring E independently represent trans-1,4-cyclohexylene group or 1,4-phenylene group; Z₆ and Z₇ independently represent -COO- or a covalent bond; Z₈ represents -COO- or -C≡C-; L₈ and L₉ independently represent H or F; X₂ represents F, OCF₃, OCF₂H, CF₃, CF₂H, or CFH₂ provided that when X₂ represents OCF₃, OCF₂H, CF₃, CF₂H, or CFH₂, then both L₈ and L₉ represent H; and e, f, and g are independently 0 or 1, 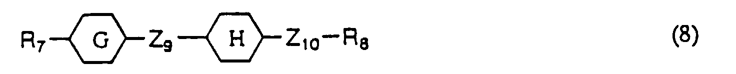 wherein R₇ and R₈ independently represent an alkyl group having 1 to 10 carbon atoms or an alkenyl group having 2 to 10 carbon atoms, at least one non-adjacent methylene group (-CH₂-) in the alkyl or alkenyl group may be replaced by oxygen atom (-O-); ring G represents trans-1,4-cyclohexylene group, 1,4-phenylene group, or pyrimidine-2,5-diyl group; ring H represents trans-1,4-cyclohexylene group or 1,4-phenylene group; Z₉ represents -C≡C-, -COO-, -(CH₂)₂-, -CH=CH-C≡C-, or covalent bond; and Z₁₀ represents -COO- or a covalent bond, 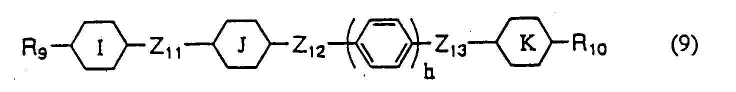 wherein R₉ and R₁₀ independently represent an alkyl group having 1 to 10 carbon atoms or an alkenyl group having 2 to 10 carbon atoms, at least one non-adjacent methylene group (-CH₂-) in the alkyl or alkenyl group may be replaced by oxygen atom (-O-); ring I represents trans-1,4-cyclohexylene group, 1,4-phenylene group, or pyrimidine-2,5-diyl group; ring J represents trans-1,4-cyclohexylene group, 1,4-phenylene group at least one hydrogen atom on which ring may be replaced by F, or pyrimidine-2,5-diyl group; ring K represents trans-1,4-cyclohexylene group or 1,4-phenylene group; Z₁₁ and Z₁₃ independently represent -COO-, -(CH₂)₂-, or a covalent bond; Z₁₂ represents -CH=CH-, -C≡C-, -COO-, or a covalent bond; and h is 0 or 1.
(9) A liquid crystal composition comprising, as a first component, at least one cyclohexane derivative recited in any one of paragraphs (1) to (5) above, comprising, as a part of a second component, at least one compound selected from the group consisting of the compounds expressed by any one of the general formulas (2), (3), and (4), and comprising, as the other part of the second component, at least one compound selected from the group consisting of the compounds expressed by any one of the general formulas (5), (6), (7), (8), and (9).
(10) A liquid crystal display device comprising a liquid crystal composition recited in any one of paragraphs (6) to (9) above.

### BRIEF DESCRIPTION OF THE DRAWING

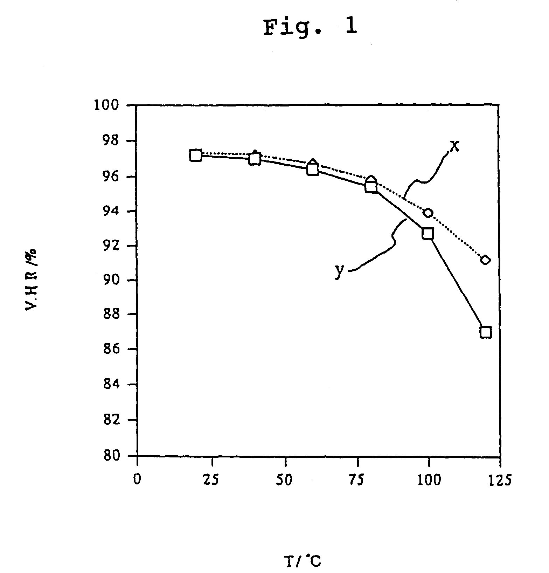

Fig. 1 is a graph showing the results of the determination of voltage holding ratio of liquid crystal compositions comprising a compound of the present invention or a comparative compound at different temperatures. In Fig. 1, x indicates the curve for the liquid crystal composition in Example 13, and y indicates the curve for that in Comparative Example 2.

### BEST MODE FOR CARRYING OUT THE INVENTION

Liquid crystalline compounds of the present invention expressed by the general formula (1) are derivatives of four ring system having, as their characteristic, butylene group in the molecule. These liquid crystalline compounds are extremely stable physically and chemically under conditions in which display devices are used. Besides, they have a good mutual solubility in liquid crystal compositions even at low temperatures, and have a high VHR and a high Δε. Further, it is possible to optionally adjust the physical properties of liquid crystalline compounds to desired ones by suitably selecting the structure of substituents or lateral chains in the constituents of the molecules. Accordingly, when the liquid crystalline compounds of the present invention are used as a component of liquid crystal compositions, novel liquid crystal compositions having such characteristics as
1) a high VHR and a small its dependency on temperature,
2) a possibility of driving display devices at a low voltage due to their low Vₜₕ, and
3) a wide temperature range of a nematic phase can be provided.

Compounds of the present invention expressed by the general formula (1) can roughly be classified into the groups of compounds expressed by one of the following formulas (1-a) to (1-k): 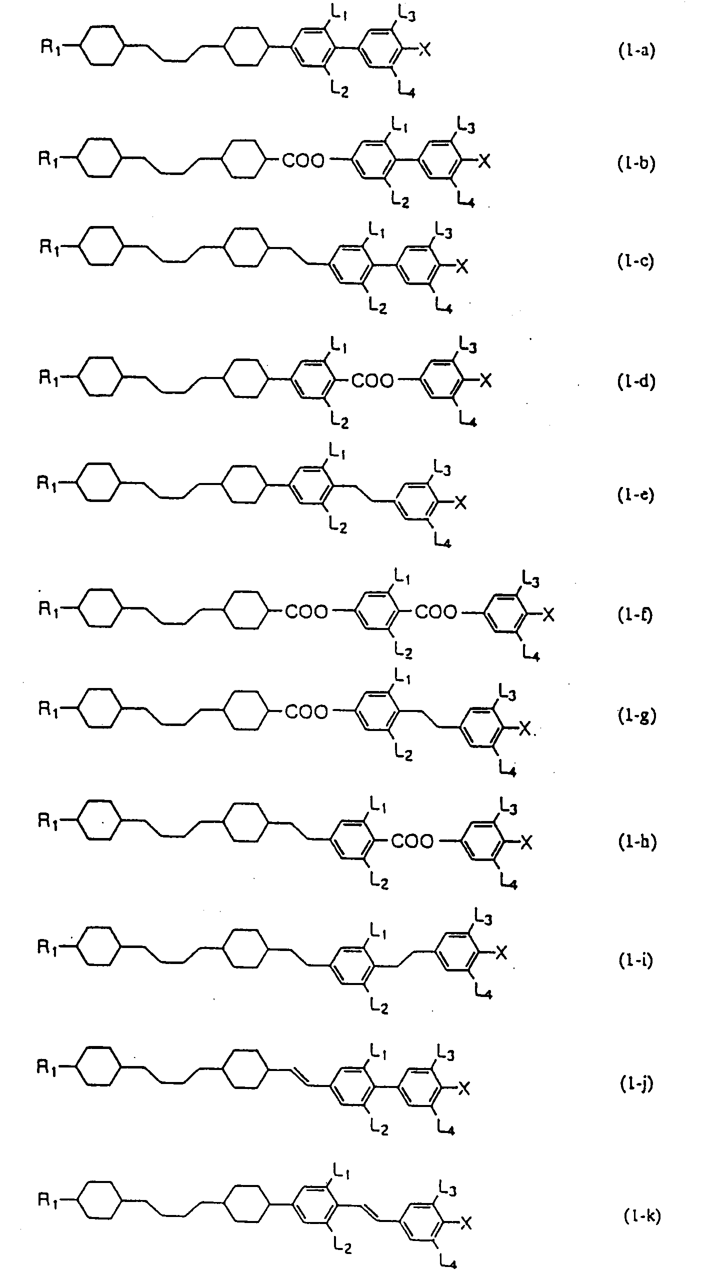 wherein R₁, L₁, L₂, L₃, and L₄ have the same meaning as described above.

Among these, the compounds expressed by the formula (1-a), (1-c), (1-e), or (1-i) are excellent as compounds for making it possible to drive TFT mode display devices at a low voltage, since they exhibit an especially wide temperature range of a nematic phase and a high VHR, and besides, they have a high Δε (they can provide compositions of a low Vₜₕ).

Among the compounds expressed by one of the formulas (1-a) to (1-k) illustrated above, the compounds of the formula (1-a) can further be expanded into those of the following formulas (1-a-1) to (1-a-45); (1-b) to (1-b-1) to (1-b-45); (1-c) to (1-c-1) to (1-c-45); (1-d) to (1-d-1) to (1-d-45); (1-e) to (1-e-1) to (1-e-45); (1-j) to (1-j-1) to (1-j-4); and the compounds of the formula (1-k) can further be expanded into those of the formulas (1-k-1) to (1-k-4), respectively, as preferable ones.

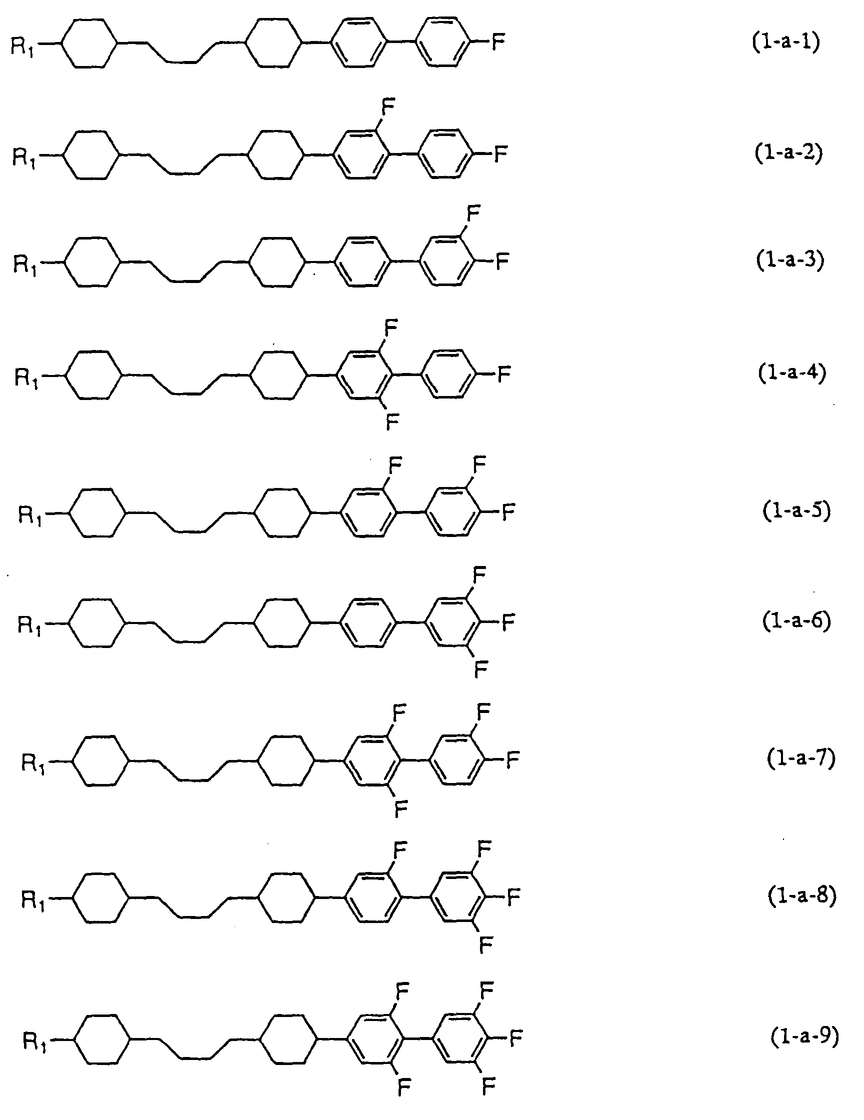

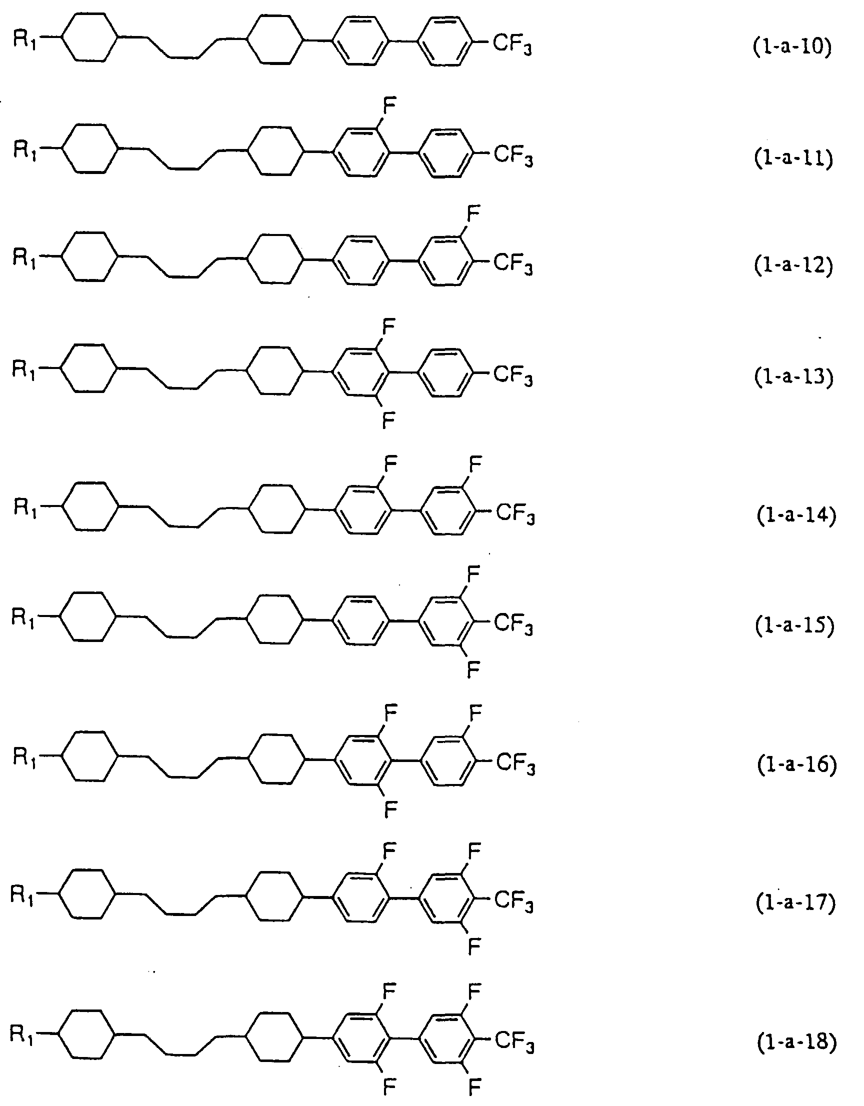

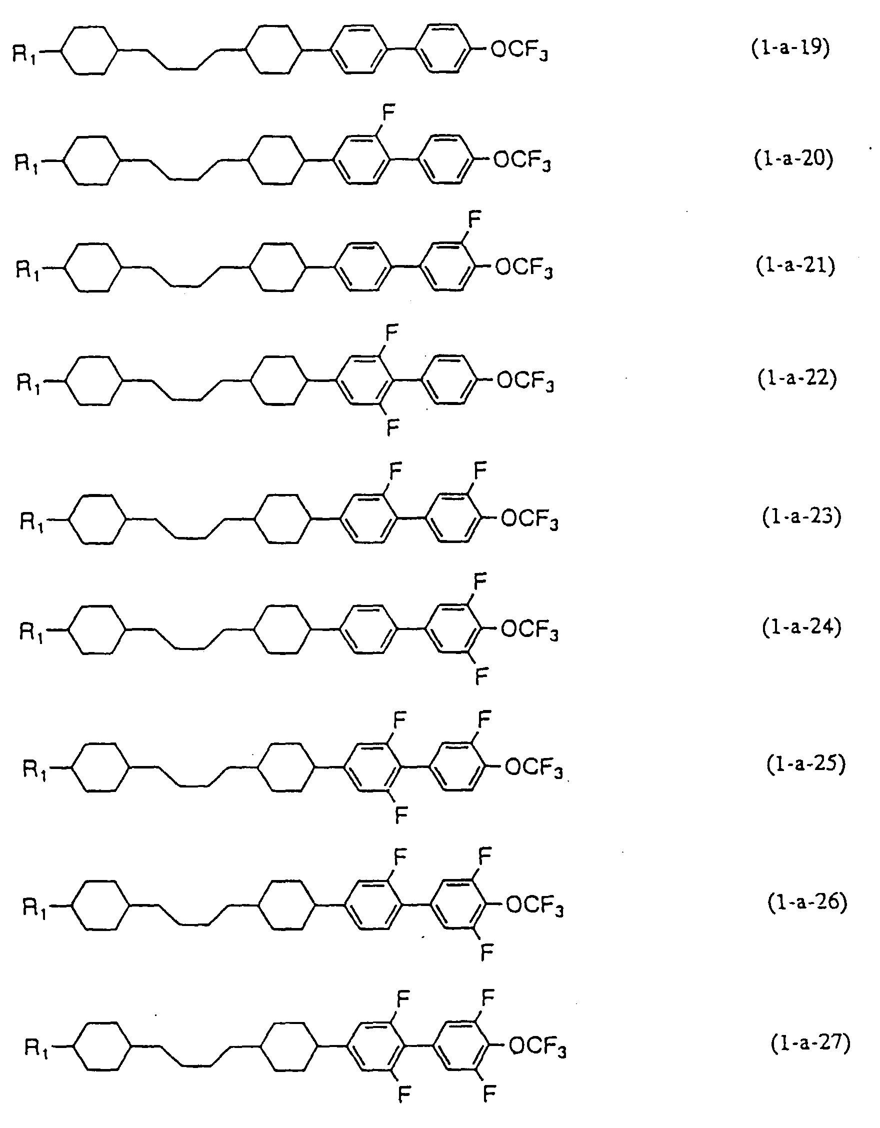

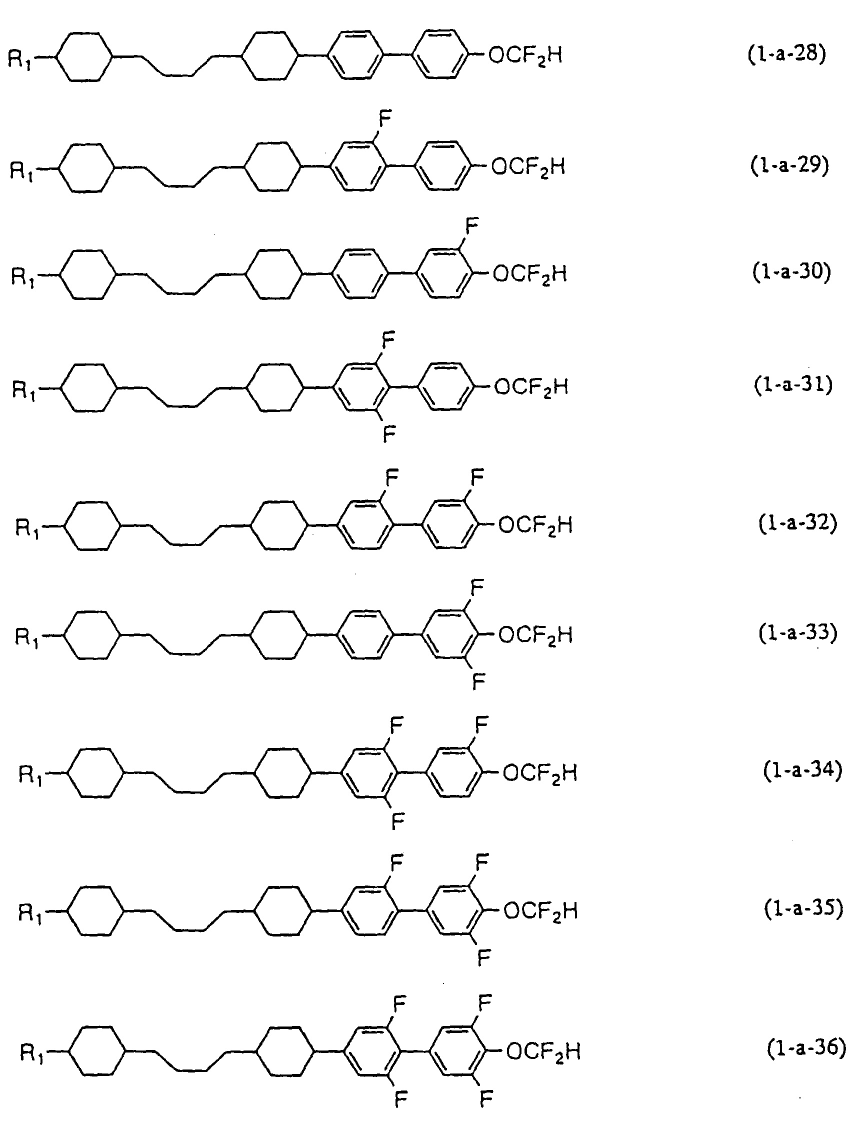

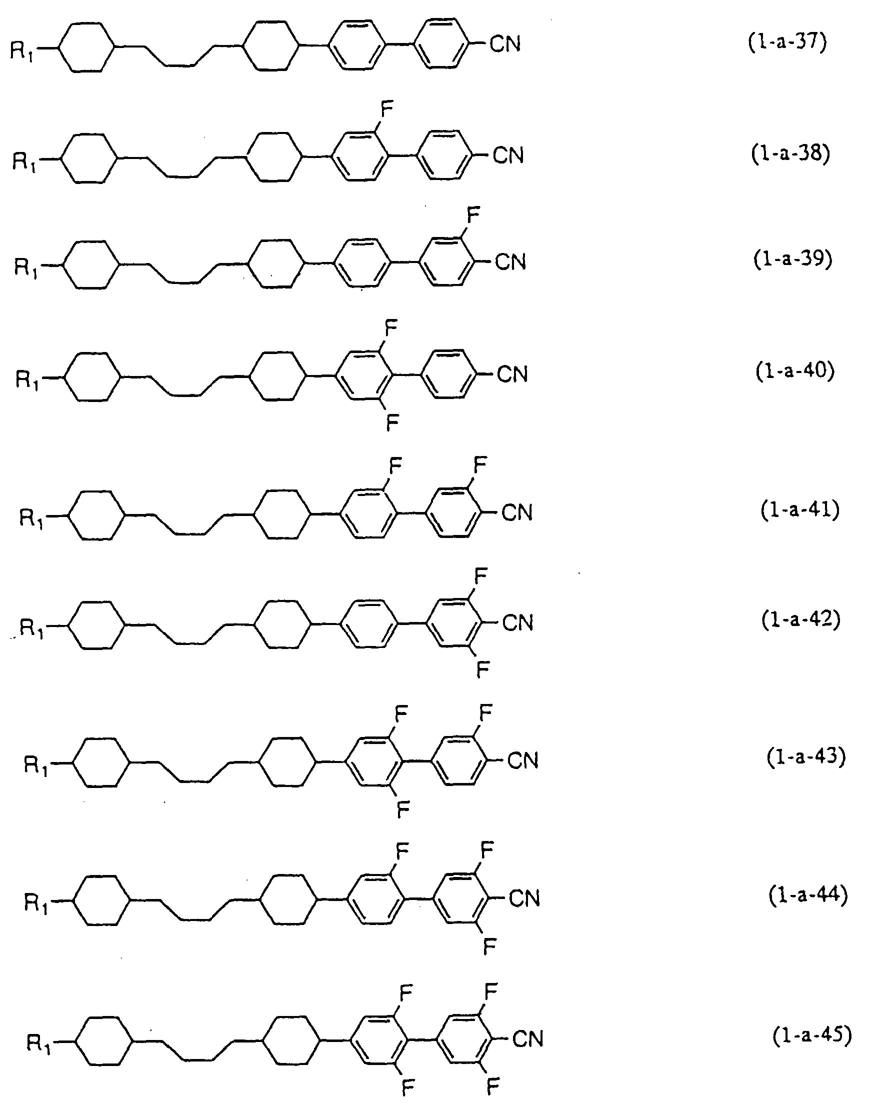

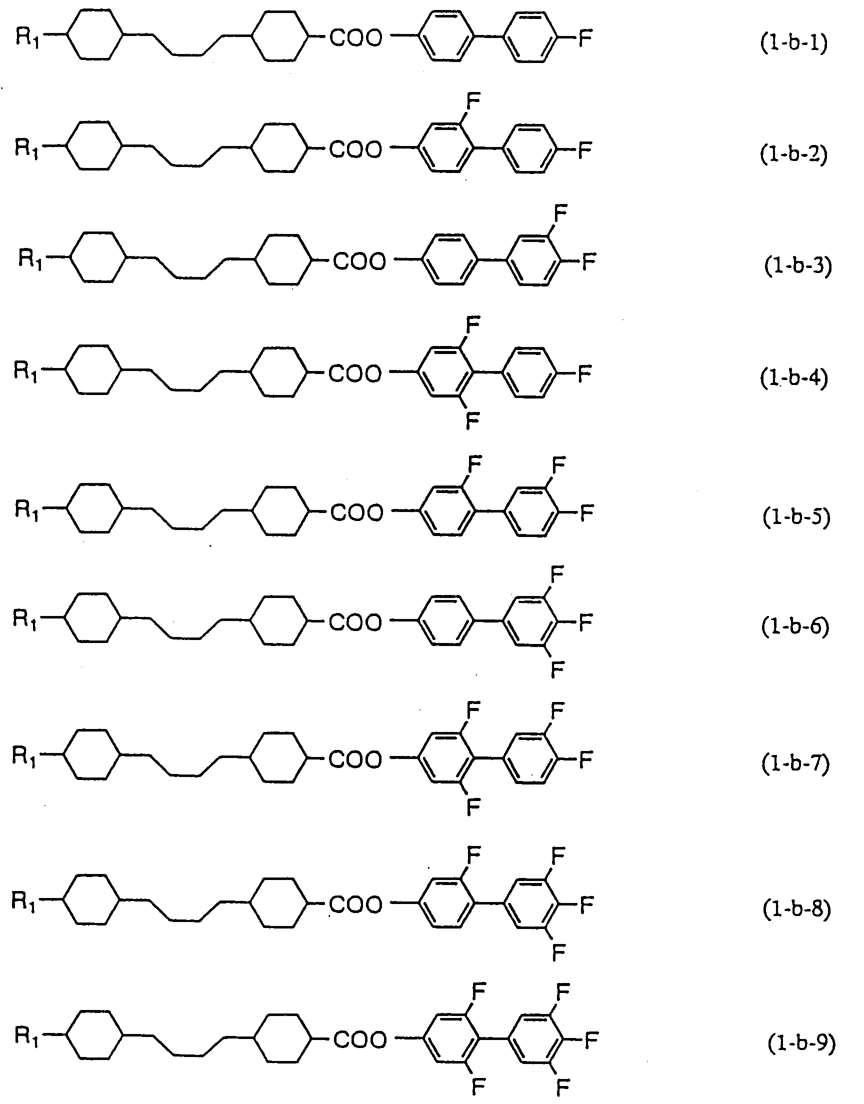

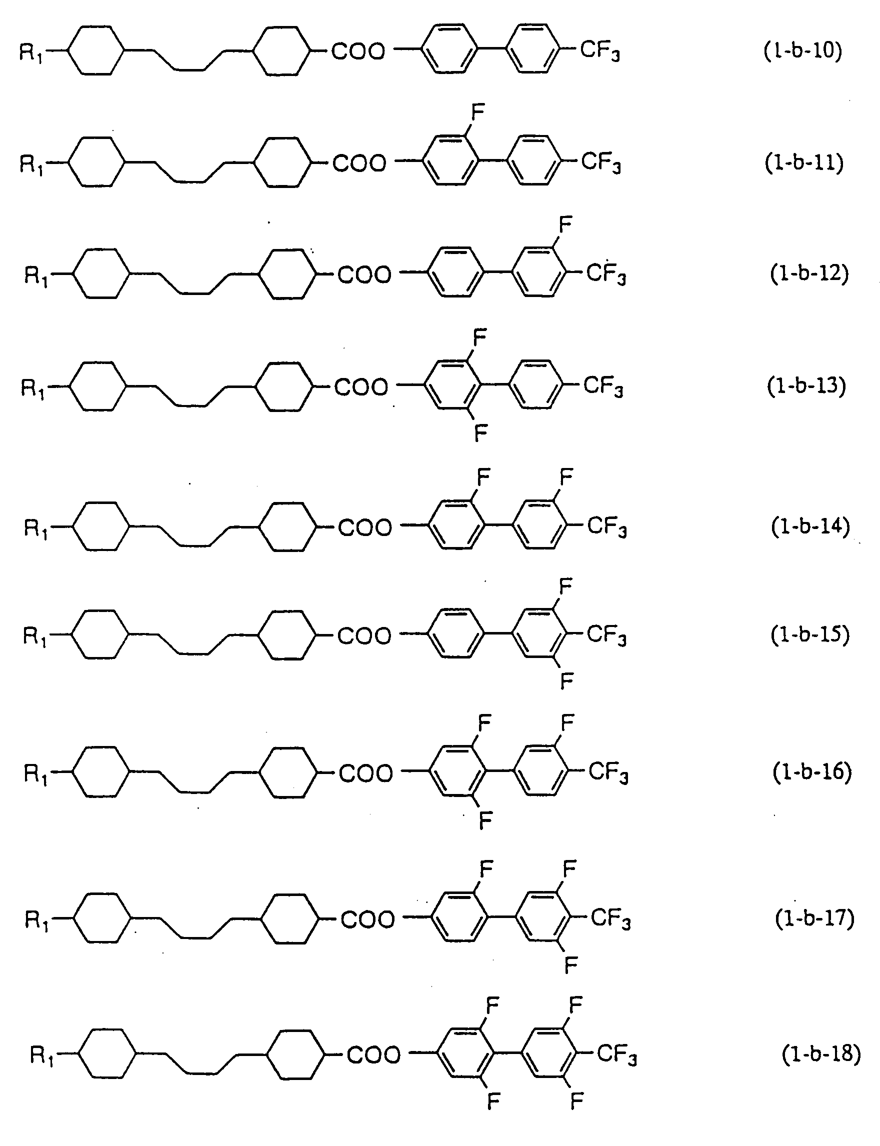

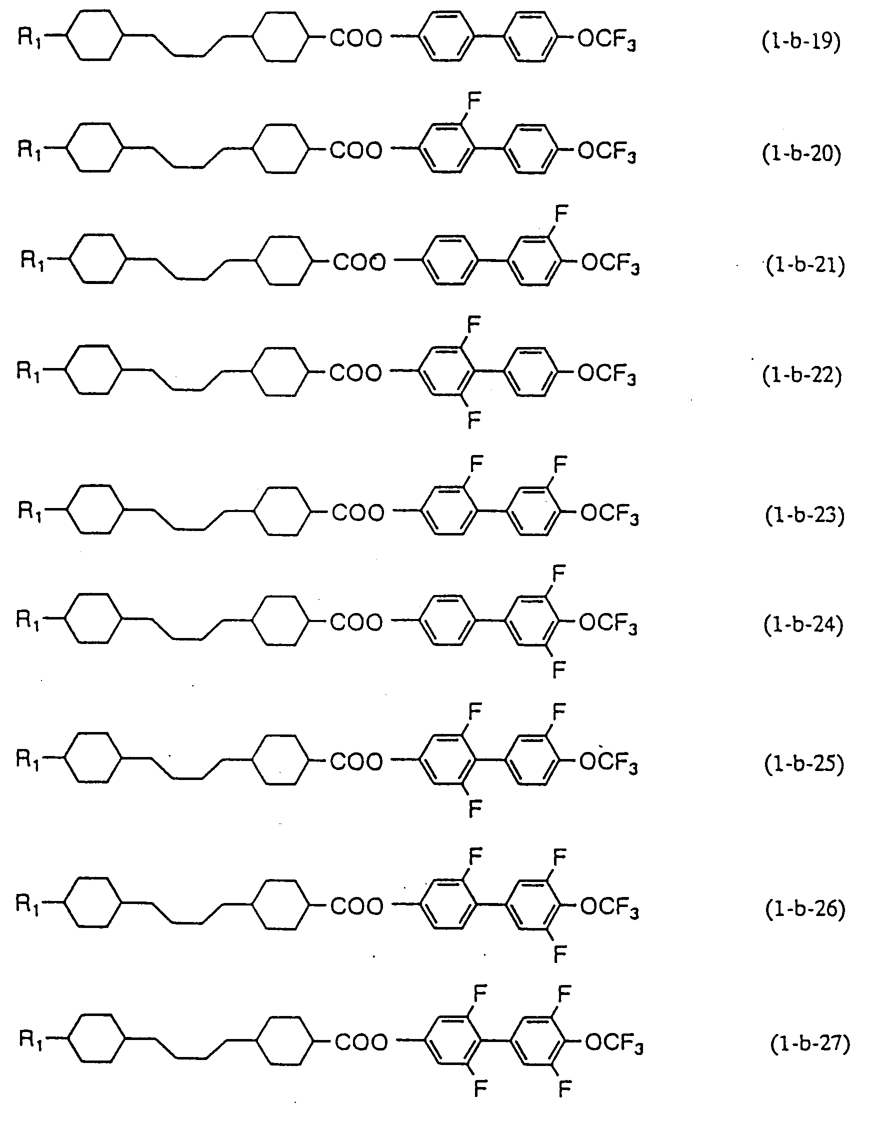

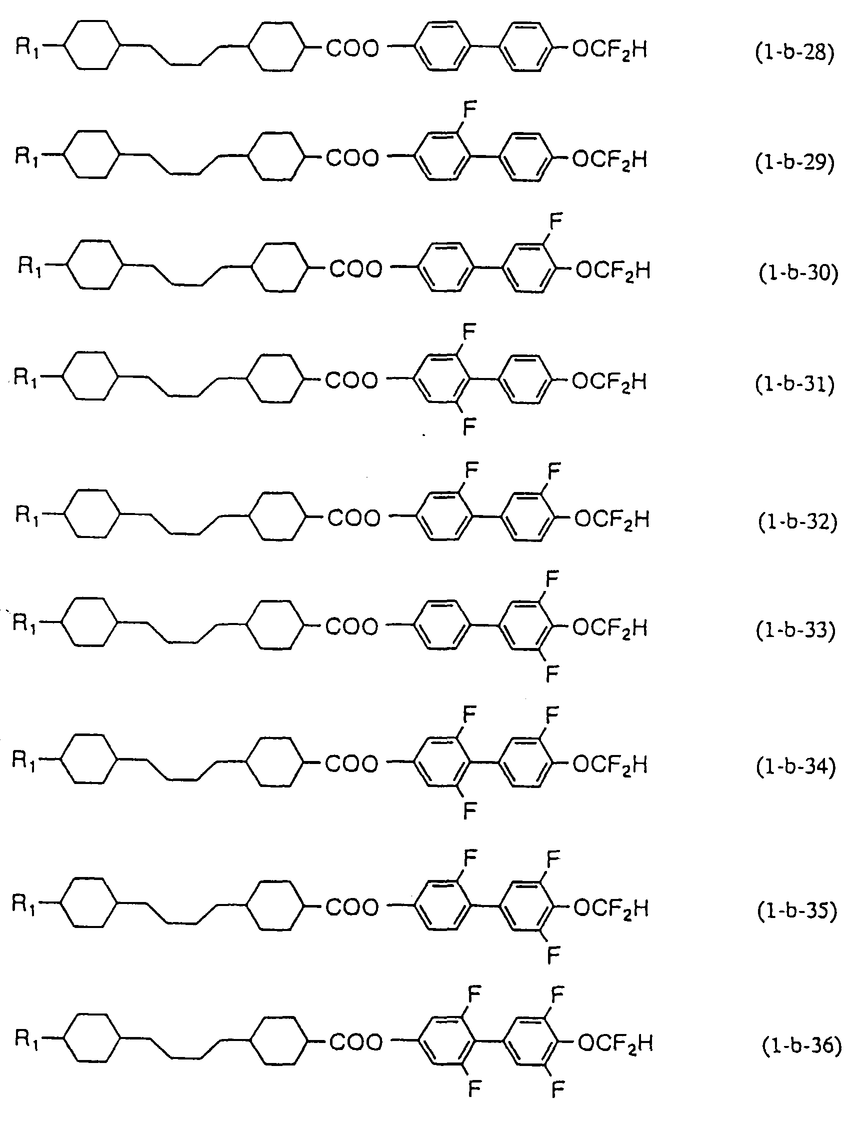

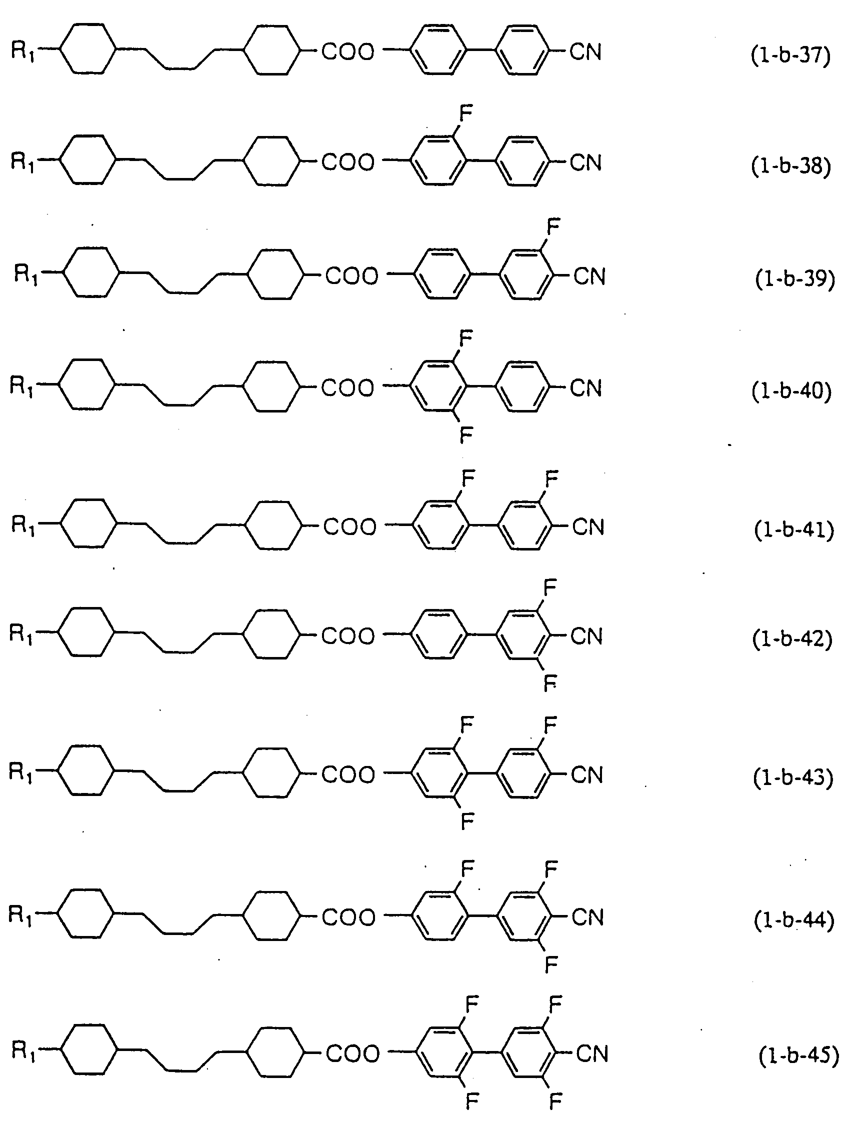

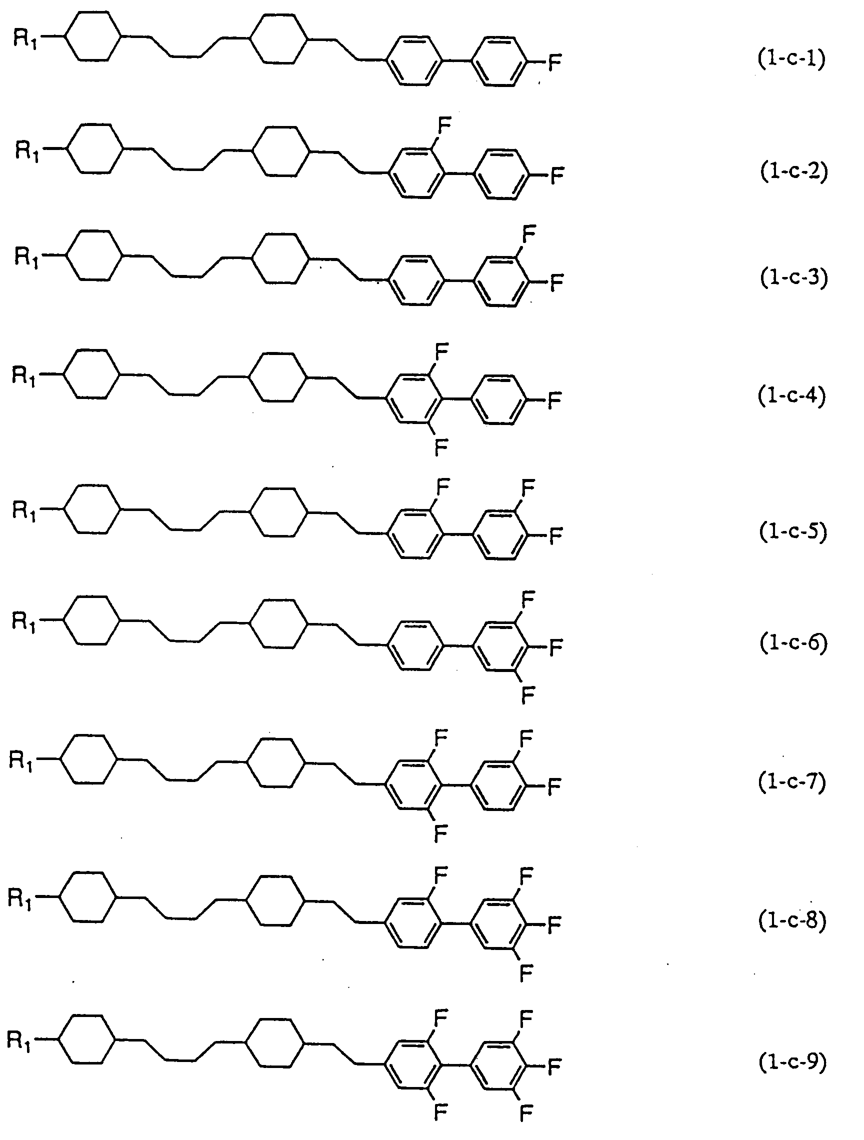

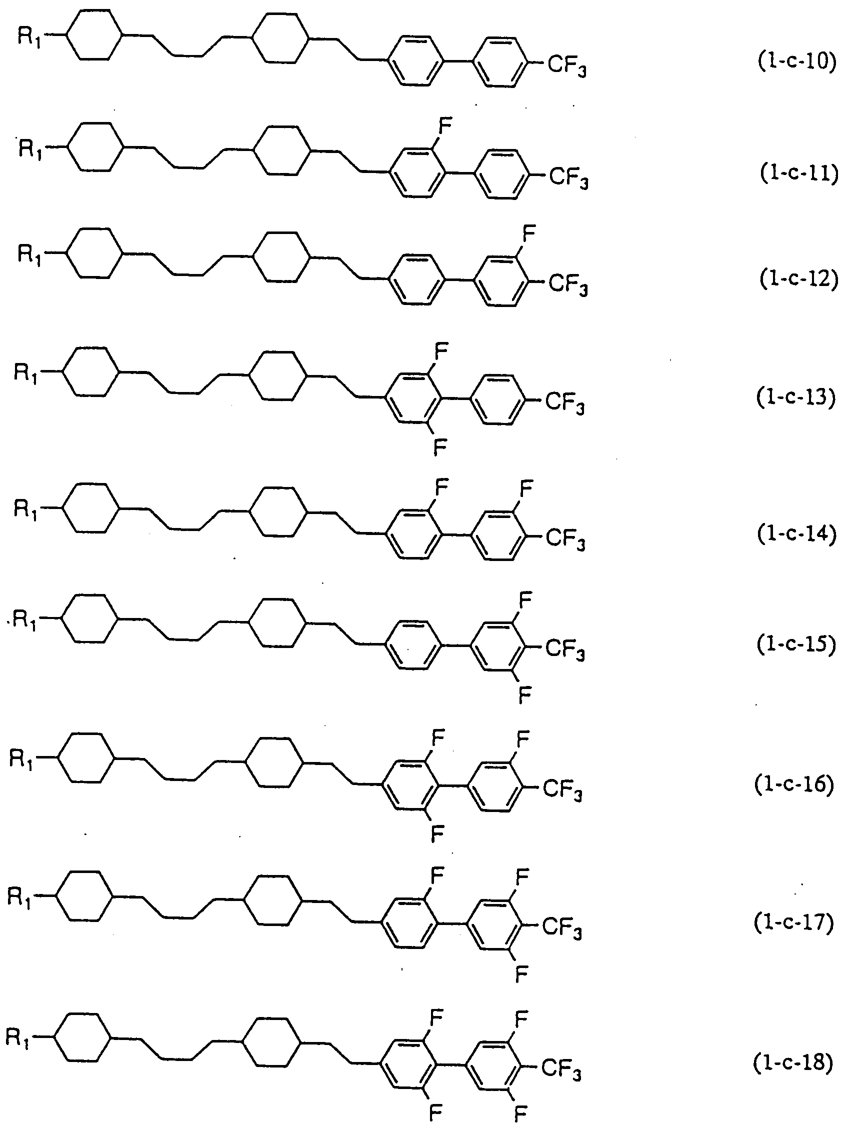

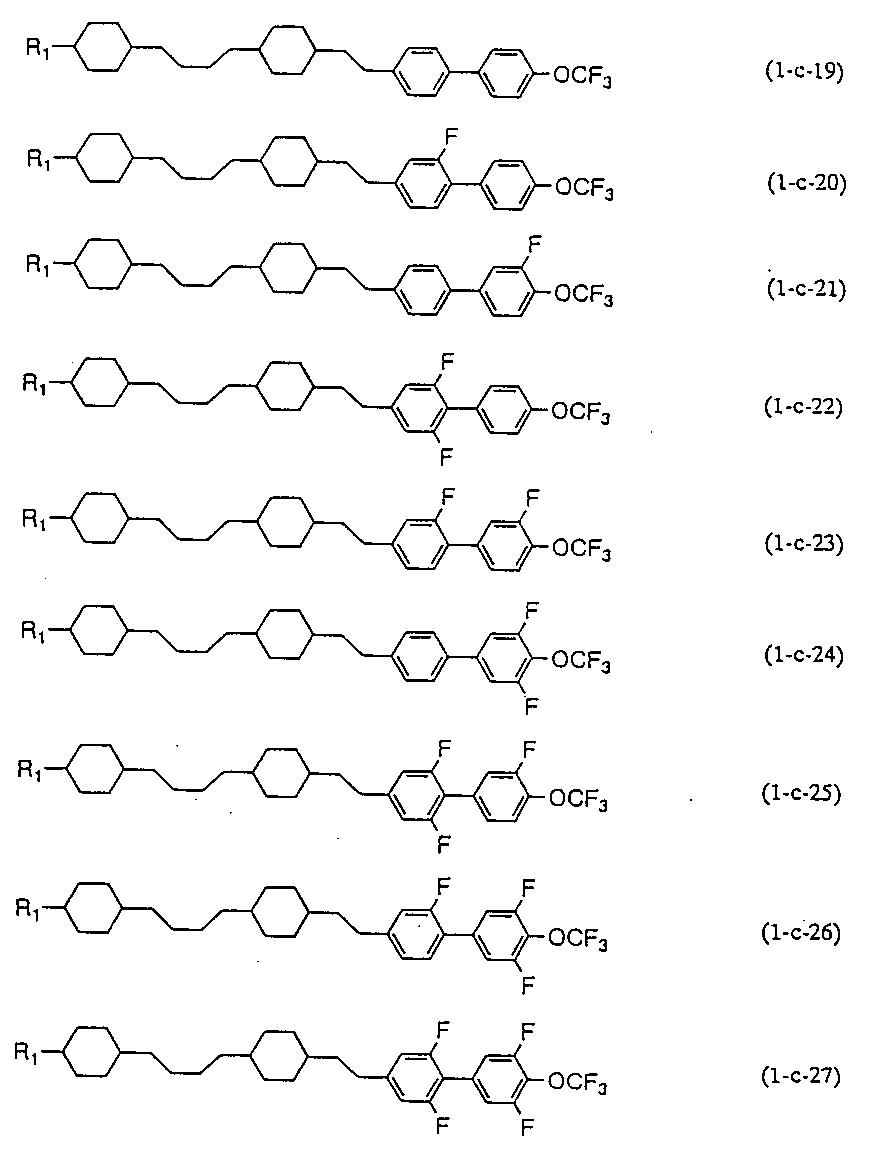

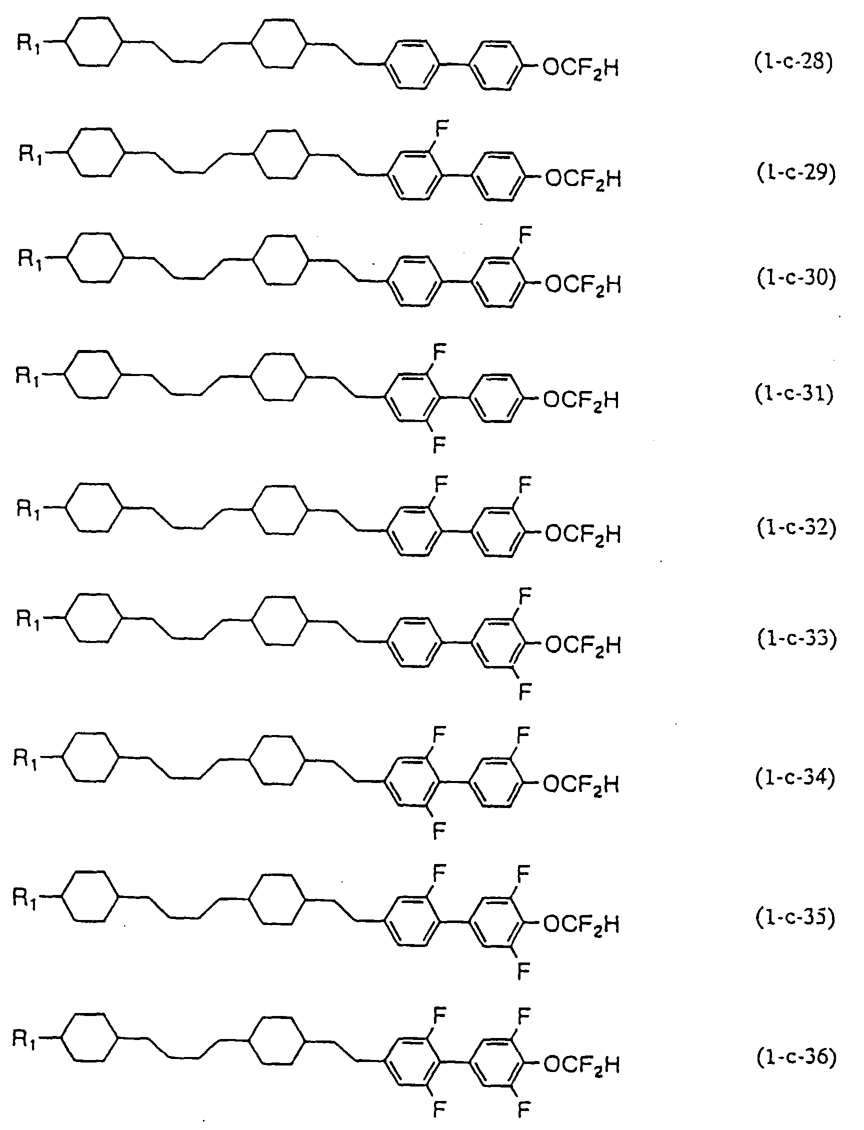

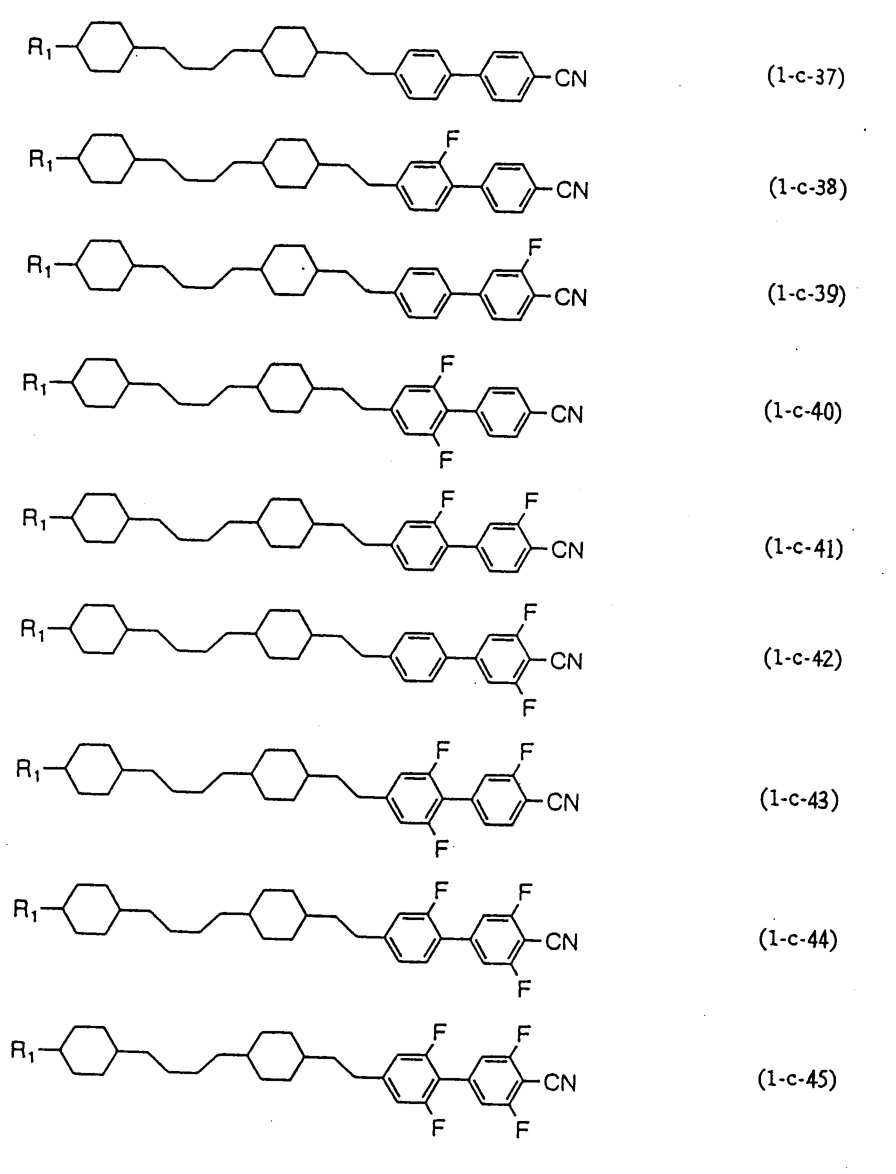

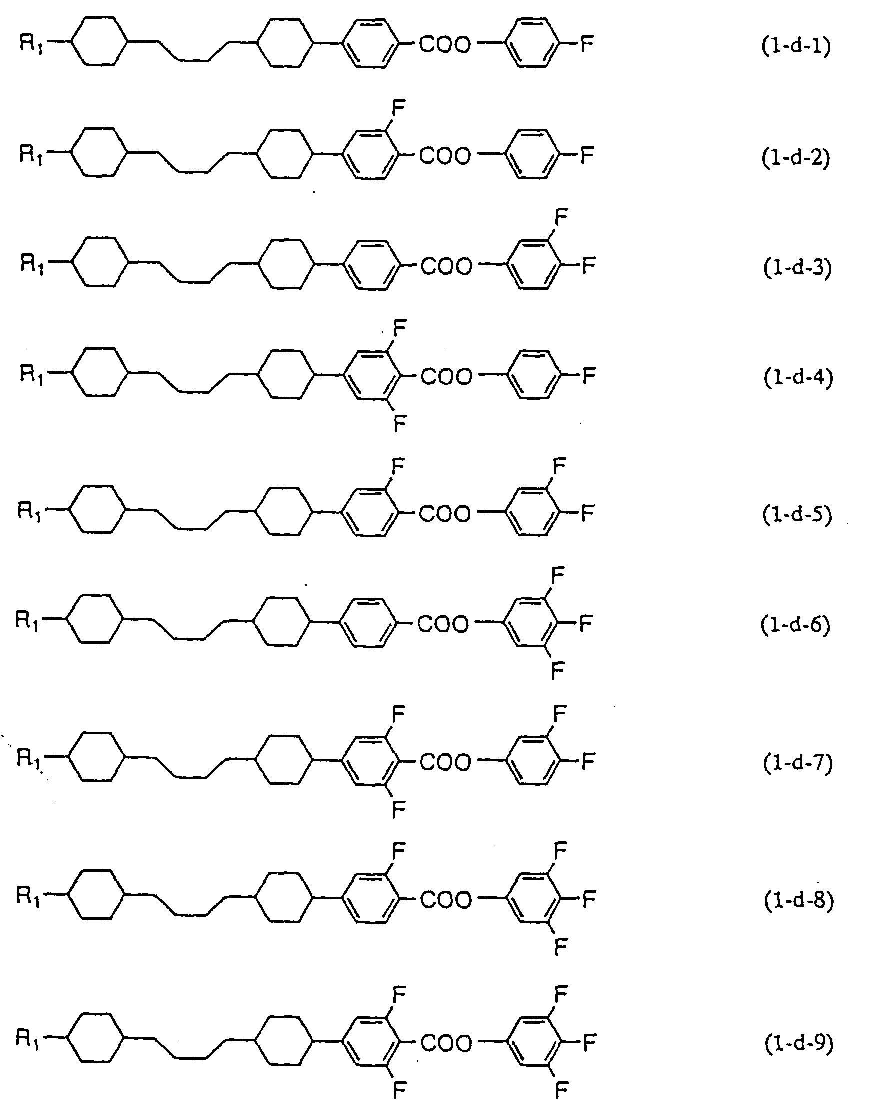

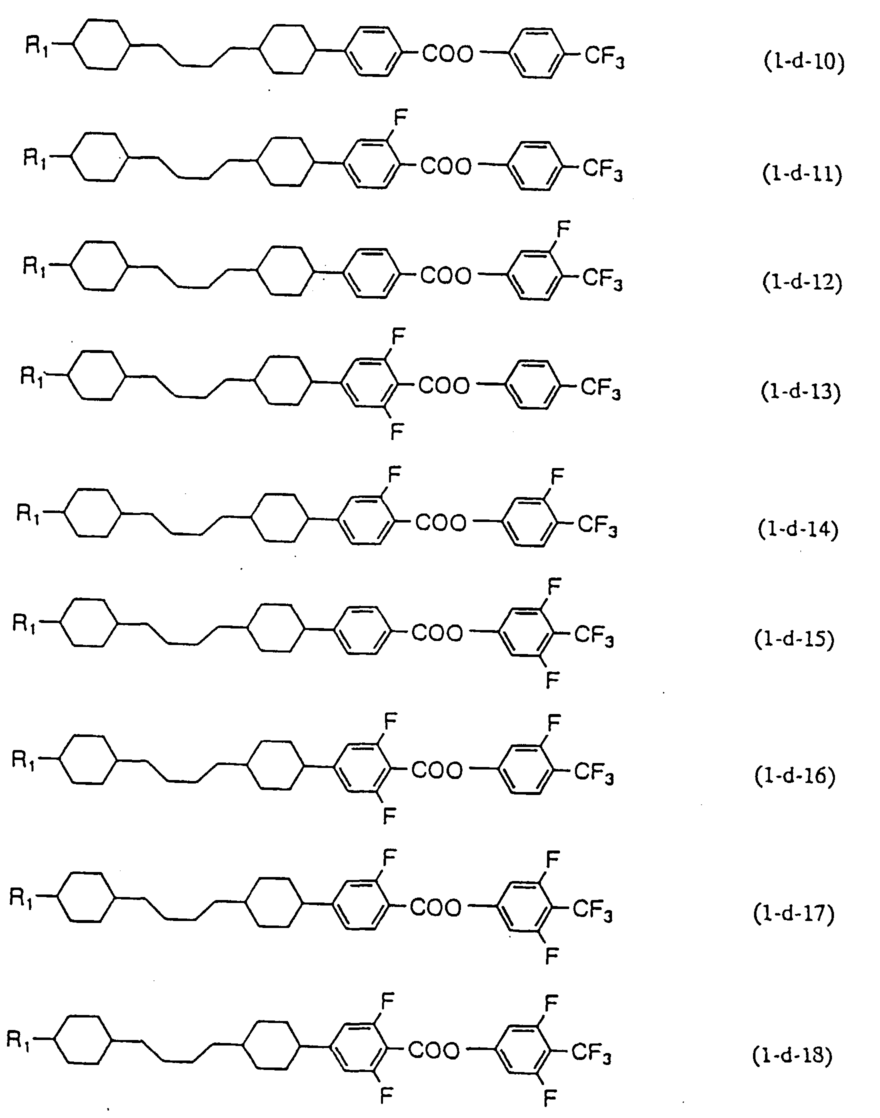

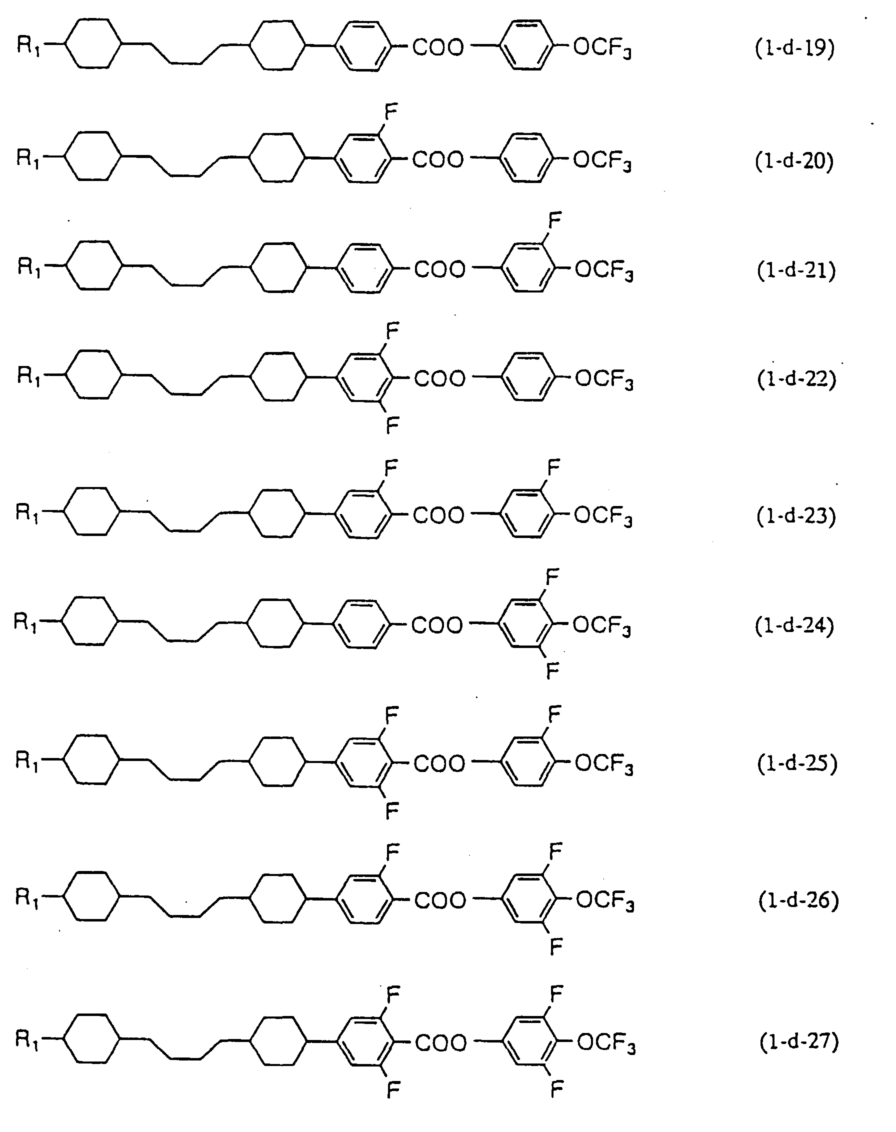

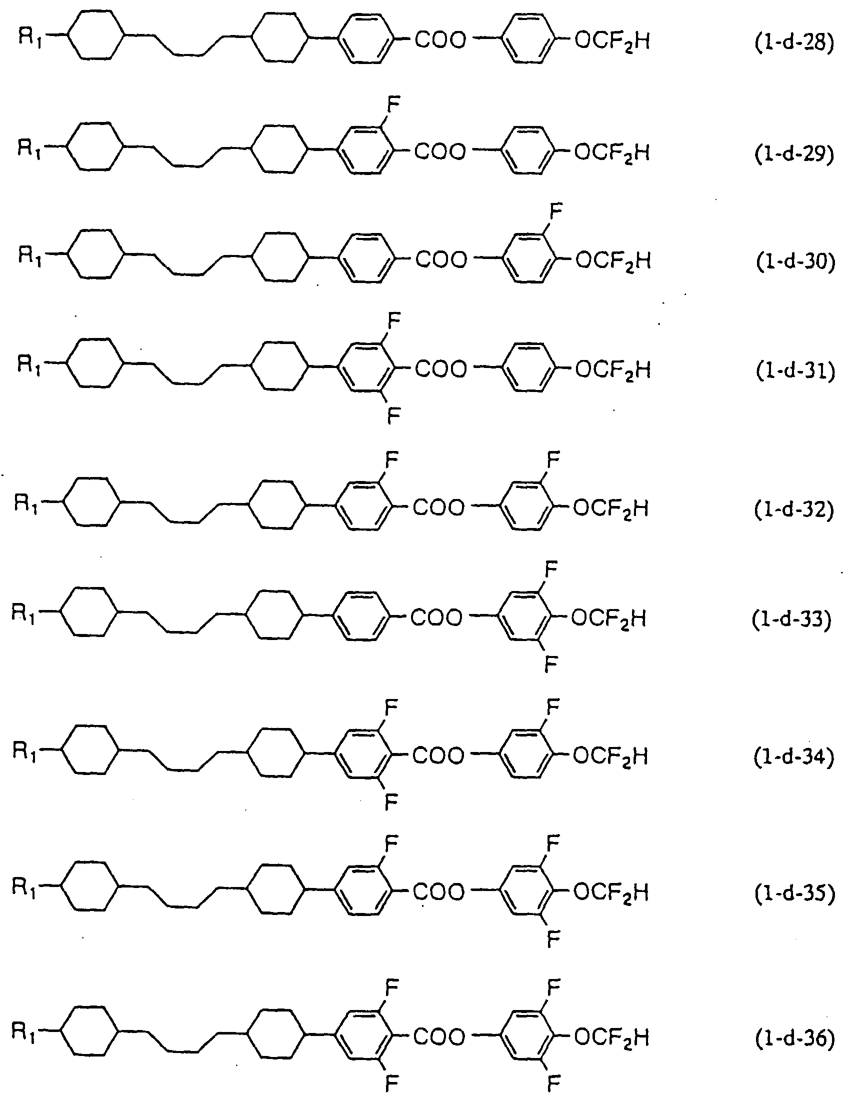

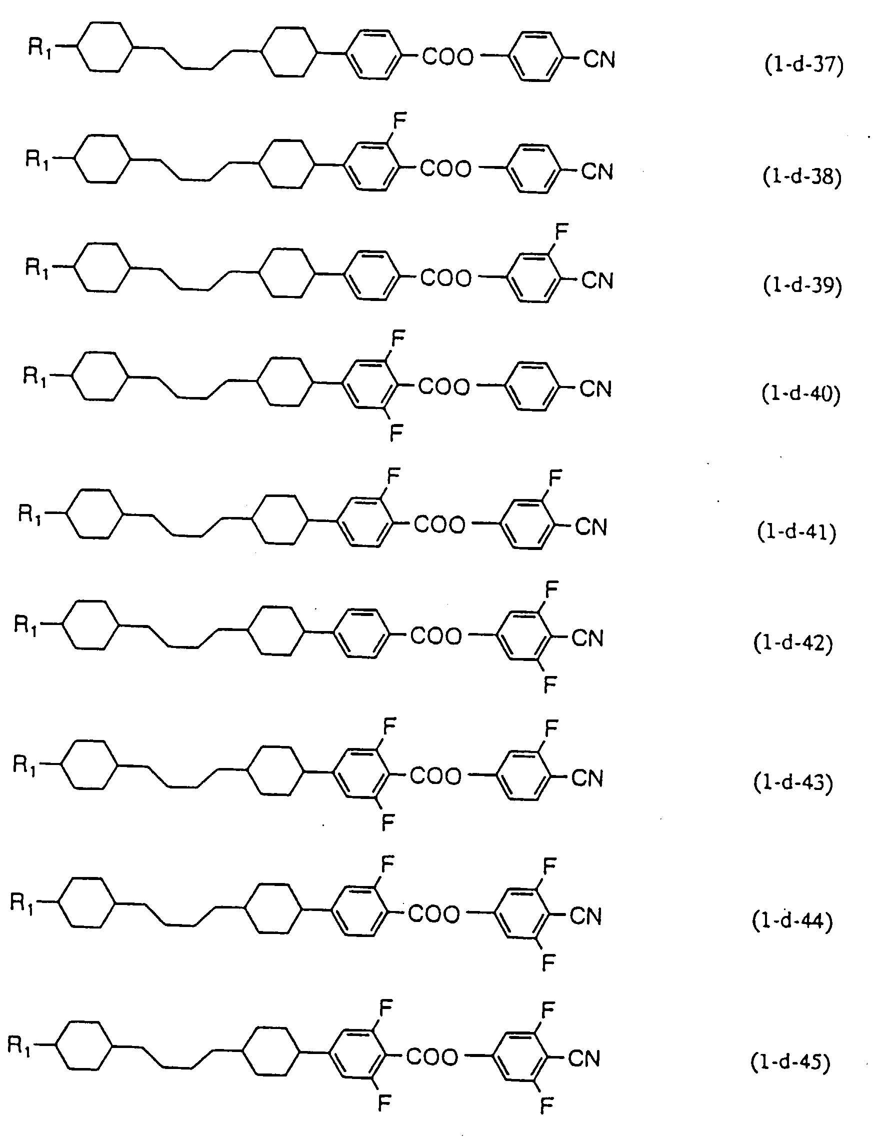

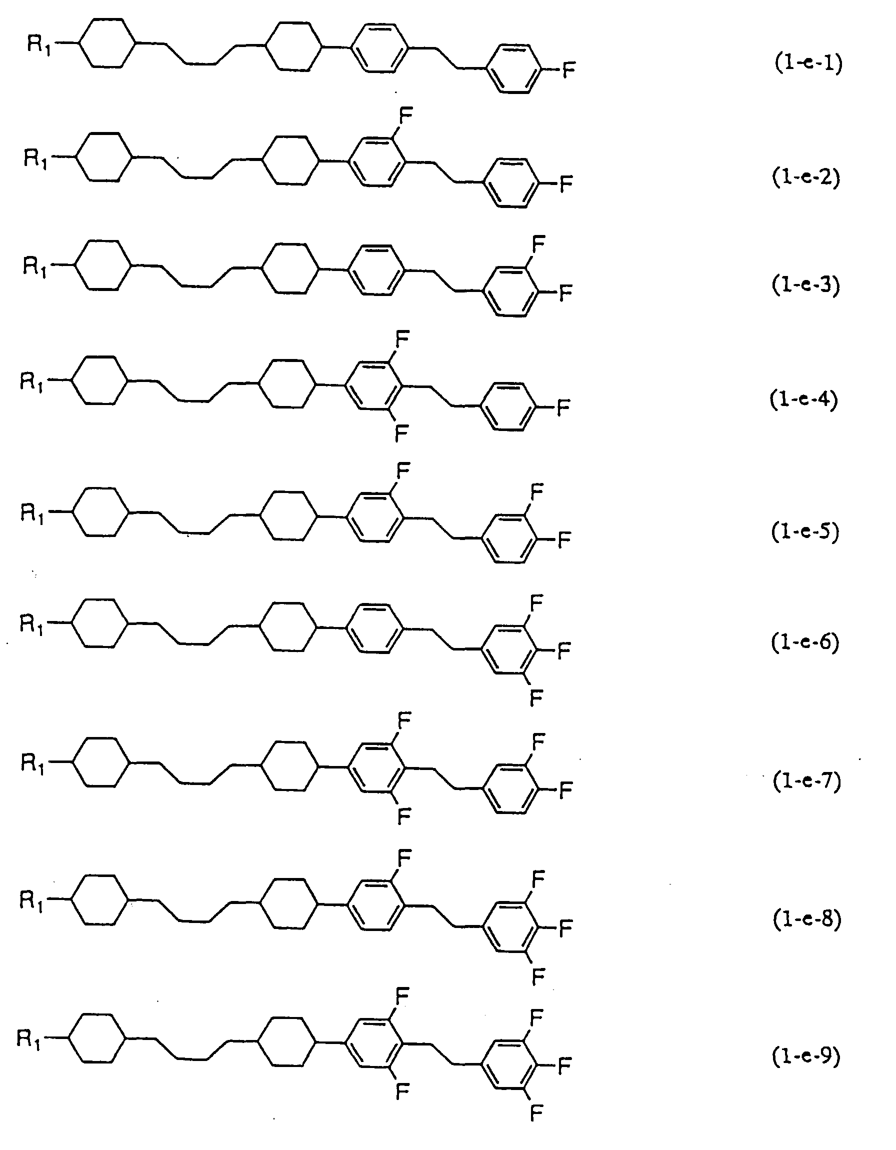

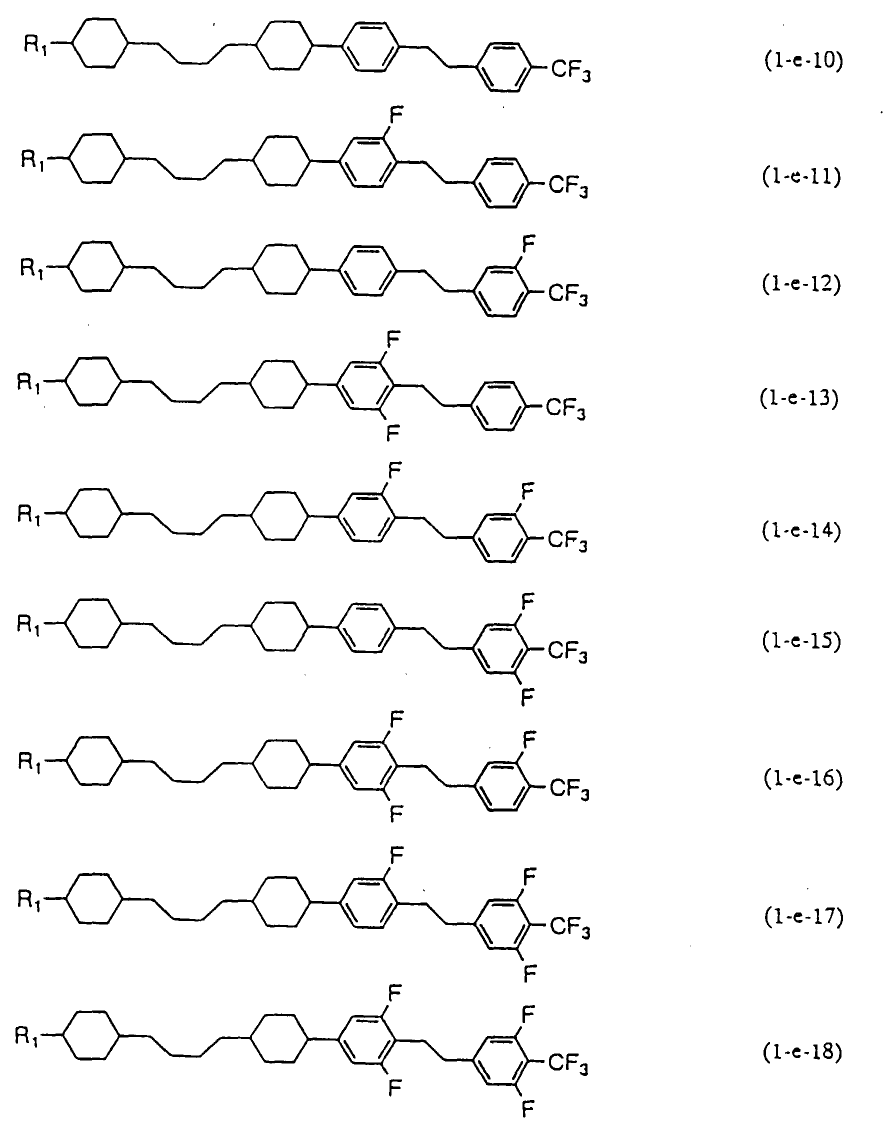

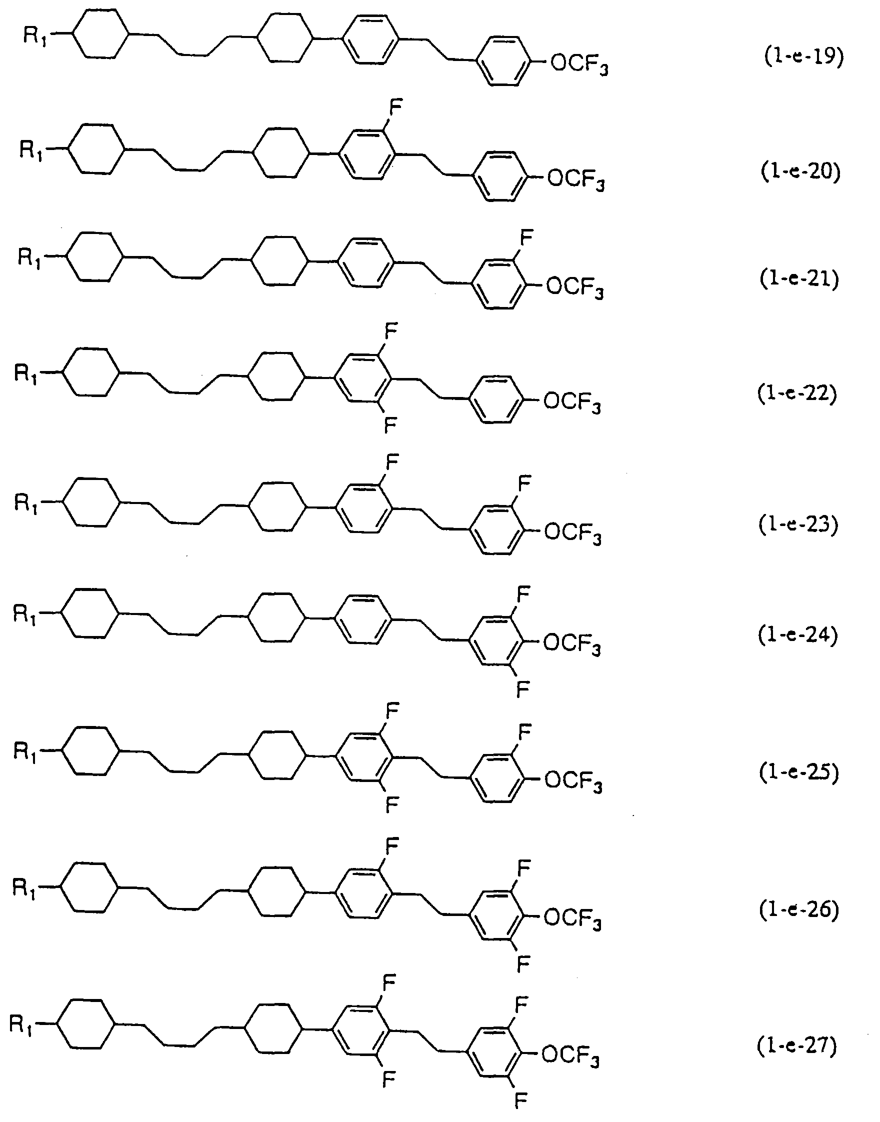

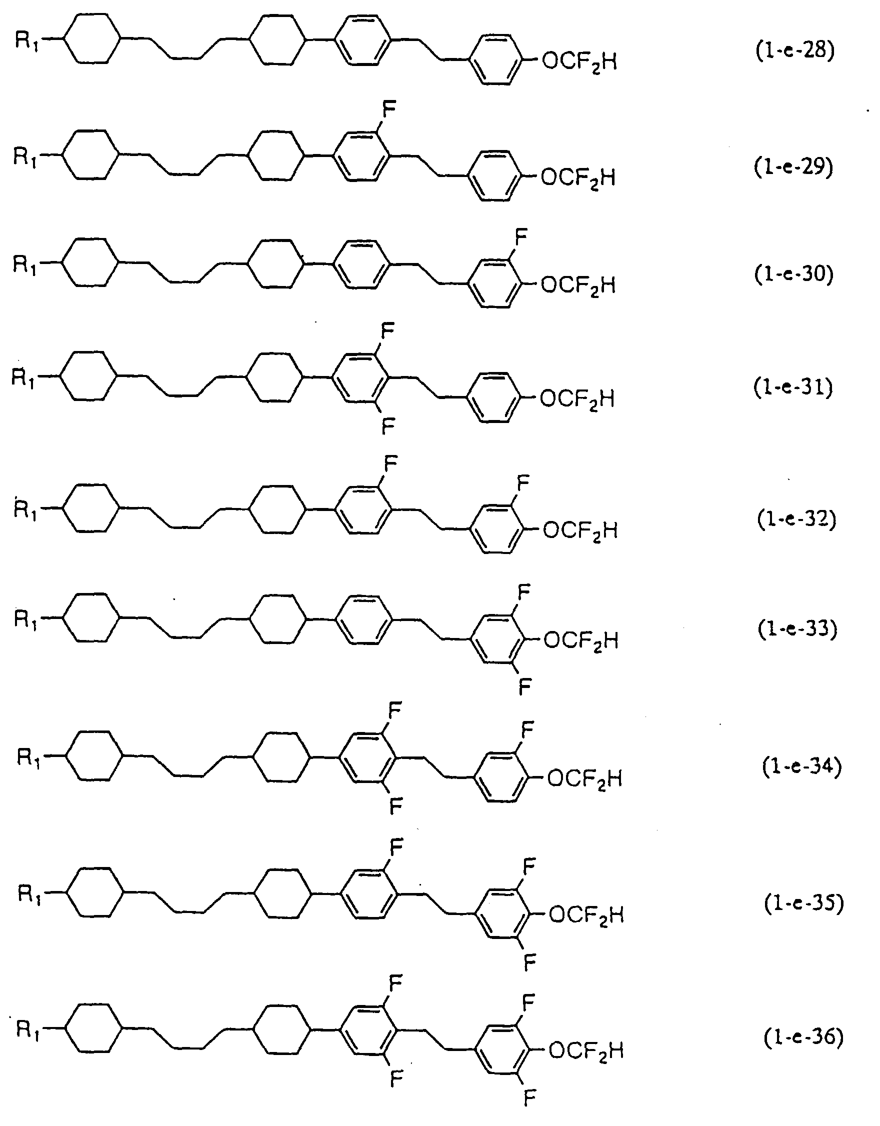

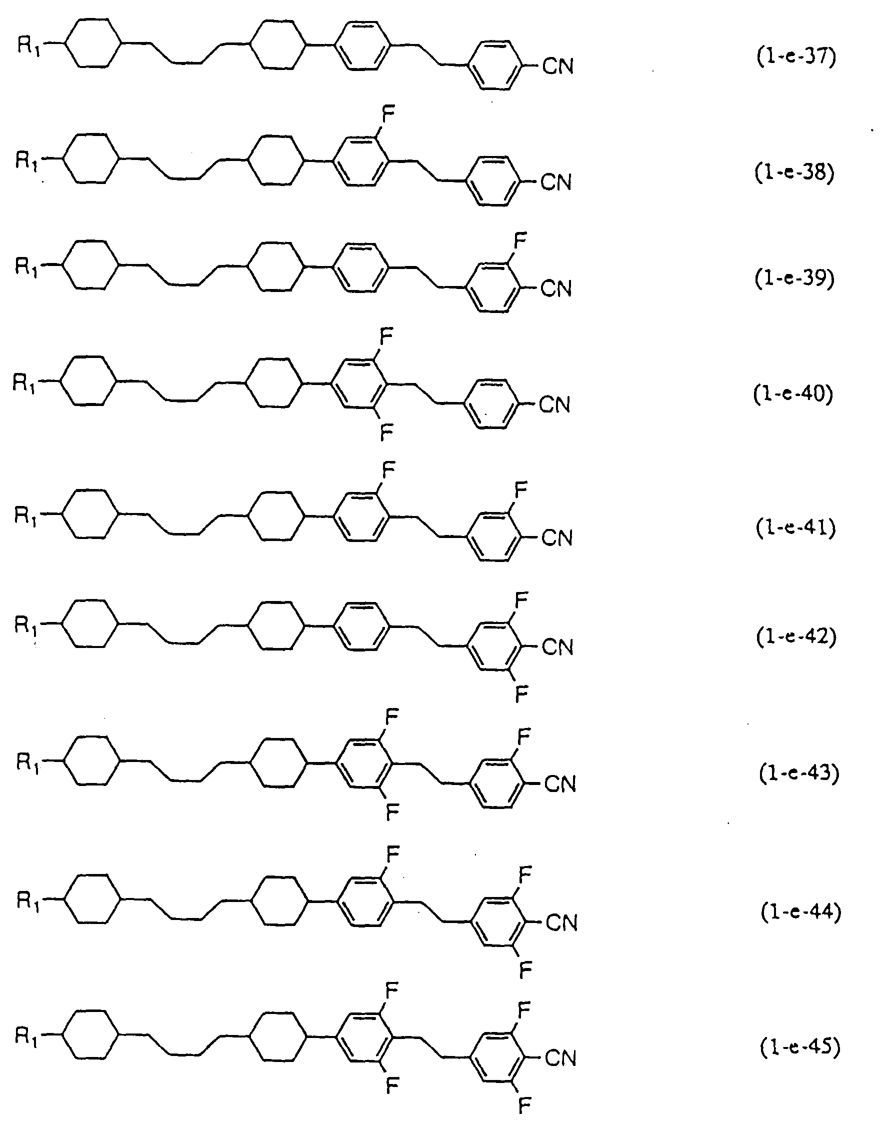

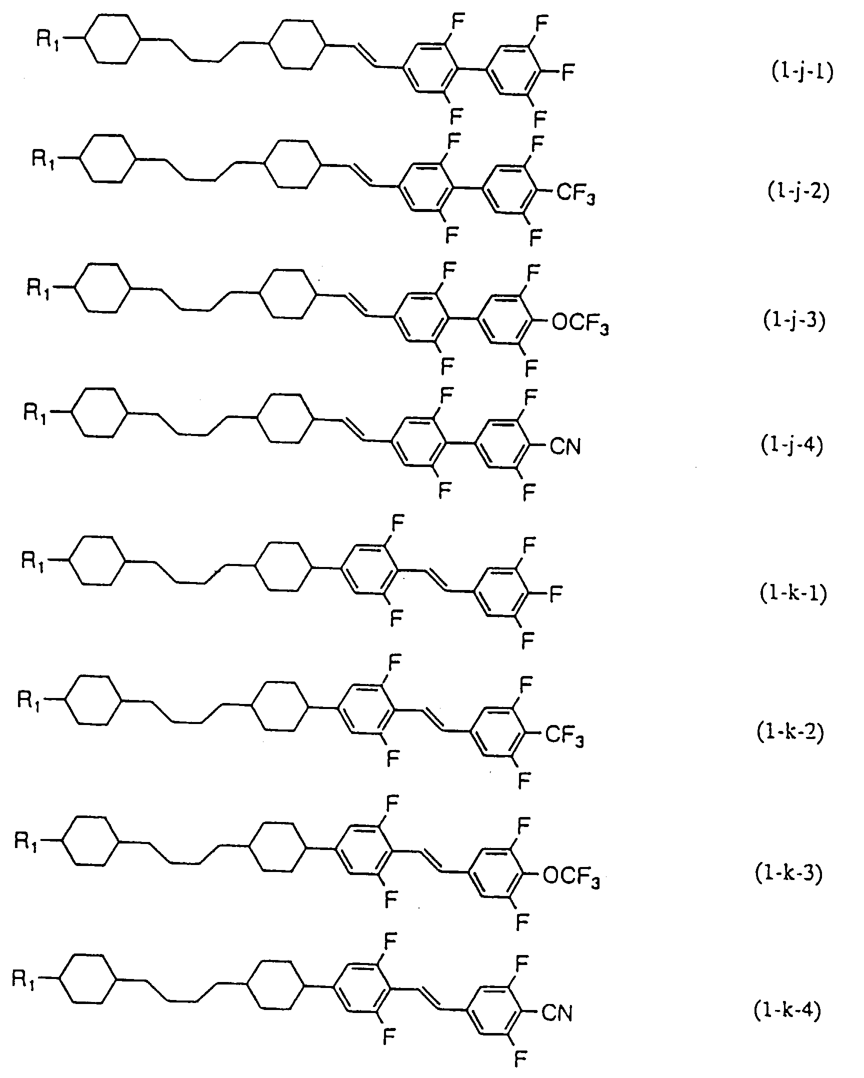

Liquid crystal compositions of the present invention may comprise only a first component comprising at least one liquid crystalline compound expressed by the general formula (1), and the compositions preferably comprise the first component in the ratio of 0.1 to 99.9 % by weight, to develop excellent characteristics.

In addition, at least one compound selected from the group consisting of the compounds expressed by one of the general formulas (2), (3), and (4) (hereinafter referred to as a second A component) and/or at least one compound selected from the group consisting of the compounds expressed by one of the general formulas (5), (6), (7), (8), and (9) (hereinafter referred to as a second B component) may be mixed to the liquid crystal compositions. Further, known compounds may be mixed as a third component for the purpose of adjusting Vₜₕ, temperature range of a liquid crystal phase, Δn, Δε, and viscosity.

Among the second A component described above, the compounds expressed by one of the following formulas (2-1) to (2-15), (3-1) to (3-48), and (4-1) to (4-55) can be mentioned as preferable examples of the compounds included in the general formula (2), (3), or (4): 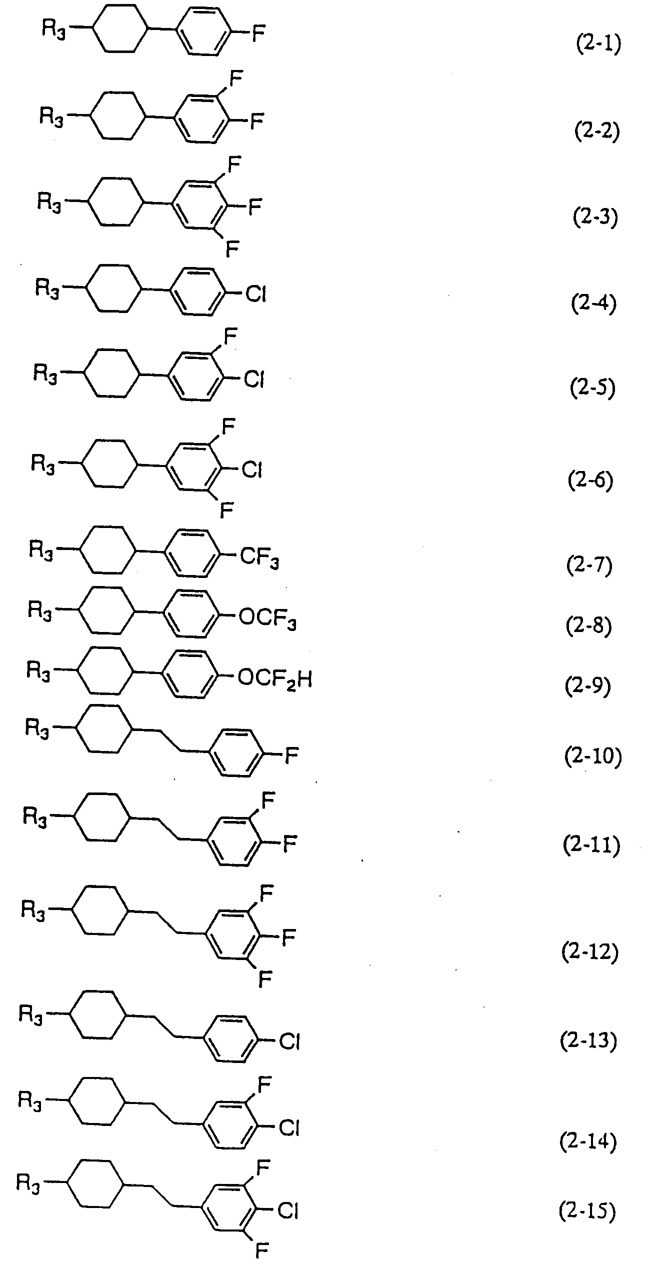 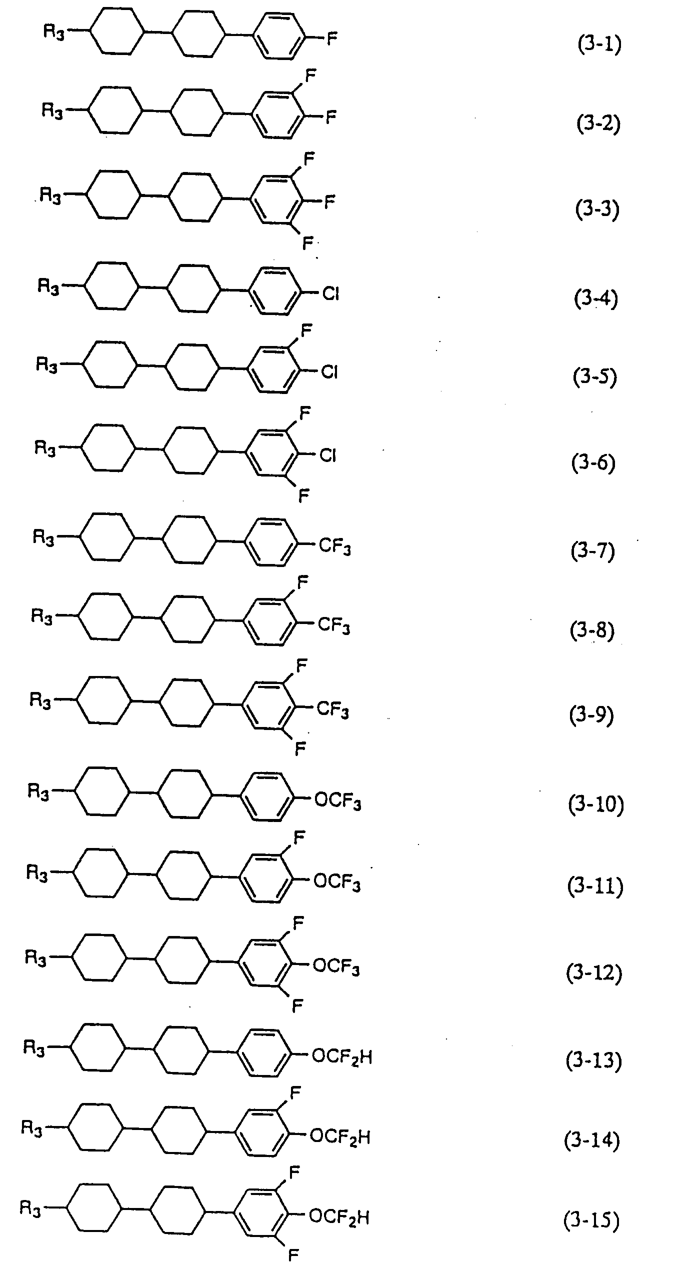 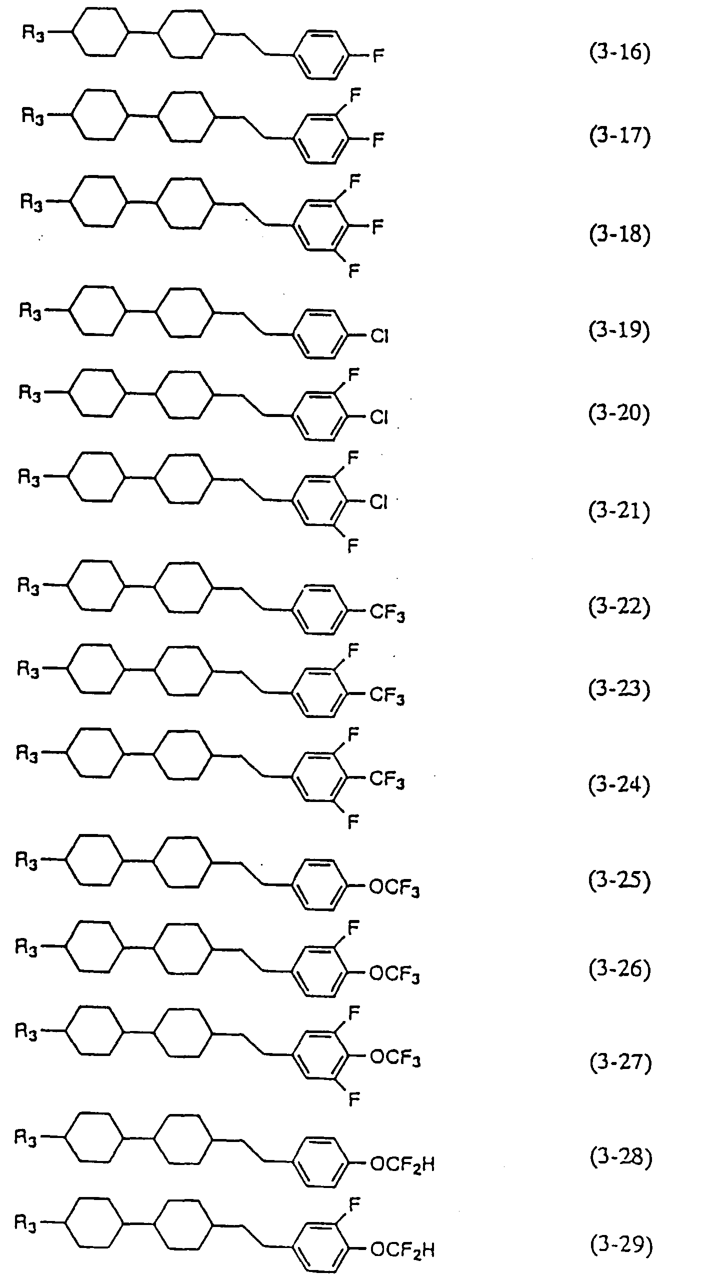 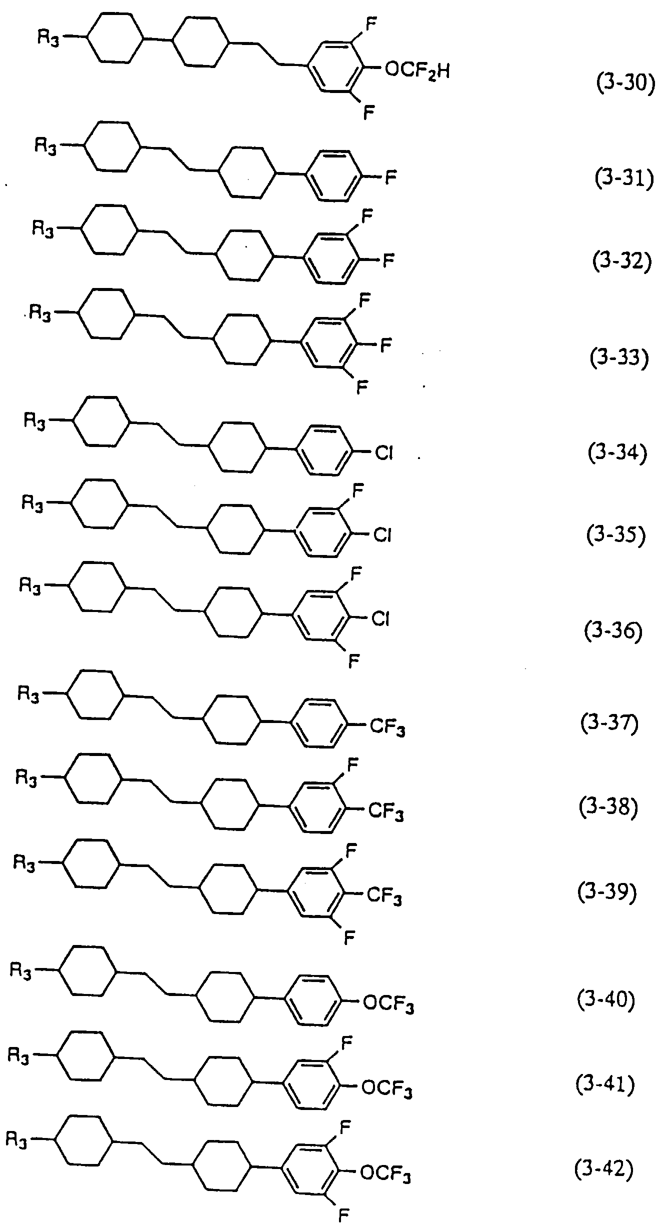 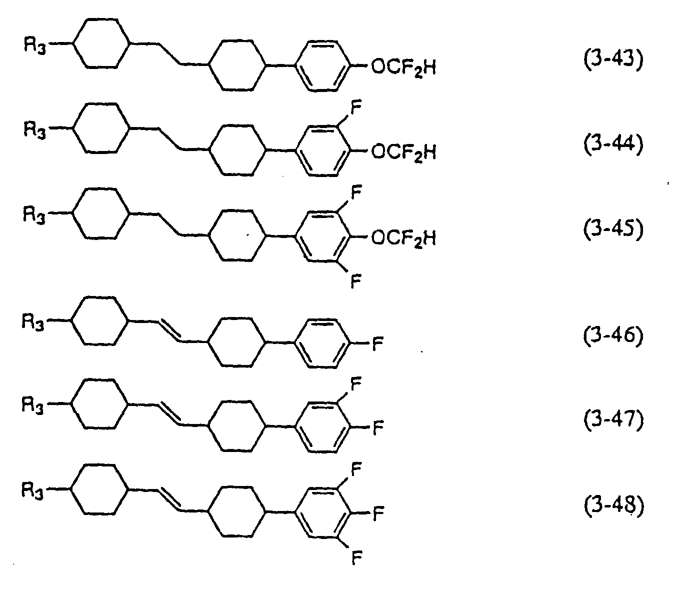 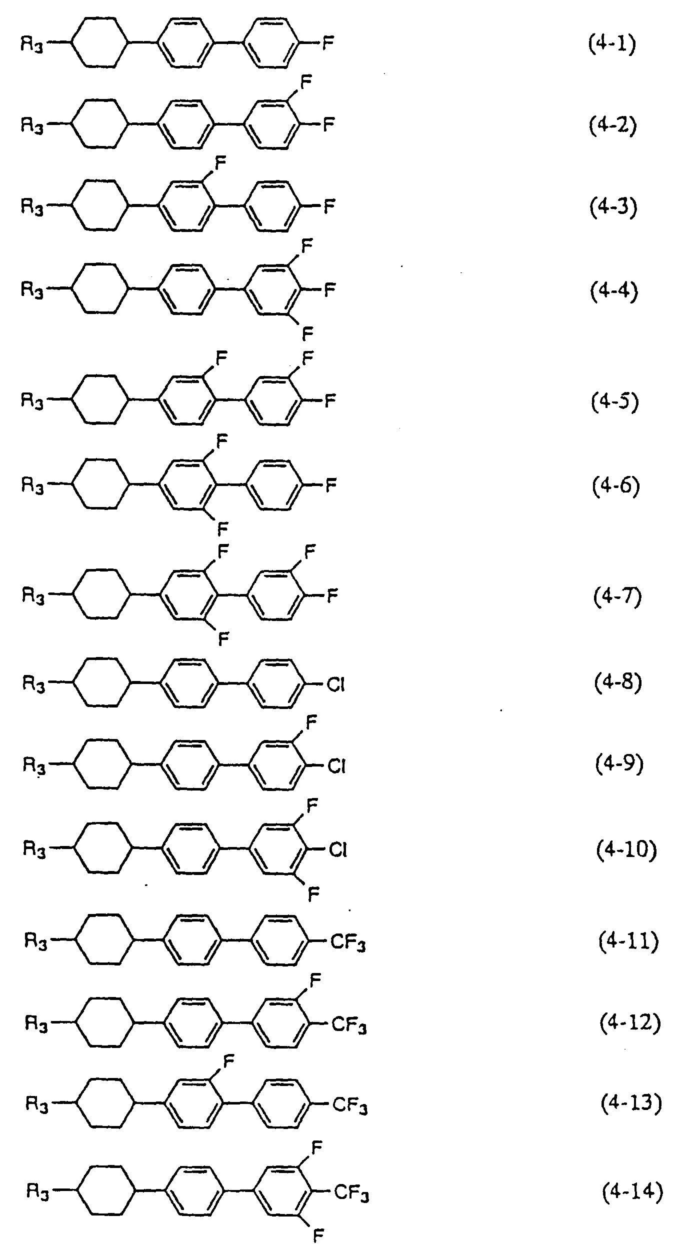 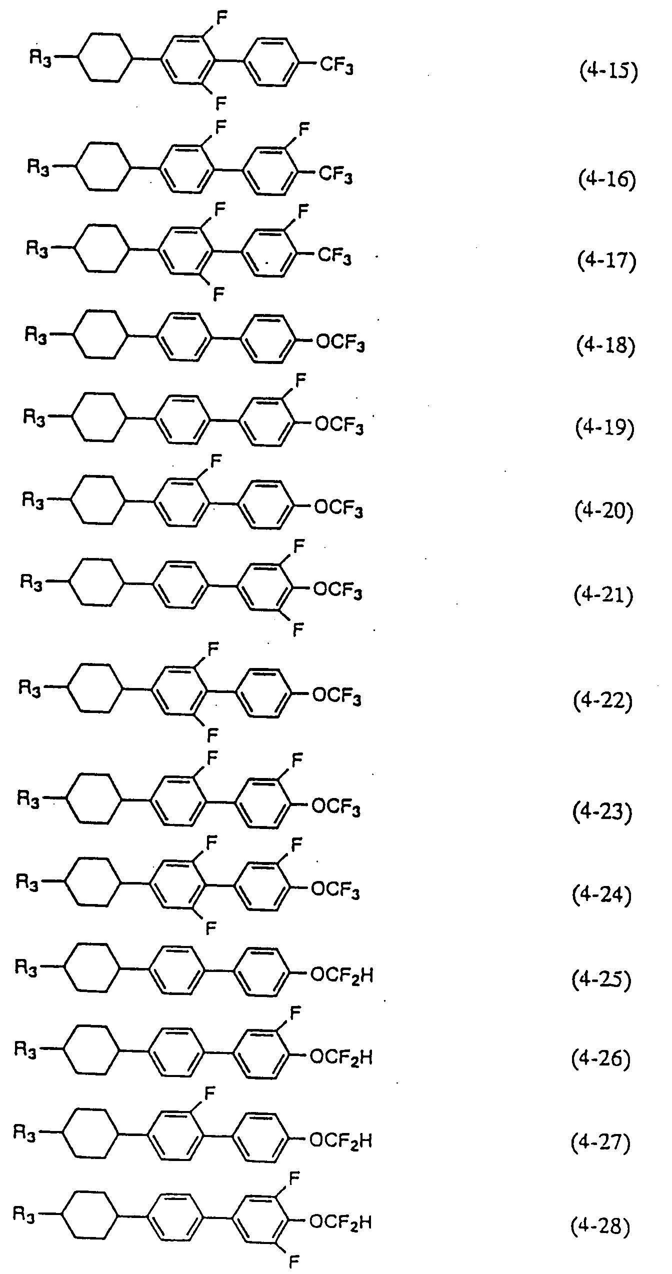 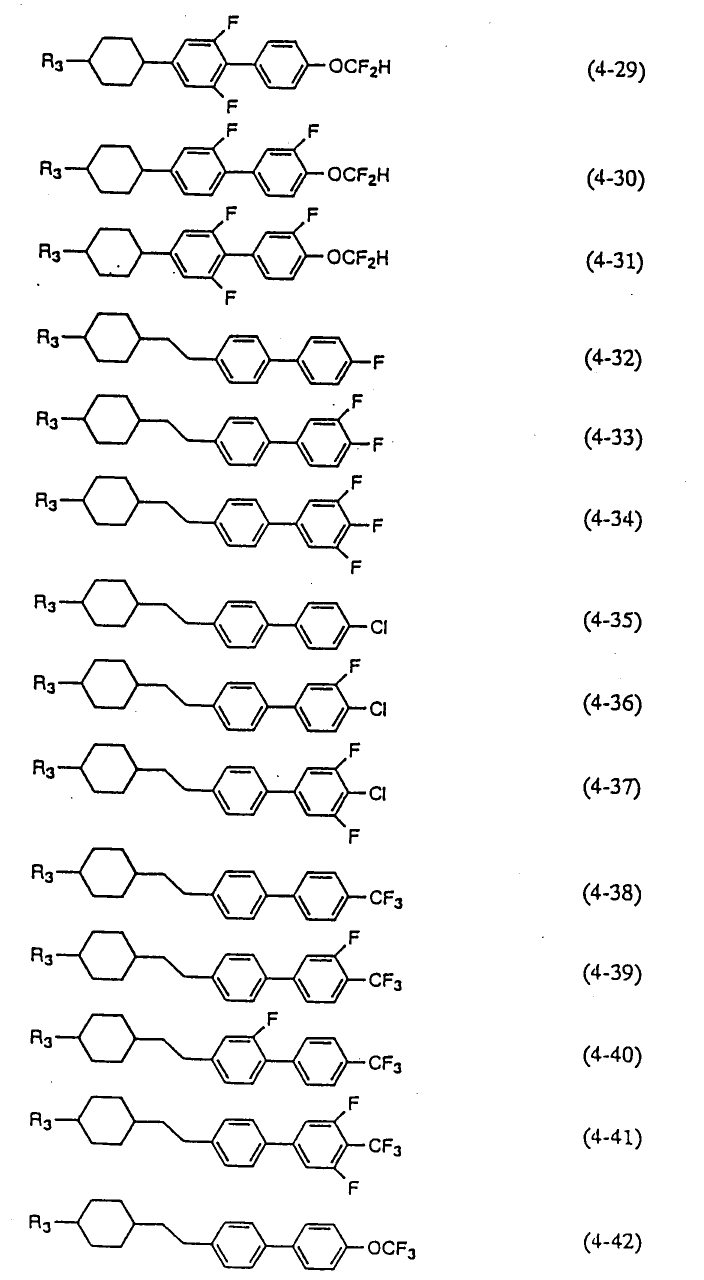 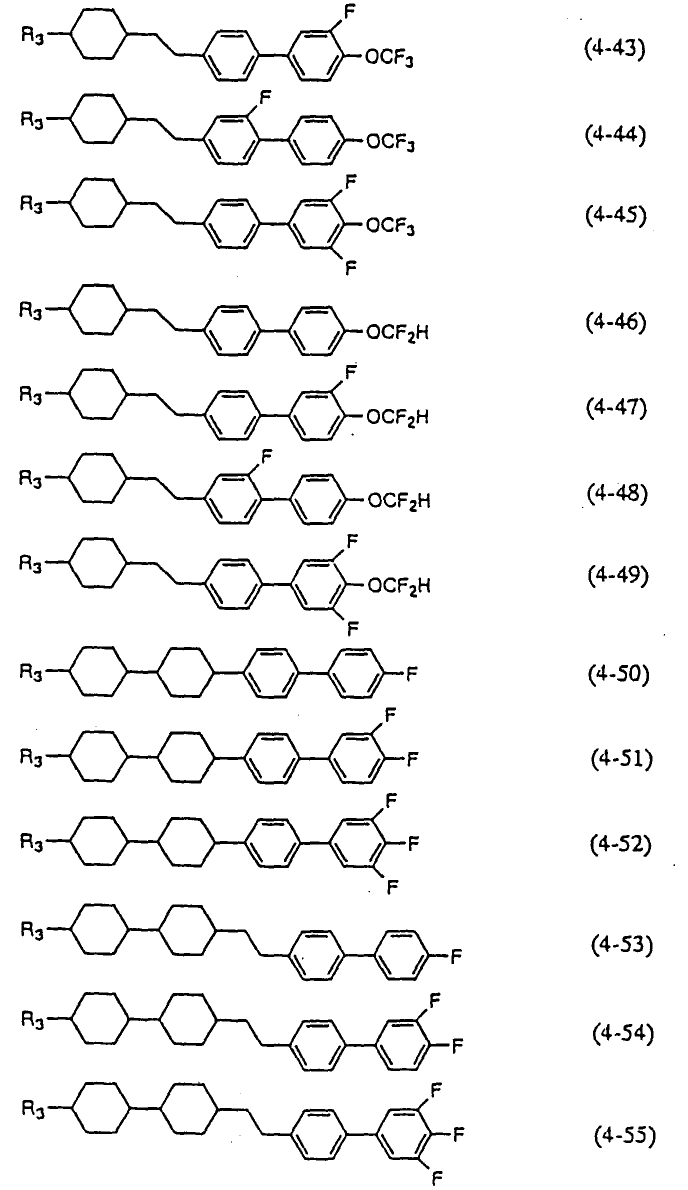 wherein R₃ has the same meaning as described above.

Compounds expressed by one of the formulas (2) to (4) exhibit a positive Δε and are very excellent in heat stability and chemical stability.

Amount of the compounds to be used is suitably in the range of 1 to 99 % by weight, preferably 10 to 97 % by weight, and more desirably 40 to 95 % by weight, based on the total amount of liquid crystal composition.

Next, among the second B component described above, compounds expressed by one of the following formulas (5-1) to (5-24), (6-1) to (6-3), and (7-1) to (7-17) can be mentioned as preferable examples of the compounds included in the general formula (5), (6), or (7): 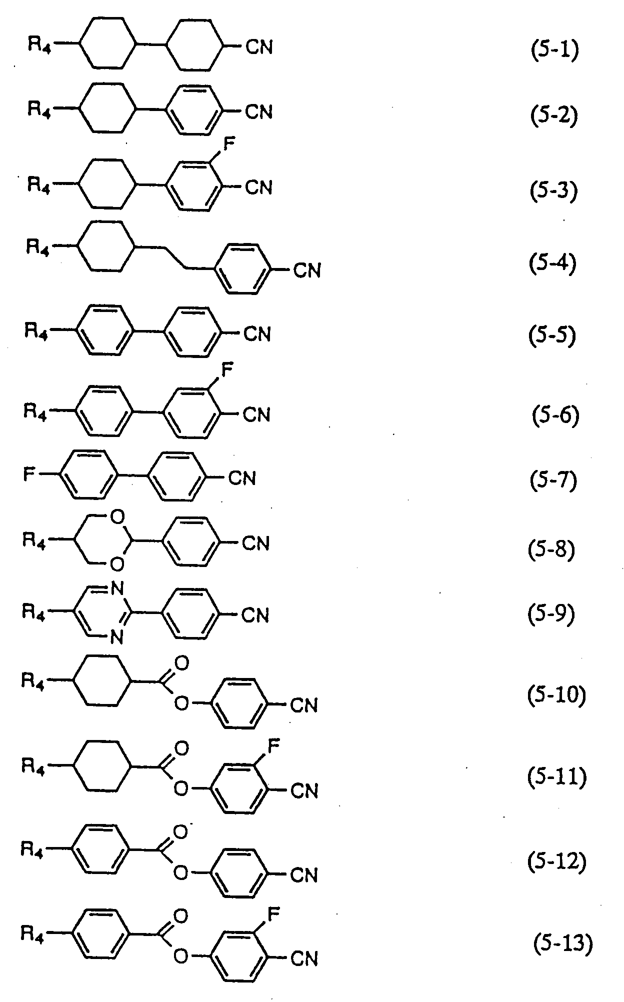 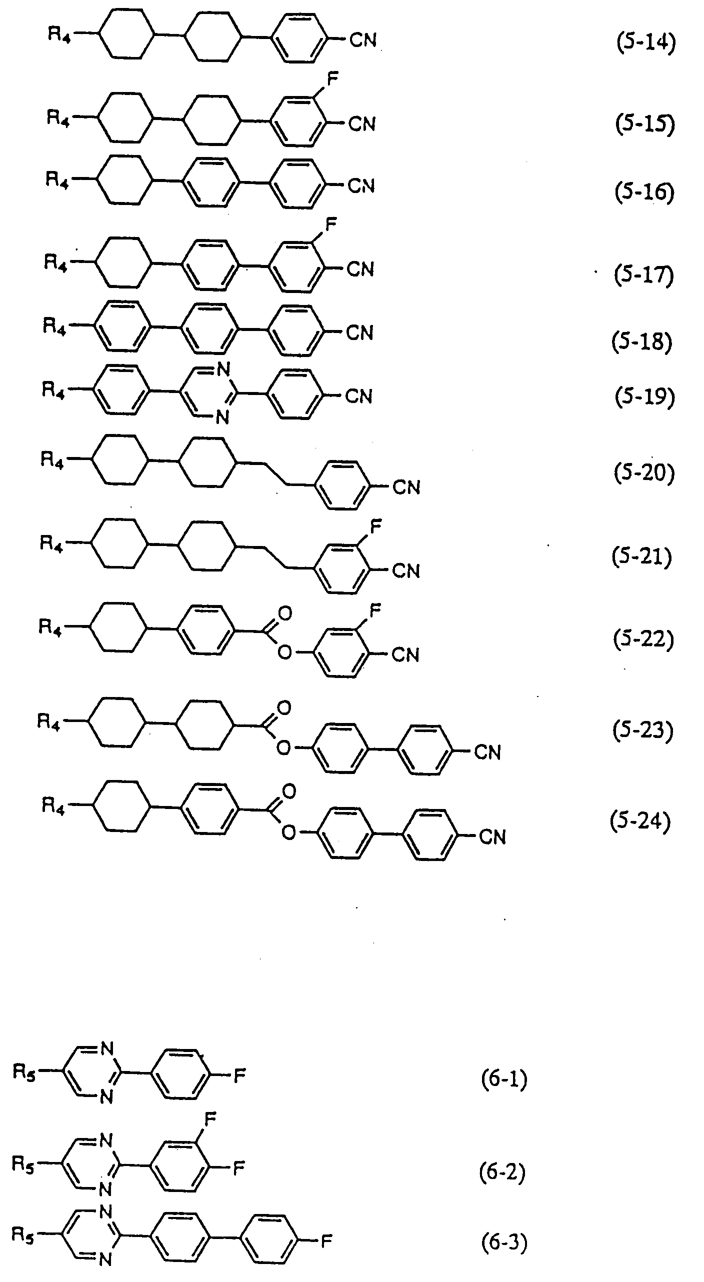 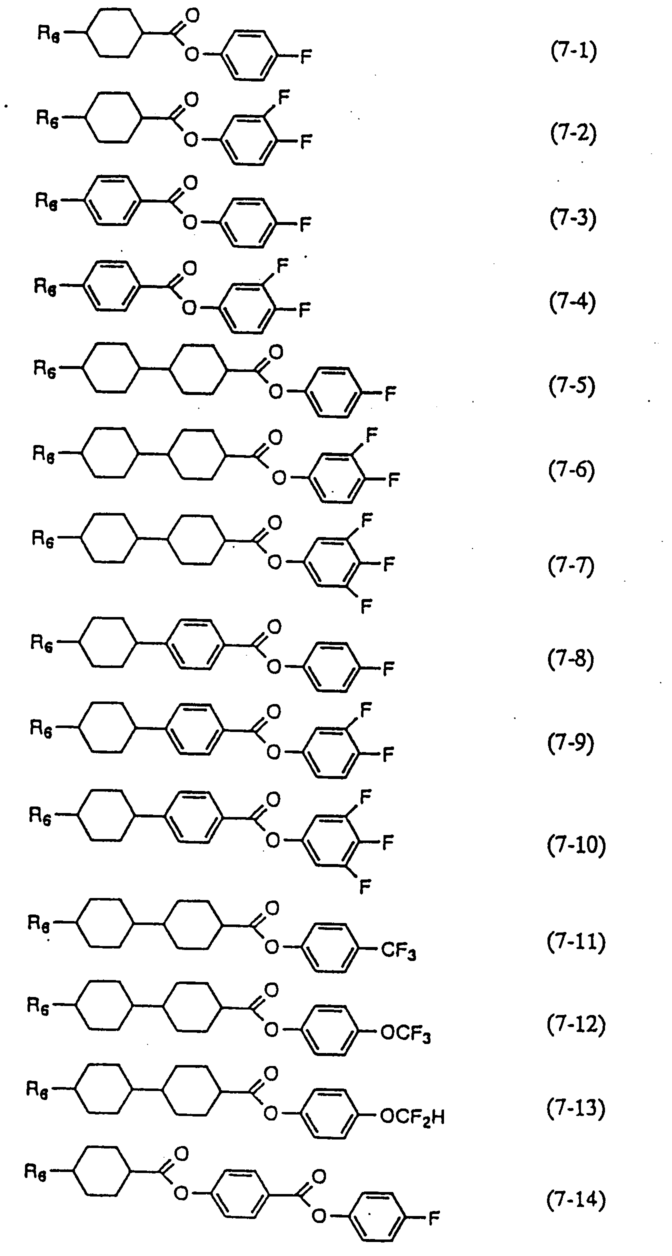 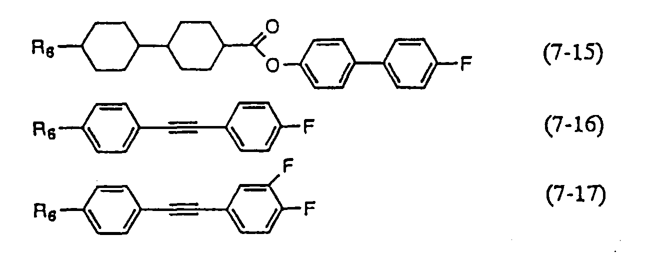 wherein R₄, R₅, R₆ have the same meaning as described above.

Compounds expressed by one of the general formulas (5) to (7) have a large positive value of Δε and are used particularly for the purpose of lowering Vₜₕ.

Also, the compounds are used for the purpose of adjusting viscosity, adjusting Δn, and expanding the temperature range of a liquid crystal phase, and further, for the purpose of improving steepness.

Further; among the second B component, the compounds expressed by one of the following formulas (8-1) to (8-8), and (9-1) to (9-13) can be mentioned as preferable compounds included in the general formula (8) or (9): 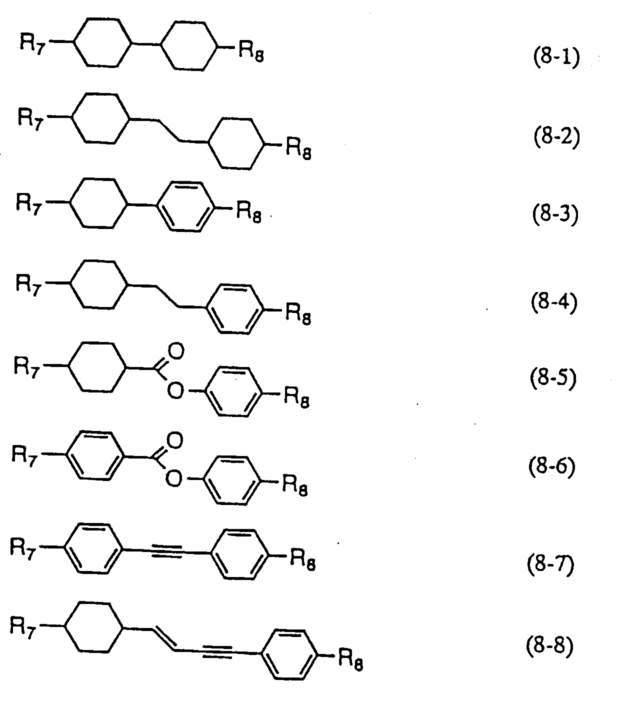 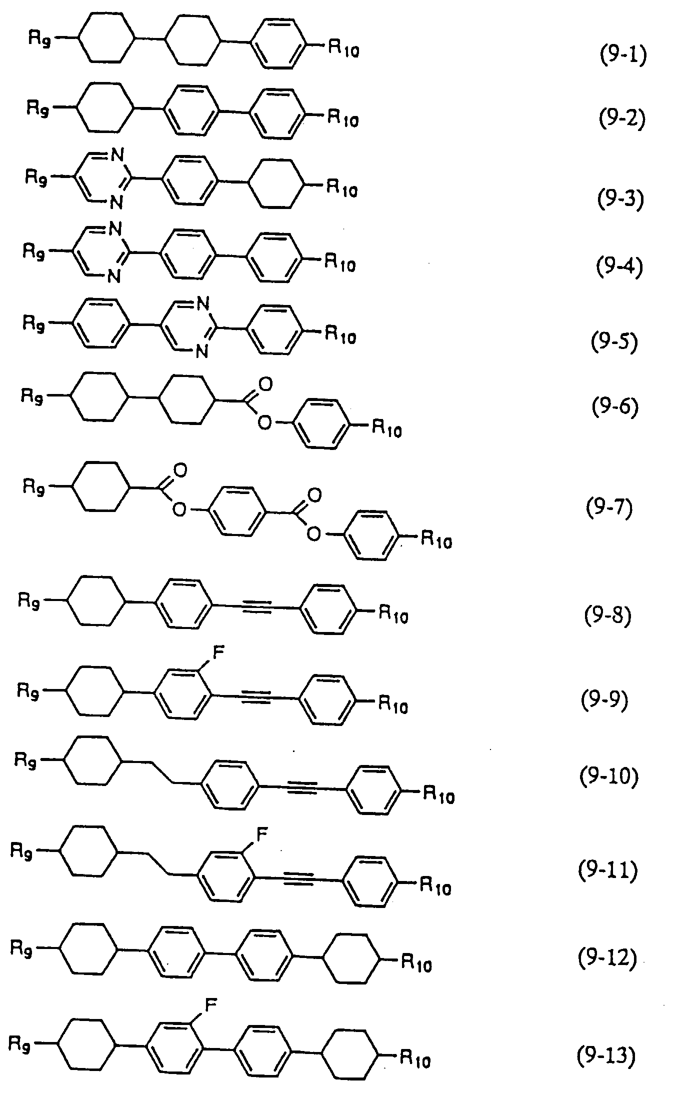 wherein R₇ and R₈ have the same meaning as described above.

Compounds expressed by the general formula (8) or (9) exhibit a negative or a slightly positive Δε, and the former are used for the purpose of mainly reducing viscosity and/or adjusting Δn and the latter are used for the purpose of expanding the nematic range such as raising clearing point and/or for the purpose of adjusting Δn, respectively.

Compounds of the general formulas (5) to (9) are indispensable especially when liquid crystal compositions particularly for STN display mode or ordinary TN display mode are produced.

Amount of the compounds to be used is suitably in the range of 1 to 99 % by weight, preferably 10 to 97 % by weight, and more desirably 40 to 95 % by weight, based on the total amount of liquid crystal composition when liquid crystal compositions for ordinary TN display mode or STN display mode are produced. At that time, the compounds expressed by one of the general formulas (2) to (4) may be used together as a component of the compositions.

Liquid crystal compositions of the present invention can improve the steepness and view angle of display devices when used for TFT liquid crystal display devices.

Besides, the compounds of the present invention expressed by the general formula (1) can improve the response speed of liquid crystal display devices by using the compounds since the compounds have a low viscosity.

Liquid crystal compositions used according to the present invention are produced by methods which are conventional by themselves.

Whereas the compositions are generally produced by dissolving various liquid crystal components with one another at a high temperature, they can also be produced by dissolving liquid crystal components in an organic solvent, mixing the components, and then distilling off the solvent from the mixture under a reduced pressure.

Liquid crystal compositions of the present invention can be improved and optimized depending on their intended uses by adding a suitable additive. Such additives are well known in the art and described in detail in the literature.

For example, a dichroic dye such as merocyanine type, styryl type, azo type, azomethine type, azoxy type, quinophthalone type, anthraquinone type, and tetrazine type can be mentioned, and the compositions can be used as liquid crystal compositions for guest-host (GH) mode by adding such a dichroic dye.

Alternatively, the liquid crystal compositions can be used as NCAP which is prepared by the microencapsulation of a nematic liquid crystal, or as liquid crystal compositions for polymer dispersed liquid crystal display devices (PDLCD) represented by polymer net work liquid crystal display devices (PNLCD) prepared by forming a polymer of three-dimensional reticulated structure in a liquid crystal. Further, the liquid crystal compositions of the present invention can be used as ones for electrically controlled birefringence (ECB) mode or dynamic scattering (DS) mode.

As examples of nematic liquid crystal compositions comprising the compound of the present invention, the following Composition Examples 1 to 13 can be mentioned.

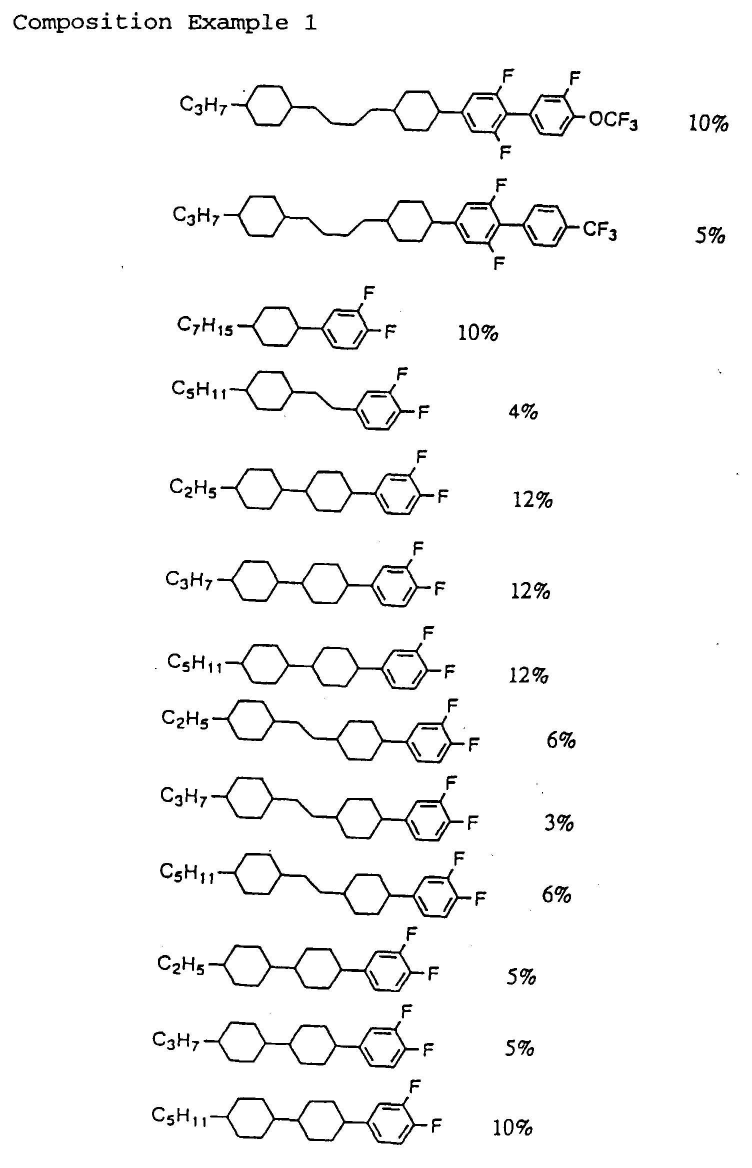

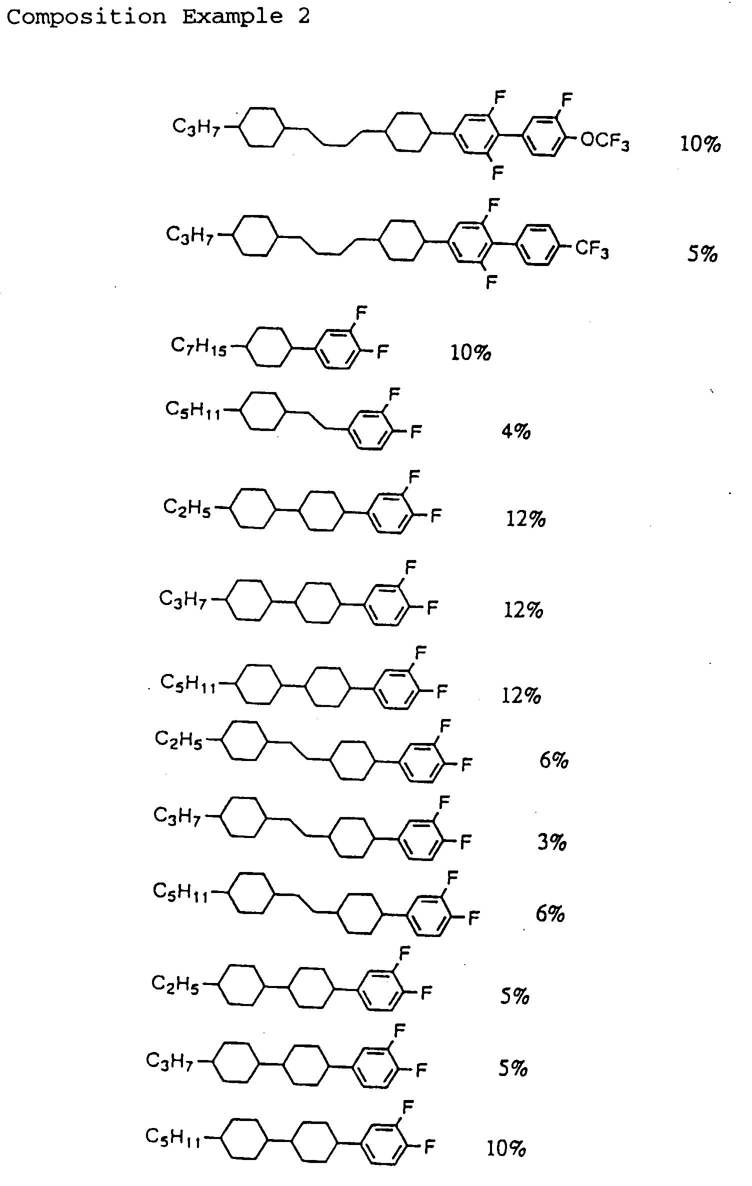

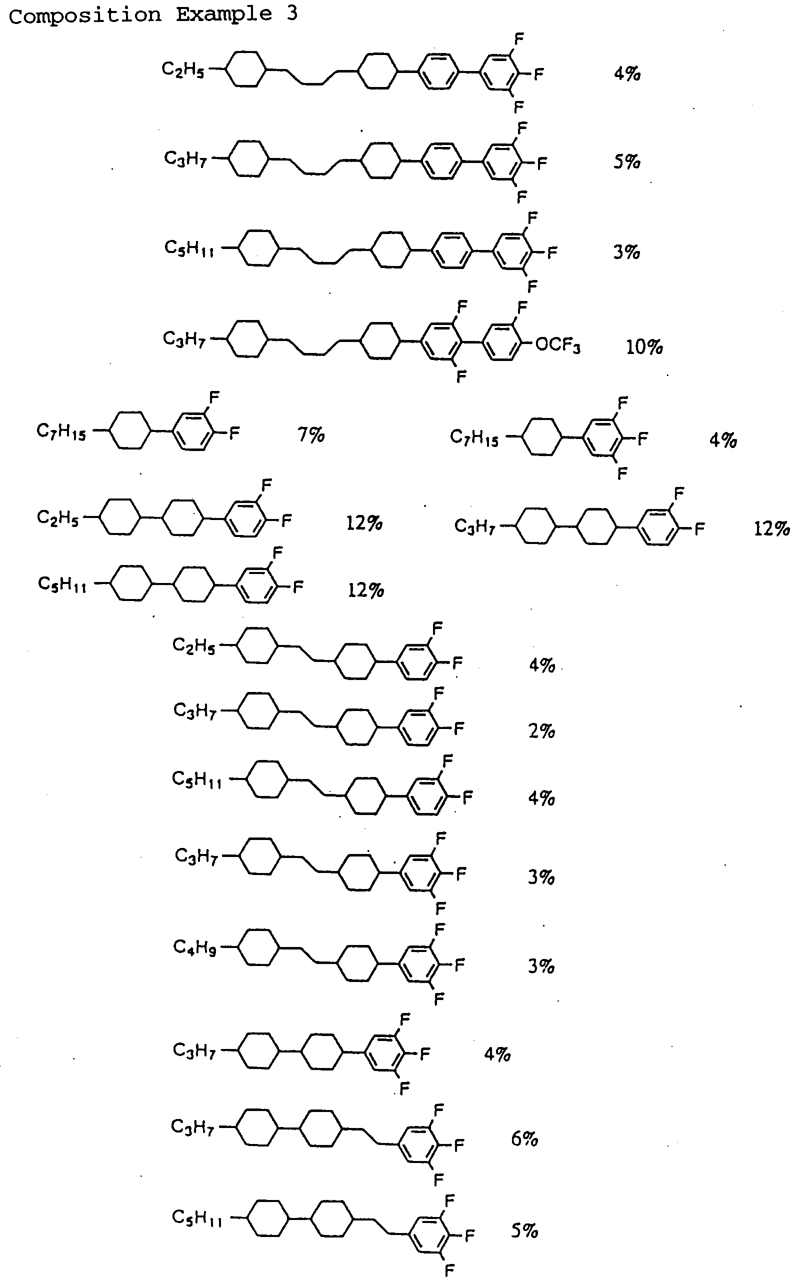

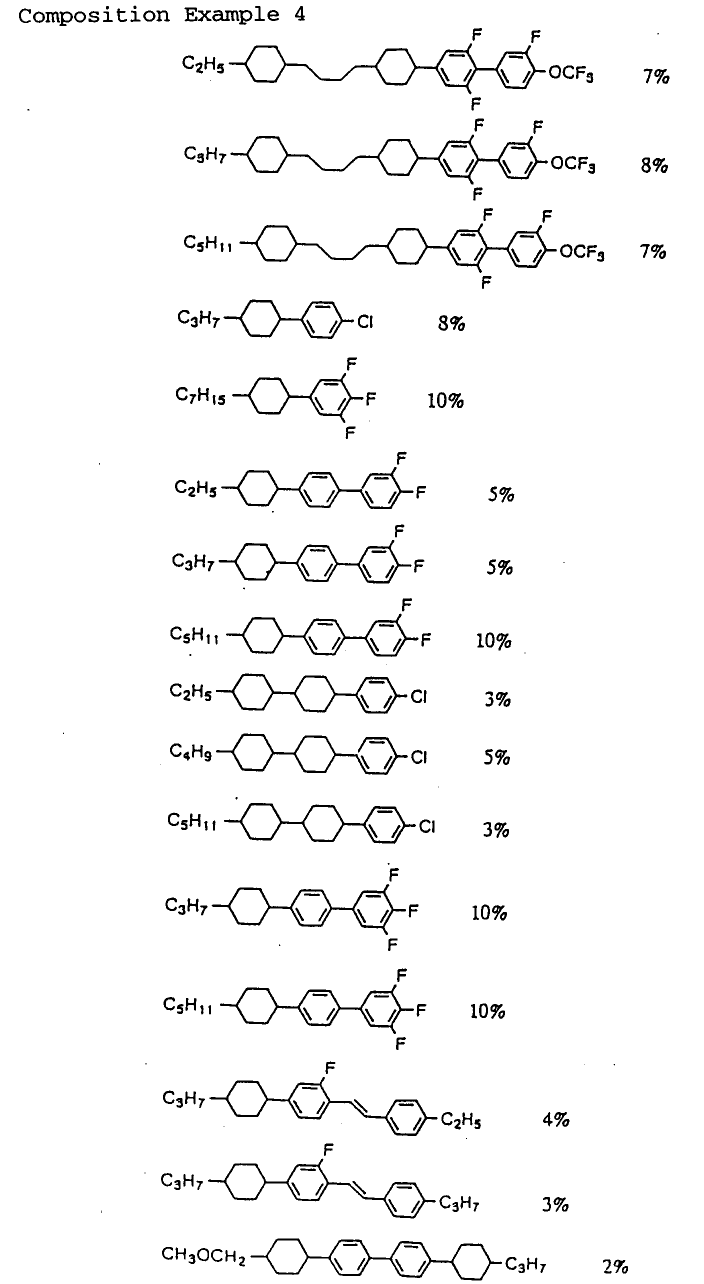

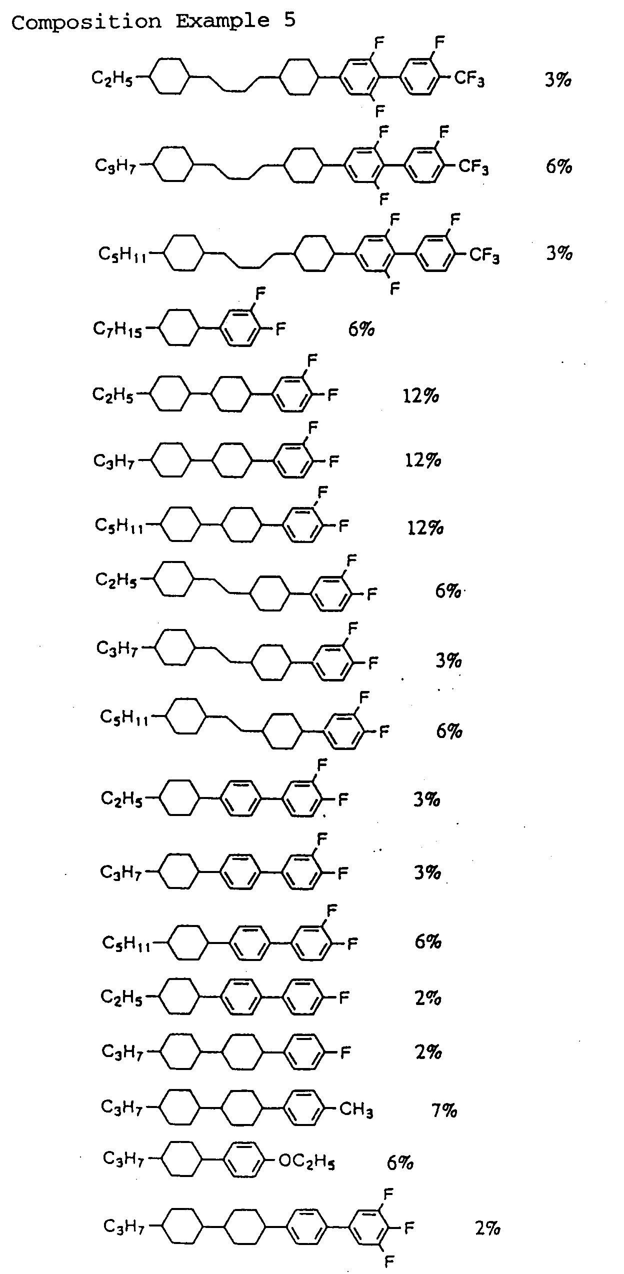

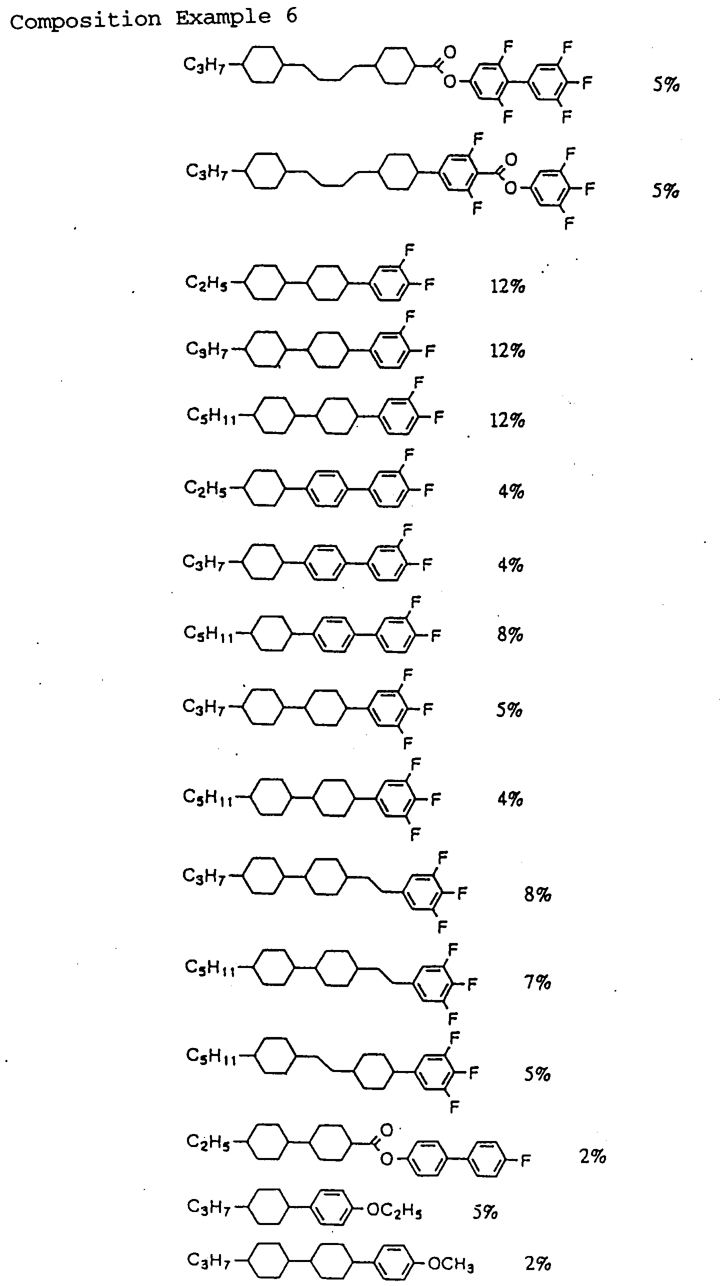

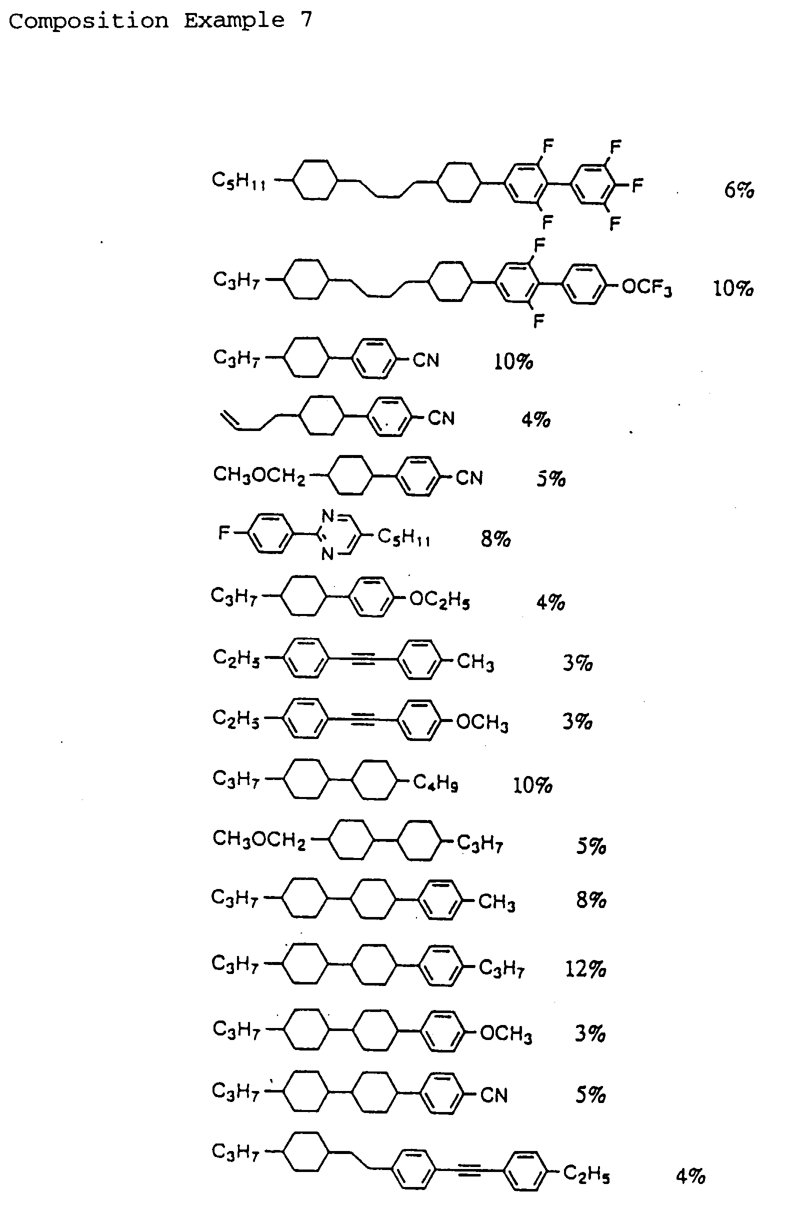

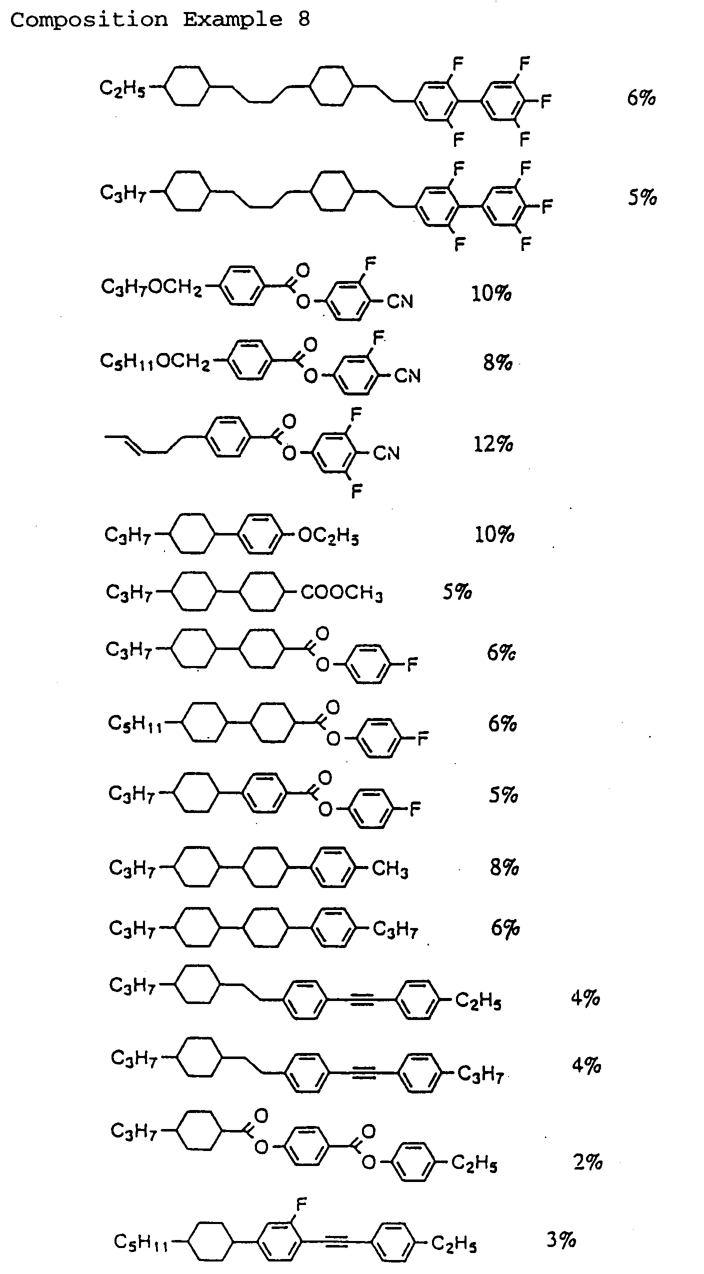

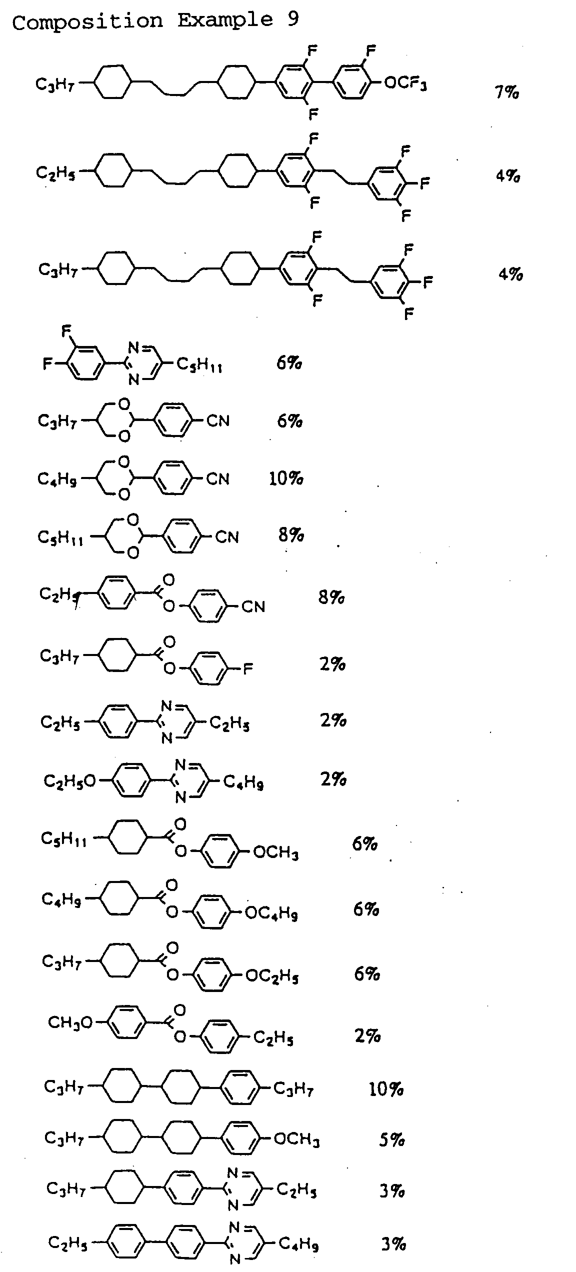

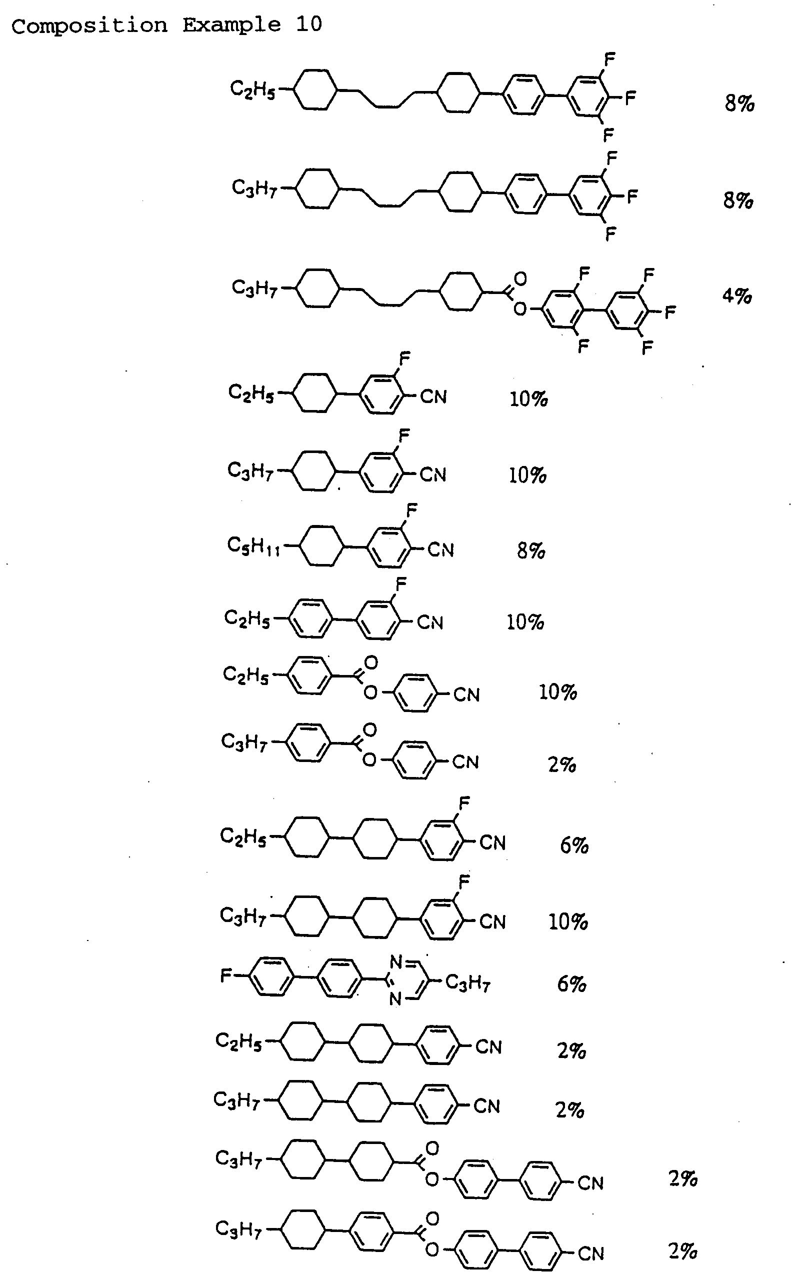

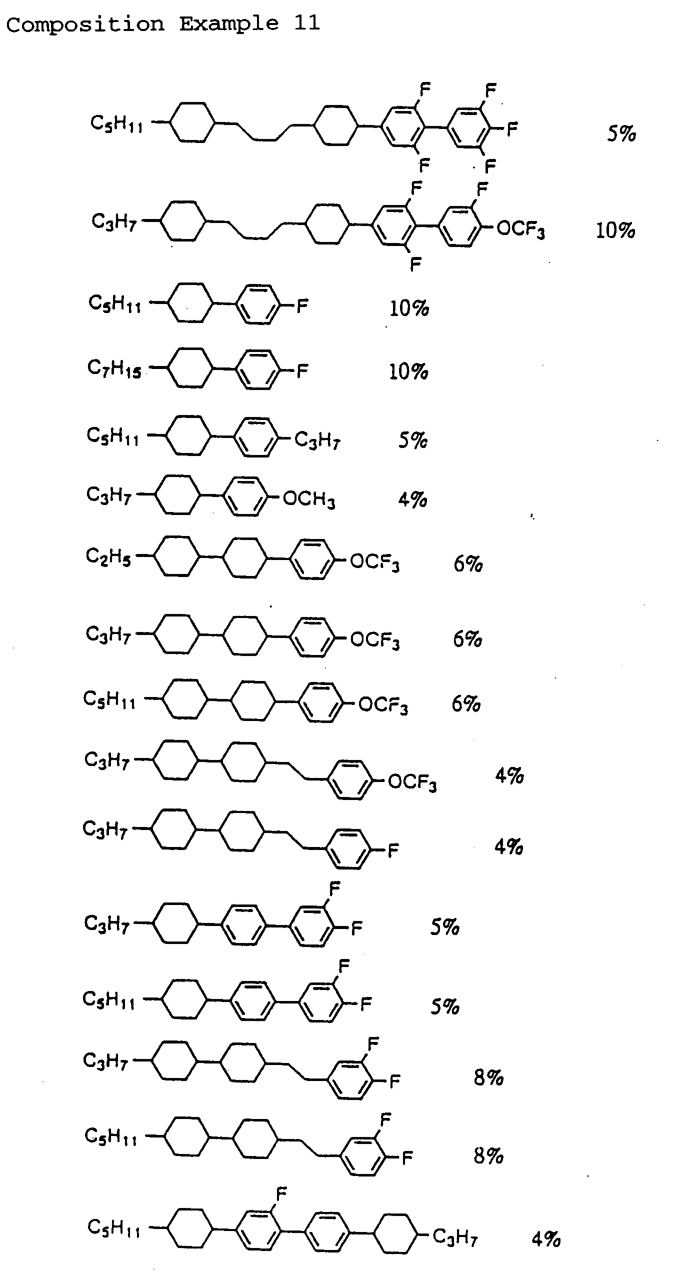

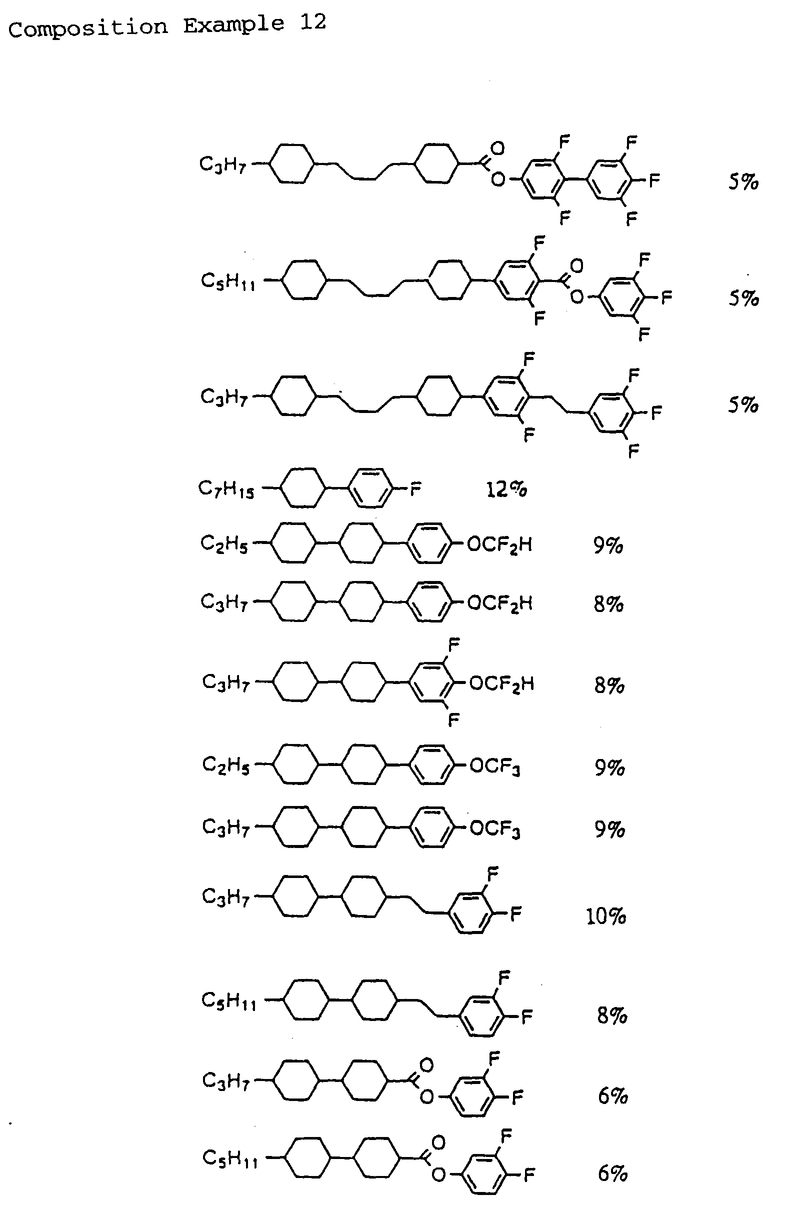

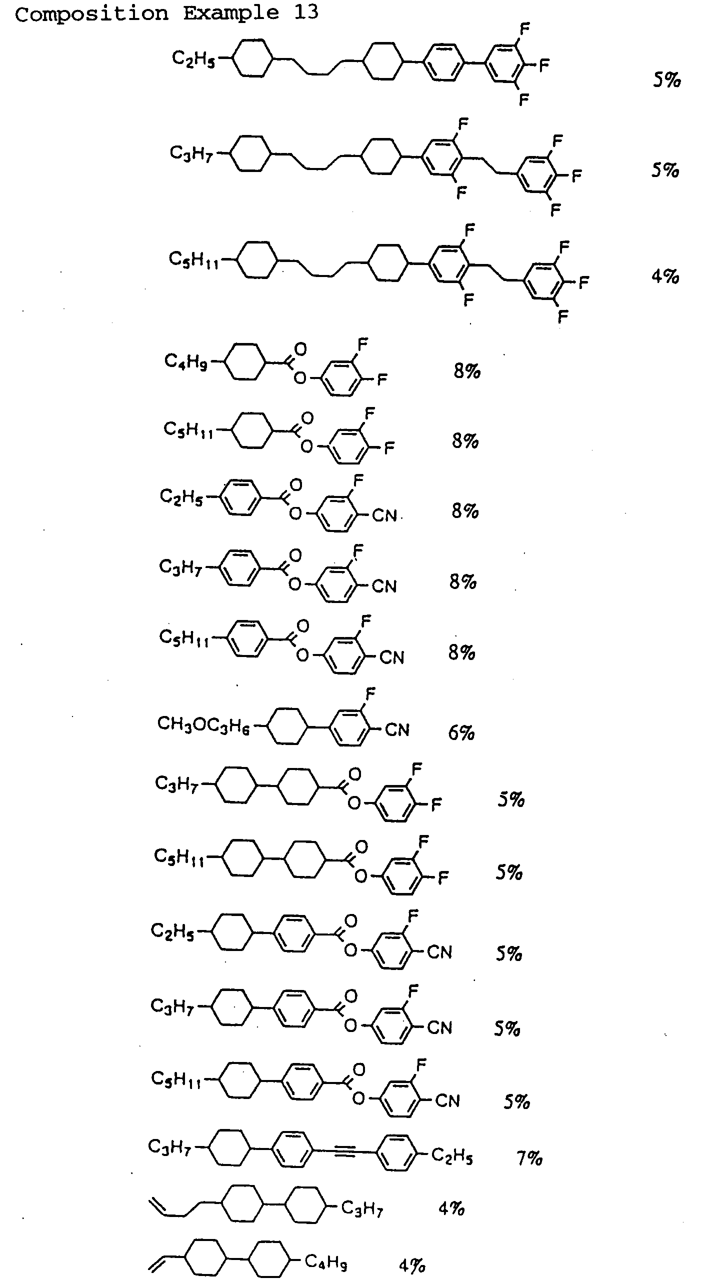

Compounds of the present invention expressed by the general formula (1) can be produced by general methods of organic synthesis known heretofore.

### Production of compounds expressed by the formula (1-a):

Compounds of the formula (1-a) can be produced by the method as follows:

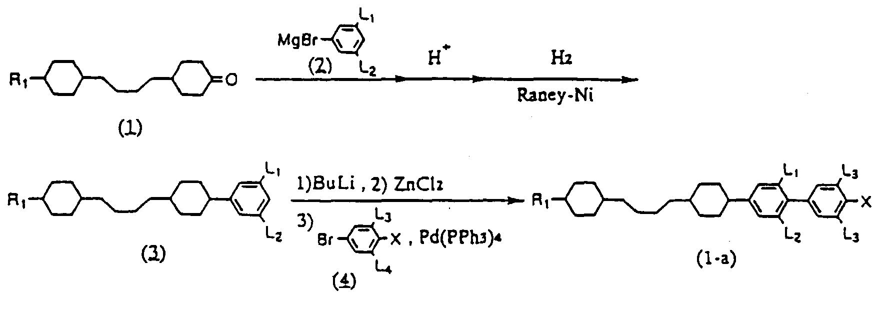

That is, cyclohexanone derivative (1) described in Laid-open Japanese Patent Publication No. Hei 5-310,605 is used as starting material for the synthesis. After Grignard reagent (2) was reacted with the cyclohexanone derivative in an ether type solvent such as tetrahydrofuran (THF) or diethyl ether, the derivative is subjected to a dehydrating treatment in the presence of an acid catalyst, for example, a mineral acid such as hydrochloric acid and sulfuric acid, an organic acid such as acetic acid and p-toluene sulfonic acid; or a non-aqueous ion-exchange resin such as Amberlist, and then subjected to a hydrogenation reduction in the presence of a Raney nickel or a catalyst derived from platinum group metals such as palladium, rhodium and platinum to obtain intermediate (3). This intermediate is reacted with a lithiating agent known in the public, for example, an alkyl lithium to lithiate the position 4 of its phenyl group, reacted with a zinc halide or the like to perform a metal-exchange reaction, and then reacting with compound (4) in the presence of a palladium catalyst such as tetrakistriphenylphosphine palladium to produce objective compound (1-a).

### Production of compounds expressed by the formula (1-b):

Compounds of the formula (1-b) can be produced by the method as follows:

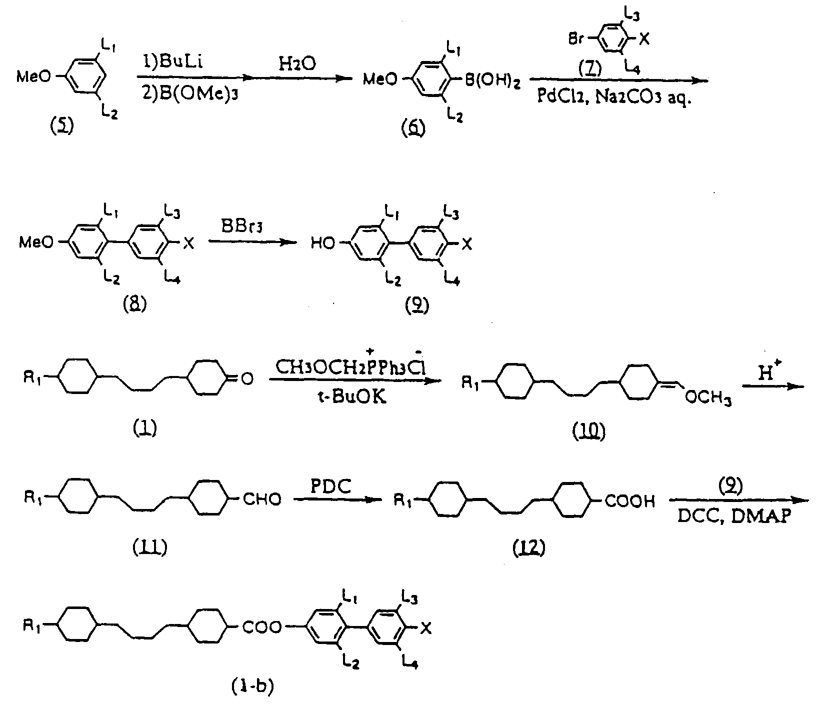

That is, according to a known method (Organic Reactions Vol. 14, Chapter 3, p 270) employing the Wittig reaction, methoxymethyltriphenylphosphonium chloride is reacted with the derivative (1) described above in an ether type solvent such as THF and diethyl ether in the presence of a base such as sodium methylate, potassium-t-butoxide (t-BuOK), and butyl lithium to obtain compound (10). An acid, for example, a mineral acid such as hydrochloric acid and sulfuric acid, or an organic acid such as formic acid, acetic acid, and p-toluene sulfonic acid is reacted with the compound (10) to produce aldehyde derivative (11). Subsequently, the derivative (11) is oxidized with an oxydizing agent such as pyridinum dichromate (hereinafter abbreviated as PDC), Jones reagent (cf. Jikken Kagaku Kouza (Course of Chemical Experiment), Fourth edition, Vol. 23, Chapter 2 (Maruzen)), and an activated manganese dioxide to obtain carboxylic acid derivative (12).

On the other hand, anisole derivative (5) is lithiated with an alkyl lithium or the like, and then the product is reacted with trimethyl borate and water in turn to obtain boric acid derivative (6). Subsequently, the derivative (6) is reacted with compound (7) in the presence of a base such as sodium carbonate and a palladium catalyst to obtain compound (8), and then the compound (8) is subjected to a demethylation with boron tribromide to obtain compound (9).

The compound (9) is reacted with the carboxylic acid derivative (12) described above in the presence of N,N-dicyclohexylcarbodiimide (hereinafter abbreviated as DCC) and 4-dimethylaminopyridine (hereinafter abbreviated as DMAP) to obtain objective compound (1-b).

### Production of compounds expressed by the formula (1-c):

Compounds of the formula (1-c) can be produced by the method as follows:

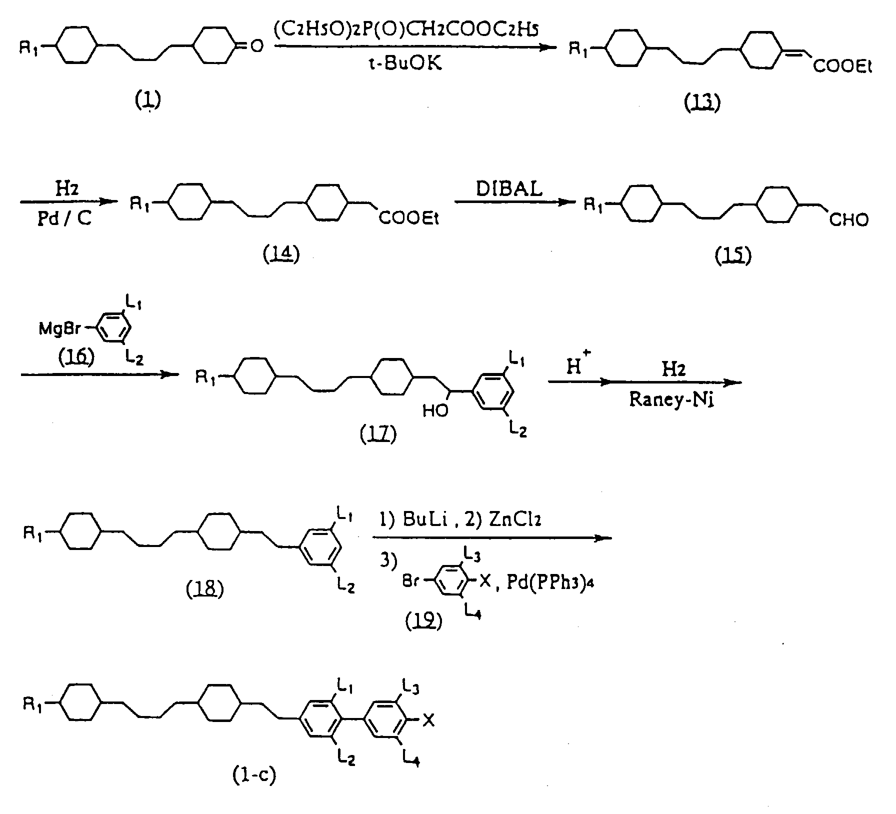

That is, the cyclohexanone derivative (1) described above is subjected to the Wittig-Horner reaction (M. Fieser et al., Reagent for Organic Synthesis, Wiley, 1, 1217) to convert it into compound (13), subjected to a hydrogenation reduction in the presence of a palladium-carbon catalyst to convert into compound (14), and then reduced with diisobutylaluminum hydride (hereinafter referred to as DIBAL) to obtain aldehyde derivative (15).

This derivative (15) is reacted with Grignard reagent (16) to obtain compound (17), and the compound (17) is subjected to a dehydrating reaction and hydrogenation reduction in turn to obtain compound (18).

This compound (18) is reacted with a lithiating agent known in the public, for example, an alkyl lithium to lithiating the position 4 of the phenyl group, then subjected to a metal-exchange reaction by reacting, for example, with a zinc halide or the like, and continuously reacted with compound (19) in the presence of a palladium catalyst such as tetrakistriphenylphosphine palladium to produce objective compound (1-c) as an example.

### Production of compounds expressed by the formula (1-d):

Compounds of the formula (1-d) can be produced by the method as follows:

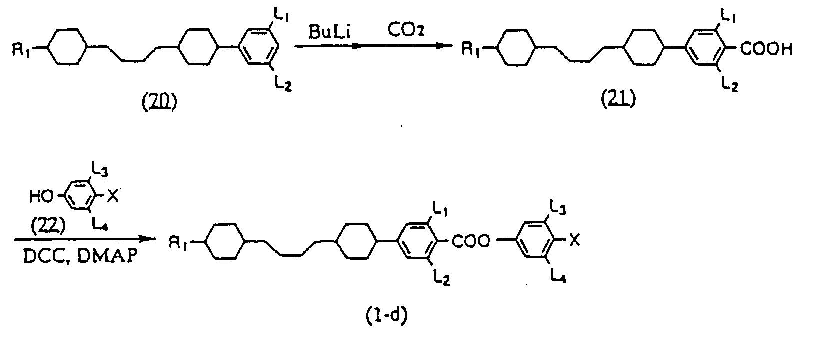

That is, after compound (20) described in the Laid-open Japanese Patent Publication No. Hei 5-310,605 mentioned above was lithiated at the position 4 of the phenyl group with butyl lithium, the lithiated compound is reacted with carbon dioxide to obtain carboxylic acid derivative (21). Subsequently, this derivative can be reacted with compound (22) in the presence of DCC and DMAP to obtain objective compound (1-d).

### Production of compounds expressed by the formula (1-e):

Compounds of the formula (1-e) can be produced by the method as follows:

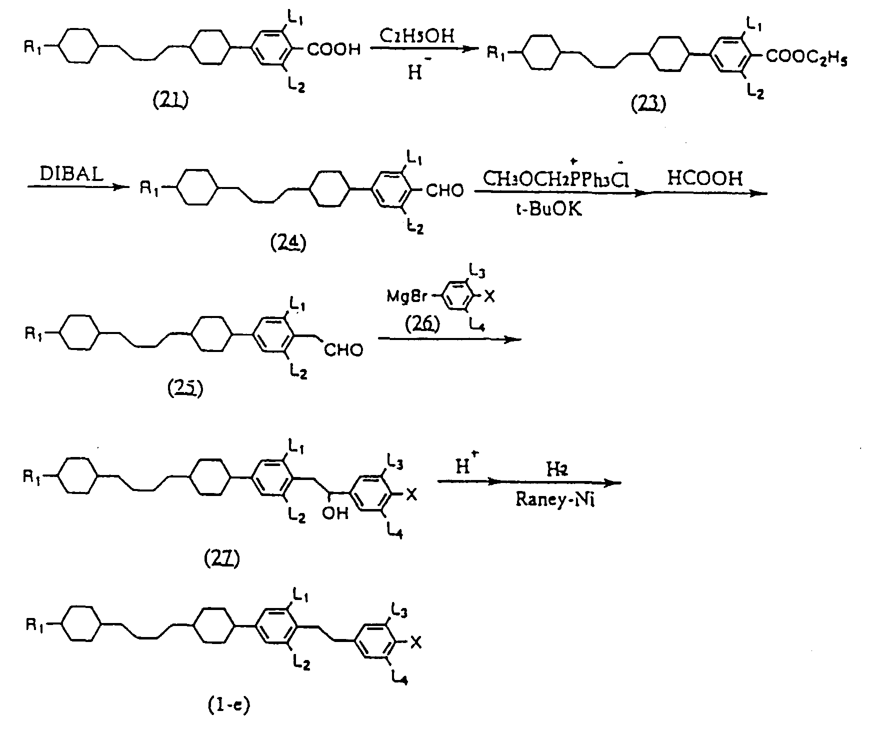

That is, after the carboxylic acid derivative (21) was converted into ester derivative (23), the ester derivative is reduced with DIBAL to obtain aldehyde derivative (24). Subsequently, the aldehyde derivative is subjected to the Wittig reaction, that is, reacted with methoxymethyltriphenylphosphonium chloride in an ether type solvent such as THF and diethyl ether in the presence of a base selected from sodium methylate, t-BuOK, and butyl lithium, and then subjected to a reaction with a mineral acid such as hydrochloric acid and sulfuric acid, or an organic acid such as formic acid, acetic acid, and p-toluene sulfonic acid to obtain aldehyde derivative (25).

Subsequently, the aldehyde derivative (25) can be reacted with Grignard reagent (26) to obtain compound (27), and then subjected to a dehydration and hydrogenation reduction in turn to produce objective compound (1-e) as an example.

Compounds expressed by one of the formulas (1-f) to (1-k) can be produced with reference to the methods for producing the compounds expressed by one of the formulas (1-a) to (1-e) described above, or produced by using the methods described above in combination with selected other known reaction procedures, when necessary.

Any liquid crystalline compounds (1) thus obtained are remarkably excellent as a component of nematic liquid crystal compositions suitable for either STN and TFT display mode, since the compounds exhibit a wide temperature range of a liquid crystal phase, have a low Vₜₕ due to a high Δε of the compounds, have a high VHR, and can readily be mixed with other liquid crystal materials.

Now, the present invention will be described in more detail with reference to Examples. However, it should be understood that the present invention is by no means restricted by such specific Examples.

In each of the Examples, structure of the compounds was confirmed by nuclear magnetic resonance spectrum (hereinafter abbreviated as ¹H-NMR) and mass spectrum (hereinafter abbreviated as MS).

In the former, d indicates doublet, m does multiplet, and J indicates coupling constant; and in the latter, M⁺ indicates molecular ion peak. Cr indicates a crystal phase, S_{B} a smectic B phase, N a nematic phase, and Iso indicates the phase of an isotropic liquid; and all phase transition temperatures are expressed by °C.

### Example 1

Preparation of 3,4,5-trifluoro-4'-(trans-4-(4-(trans-4-propylcyclohexyl)butyl)cyclohexyl)biphenyl (Compound expressed by the general formula (1) wherein R₁ represents C₃H₇, both Z₁ and Z₂ represent a covalent bond, both L₁ and L₂ represent H, and all of L₃, L₄, and X represent F: Compound No. 11)

Under nitrogen gas stream, 3.77 g (155 mmol) of magnesium was heated to dry for 30 min, and then 30 ml of THF was added thereto. To the mixture was added dropwise a solution of 20.3 g (129 mmol) of bromobenzene in 50 ml of THF so that a temperature of 50 to 60°C was maintained and stirred for 30 min. After allowed to cool, a solution of 30.0 g (108 ml) of 4-(trans-4-propylcyclohexyl)butylcyclohexanone in 100 ml of THF was added dropwise at room temperature and stirred for 1.5 hours. After the reaction product was put in 200 ml of an ice water to terminate the reaction, 30 ml of 6N-hydrochloric acid was added and then the product was extracted with 400 ml of toluene.

After the organic layer was washed with water thrice and dried over anhydrous magnesium sulfate, the solvent was distilled off under a reduced pressure. The concentrated residue thus obtained was dissolved in 300 ml of toluene in a 500 ml eggplant type flask provided with a Dean and Stark dehydrating tube, and 2.00 g of Amberlist was added and heated to reflux for 2 hours. After cooled down to room temperature, the Amberlist was filtered off, the solvent was distilled off under a reduced pressure, and then, the concentrated residue was subjected to silica gel column chromatography (eluent: heptane) and recrystallized from heptane to obtain 24.7 g (73.0 ml) of (4-(4-(trans-4-propylcyclohexyl)butyl) cyclohexenyl)benzene.

This product was dissolved in 200 ml of a mixed solvent of toluene/Solmix (1/1), 4.00 g of Raney nickel was added thereto and stirred under hydrogen gas atmosphere of 1 to 2 kg/cm² at room temperature. When the amount of consumed hydrogen reached 1700 ml, the stirring was discontinued. After Raney nickel was removed, the solvent was distilled off under a reduced pressure, and the residue was subjected to silica gel chromatography (eluent: toluene) and recrystallized from heptane to obtain 23.9 g (70.2 mmol) of (trans-4-(4-(trans-4-propylcyclohexyl)butyl)cyclohexyl)benzene.

After 20.0 g (58.7 mmol) of this product was dissolved in 800 ml of THF and cooled down to -30°C under nitrogen gas stream, a solution of 1.6 M of n-butyl lithium in 44.0 ml (70.4 mmol) of n-hexane was added dropwise thereto and stirred for 1 hour. Subsequently, the solution was cooled down to -50°C, and then a solution of 0.5 M of zinc chloride in 141 ml (70.5 mmol) of THF was added dropwise while maintaining the same temperature, stirred for 30 min, warmed up to room temperature, and stirred at room temperature for 1 hour. Then, 2.43 g (2.11 mmol) of tetrakistriphenylphosphine palladium (O) was added thereto and 14.9 g (70.6 mmol) of 1-bromo-3,4,5-trifluorobenzene was added dropwise. After finishing of the dropping, the mixture was heated to reflux for 3.5 hours and allowed to cool, and then 400 ml of water was added to the reaction product to terminate the reaction.

After 60 ml of 2N-hydrochloric acid was added to the solution of the reaction product, the product was extracted with 400 ml of toluene. The organic layer was washed with 200 ml of a saturated aqueous solution of sodium bicarbonate and 200 ml of water thrice, respectively, and then dried over anhydrous magnesium sulfate. The solvent was distilled off under a reduced pressure, and the residue was subjected to silica gel chromatography (eluent: toluene) to obtain a crude 3,4,5-trifluoro-4'-(trans-4-(4-(trans-4-propylcyclohexyl)butyl) cyclohexyl)biphenyl.

This product was recrystallized from heptane to obtain 11.1 g (23.6 mmol) of the subject compound.
Phase transition temperature: Cr 94.3 N 169.4 Iso
NMR: δ=0.40∼2.80 (35H, m), 7.16 (2H, dd, J=9.3, 6.7 Hz), 7.27 (2H, d, J=8.5 Hz), 7.43 (2H, d, J=8.5 Hz)
MS: m/e=470 (M⁺)

### Example 2

Preparation of 3,4,5,2',6'-pentafluoro-4'-(trans-4-(4-(trans-4-pentylcyclohexyl)butyl)cyclohexyl)biphenyl (Compound expressed by the general formula (1) wherein R₁ represents C₅H₁₁, both Z₁ and Z₂ represent a covalent bond, and all of L₁, L₂, L₃, L₄, and X represent F: Compound No. 18)

After 3.45 g (142 mmol) of magnesium was heated to dry under nitrogen gas stream for 30 min, it was suspended in 30 ml of THF and stirred for 30 min while adding dropwise a solution of 22.8 g (118 mmol) of 1-bromo-3,5-difluorobenzene in 50 ml THF so that a temperature of 50 to 60°C was kept. After allowed to cool, a solution of 30.0 g (98.5 mmol) of 4-(trans-4-pentylcyclohexyl) butylcyclohexanone in 100 ml of THF was added dropwise thereto and stirred for 1.5 hours while maintaining it on a water bath at 60°C. After the reaction product was put in 200 ml of an ice water to terminate the reaction, 30 ml of 6N-hydrochloric acid was added thereto, and the product was extracted with 400 ml of toluene. The organic layer was washed with 200 ml of water thrice and then dried over anhydrous magnesium sulfate. After the solvent was distilled off under a reduced pressure, the product was dissolved in 300 ml of toluene, and 1.80 g of Amberlist was added thereto and heated to reflux for 2 hours. The Amberlist was filtered off, the solvent was distilled off under a reduced pressure, and the residue was subjected to silica gel column chromatography (eluent: heptane), and recrystallized from heptane to obtain 28.2 g (70.0 mmol) of 1,3-difluoro-5-(4-(4-(trans-4-pentylcyclohexyl)butyl) cyclohexenyl)benzene.

This product was dissolved in 200 ml of a mixed solvent of toluene/Solmix (1/1), and 4.00 g of Raney nickel was added thereto and stirred at room temperature under a hydrogen gas pressure of 1 to 2 kg/cm². When the amount of consumed hydrogen reached 1550 ml, the stirring was discontinued, and the Raney nickel was filtered off. The solvent was distilled off under a reduced pressure, and the residue was subjected to silica gel column chromatography (eluent: toluene) and recrystallized from heptane to obtain 11.9 g (29.4 mmol) of 1,3-difluoro-5-(trans-4-(4-(trans-4-pentylcyclohexyl)butyl)cyclohexyl)benzene.

After 10.0 g (24.7 mmol) of this product was dissolved in 400 ml of THF, the solution was cooled down to -30°C under nitrogen gas stream. Then, a solution of 1.6 M of n-butyl lithium in 18.5 ml (29.8 mmol) of n-hexane was added dropwise to the solution while maintaining the same temperature and stirred for 1 hour. After cooled down to -50°C, a solution of 0.5 M of zinc chloride in 59.2 ml (29.6 mmol) of THF was added dropwise to the solution, and stirred for 30 min, warmed up to room temperature, and then further stirred at room temperature for 1 hour.

To this solution was added 1.00 g (0.868 mmol) of tetrakistriphenylphosphine palladium (O), and 6.25 g (29.8 mmol) of 1-bromo-3,4,5-trifluorobenzene was further added dropwise. After finishing of the dropping, the solution was heated to reflux for 3.5 hours and allowed to cool, and then 200 ml of water was added to the reaction product to terminate the reaction. To the solution of the product was added 30 ml of 2N-hydrochloric acid, and the product was extracted with 200 ml of toluene. The organic layer was washed with 200 ml of a saturated aqueous solution of sodium bicarbonate and 200 ml of water each thrice in turn, and then dried over anhydrous magnesium sulfate. The solvent was distilled off under a reduced pressure, and the residue was subjected to silica gel column chromatography (eluent: toluene) to obtain a crude 3,4,5,2',6'-pentafluoro-4'-(trans-4-(4-(trans-4-pentylcyclohexyl)butyl) cyclohexyl)biphenyl. This product was recrystallized from heptane to obtain 5.64 g (10.5 mmol) of the subject compound.
Phase transition temperature: Cr 118.0 N 138.2 Iso
NMR: δ=0.40∼2.70 (39H, m), 6.85 (2H, d, J=9.5 Hz), 6.98∼7.23 (2H, m)
MS: m/e=534 (M⁺)

### Example 3

Preparation of 4-trifluoromethyl-2',6'-difluoro-4'-(trans-4-(4-(trans-4-propylcyclohexyl)butyl)cyclohexyl)biphenyl (Compound expressed by the general formula (1) wherein R₁ represents C₃H₇, both Z₁ and Z₂ represent a covalent bond, both L₁ and L₂ represent F, both L₃ and L₄ represent H, and X represents CF₃: Compound No. 25)

In the same manner as in Example 2 with the exception that 4-(trans-4-propylcyclohexyl)butylcyclohexanone was used in place of 4-(trans-4-pentylcyclohexyl)butylcyclohexanone, 1,3-difluoro-5-(trans-4-(4-(trans-4-propylcyclohexyl)butyl)cyclohexyl)benzene was prepared. After 5.00 g (13.3 mmol) of the benzene derivative was dissolved in 200 ml of THF, the solution was cooled down to -30°C under nitrogen gas stream, and a solution of 1.6 M of n-butyl lithium in 10.0 ml (16.0 mmol) of n-hexane was added dropwise to the solution while maintaining the same temperature and stirred for 1 hour. After cooled the solution down to -50°C, a separate solution of 0.5 M of zinc chloride in 35.0 ml (17.5 mmol) of THF was added dropwise thereto while maintaining the same temperature, stirred for 30 min, warmed up to room temperature, and then further stirred at room temperature for 1 hour.

To this solution was added 0.602 g (0.521 mmol) of tetrakistriphenylphosphine palladium (O), and a solution of 3.59 g (16.0 mmol) of 1-bromo-4-trifluoromethylbenzene in 30 ml of THF was further added dropwise. After finishing of the dropping, the solution was heated to reflux for 4 hours and allowed to cool, and 200 ml of water was added to the reaction product to terminate the reaction. Subsequently, 15 ml of 2N-hydrochloric acid was added to the solution and the reaction product was extracted with 150 ml of toluene. The organic layer was washed with 50 ml of a saturated aqueous solution of sodium bicarbonate and 50 ml of water each thrice in turn, and dried over anhydrous magnesium sulfate. The solvent was distilled off under a reduced pressure, and the concentrated residue thus obtained was subjected to silica gel column chromatography (eluent: toluene) to obtain a crude 4-trifluoromethyl-2',6'-difluoro-4'-(trans-4-(4-(trans-4-propylcyclohexyl)butyl) cyclohexyl)biphenyl.

This product was recrystallized from heptane to obtain 1.22 g (2.34 mmol) of the subject compound.
Phase transition temperature: Cr 138.4 N 153.6 Iso
NMR: δ=0.54∼2.74 (35H, m), 6.86 (2H, d, J=9.2 Hz), 7.45∼7.90 (4H, m)
MS: m/e=520 (M⁺)

### Example 4

Preparation of 3,2',6'-trifluoro-4-trifluoromethoxy-4'-(trans-4-(4-(trans-4-propylcyclohexyl)butyl)cyclohexyl)biphenyl (Compound expressed by the general formula (1) wherein R₁ represents C₃H₇, both Z₁ and Z₂ represent a covalent bond, all of L₁, L₂, and L₃ represent F, L₄ represents H, and X represents OCF₃: Compound No. 49)

After 6.16 g (16.4 mmol) of the 1,3-difluoro-5-(trans-4-(4-(trans-4-propylcyclohexyl)butyl) cyclohexyl)benzene prepared in the same manner as in Example 3 was dissolved in 200 ml of THF, the solution was cooled down to -30°C under nitrogen gas stream, and a solution of 1.6 M of n-butyl lithium in 13.0 ml (21.3 mmol) of n-hexane was added dropwise to the solution while maintaining the same temperature and stirred for 1 hour. After the solution was cooled down to -50°C, a separate solution of 0.5 M of zinc chloride in 40.0 ml (20.0 mmol) of THF was added dropwise thereto while maintaining the same temperature, stirred for 30 min, warmed up to room temperature, and then further stirred at room temperature for 1 hour.

Subsequently, 0.513 g (0.444 mmol) of tetrakistriphenylphosphine palladium (O) was added thereto, and a solution of 5.11 g (19.7 mmol) of 1-bromo-3-fluoro-4-trifluoromethoxybenzene in 30 ml of THF was added dropwise. After finishing of the dropping, the solution was heated to reflux for 3.5 hours and allowed to cool, and 200 ml of water was added to the reaction product to terminate the reaction. To the solution of the reaction product was added 15 ml of 2N-hydrochloric acid, and the product was extracted with 200 ml of toluene. The organic layer was washed with 100 ml of a saturated aqueous solution of sodium bicarbonate and 100 ml of water each five times in turn and then dried over anhydrous magnesium sulfate. The solvent was distilled off under a reduced pressure, and the concentrated residue thus obtained was subjected to silica gel column chromatography (eluent: toluene) to obtain a crude 3,2',6'-trifluoro-4-trifluoromethoxy-4'-(trans-4-(4-(trans-4-propylcyclohexyl)butyl)cyclohexyl)biphenyl. This product was recrystallized from heptane to obtain 3.75 g (6.76 mmol) of the subject compound.
Phase transition temperature: Cr 93.3 N 164.1 Iso
NMR: δ=0.40∼2.70 (35H, m), 6.85 (2H, d, J=9.2 Hz), 7.10∼7.55 (3H, m)
MS: m/e=554 (M⁺)

### Example 5

Preparation of trans-4-(4-(trans-4-propylcyclohexyl)butyl) cyclohexyl carboxylic acid 3,4,5,2',6'-pentafluoro-4'-biphenyl ester (Compound expressed by the general formula (1) wherein R₁ represents C₃H₇, Z₁ represents COO, Z₂ represents a covalent bond, and all of L₁, L₂, L₃, L₄, and X represent F: Compound No. 107)

After 30.0 g (208 mmol) of 3,5-difluoroanisole was dissolved in 500 ml of THF, the solution was cooled down to -50°C under nitrogen gas stream, and a solution of 1.6 M of n-butyl lithium in 156 ml (250 mmol) of n-hexane was added dropwise thereto while maintaining the same temperature and stirred for 1 hour. While maintaining the same temperature, 26.0 ml (250 mmol) of trimethyl borate was added dropwise to the solution, stirred for 2 hours, and warmed up to room temperature, and then 50 ml of water was added to the solution and stirred for 30 min. The reaction product in the solution was extracted with 800 ml of toluene, and the organic layer was washed with 200 ml of water thrice and dried over anhydrous magnesium sulfate. Then, the solvent was distilled off under a reduced pressure to obtain 24.2 g (129 mmol) of 2,6-difluoro-4-methoxyphenyl boronic acid. Subsequently, this product was dissolved in 500 ml of N,N-dimethyl formamide (DMF), 0.921 g (5.16 mmol) of palladium chloride and 50 ml of a saturated aqueous solution of sodium bicarbonate were added to the solution and warmed up to about 80°C under nitrogen gas stream, and then 32.7 g (155 mmol) of 1-bromo-3,4,5-trifluorobenzene was added thereto while maintaining the same temperature. After finishing the dropping, the solution was stirred at the same temperature for 3 hours and allowed to cool, and then 500 ml of water was added thereto at room temperature to terminate the reaction. To the solution of the reaction product was added 100 ml of 6N-hydrochloric acid, the reaction product was extracted with 700 ml of toluene, and the organic layer was washed with 200 ml of water thrice and dried over anhydrous magnesium sulfate. Subsequently, the solvent was distilled off under a reduced pressure to obtain 30.2 g of 3,5-difluoro-4-(3,4,5-trifluorophenyl)anisole.

After 30.0 g (109 mmol) of this product was dissolved in 500 ml of dichloromethane, the solution was cooled down to -50°C under nitrogen gas stream, and then 33.1 g (132 mmol) of boron tribromide was added dropwise thereto while maintaining the same temperature. After finishing of the dropping, the solution was stirred for 1 hour, slowly warmed up to room temperature, and further stirred for 3 hours, and then 500 ml of water was added to the reaction product to terminate the reaction. After the layer of dichloromethane (organic layer) was separated, it was washed with 100 ml of water thrice and dried over anhydrous magnesium sulfate. The solvent was distilled off under a reduced pressure, and the concentrated residue thus obtained was recrystallized from a mixed solvent of ethyl acetate/ethanol (7/3) to obtain 25.8 g (99.2 mmol) of 3,4,5,2',6'-pentafluoro-4'-hydroxy-biphenyl.

Under nitrogen gas stream, 46.3 g (135 mmol) of methoxymethyltriphenylphosphonium chloride was dissolved in 700 ml of THF and cooled down to -50°C. To this solution was added 16.7 g (149 mmol) of t-BuOK and stirred for 1 hour. To this mixture was added dropwise a solution of 30.0 g (108 mmol) of 4-(trans-4-propylcyclohexyl)butylcyclohexanone in 300 ml of THF while maintaining a temperature of lower than -50°C. After finishing of the dropping, the reaction temperature was slowly raised up to room temperature, and the mixture was further stirred for 5 hours. After 200 ml of water was added to the reaction product to terminate the reaction, the product was extracted with 1000 ml of toluene. The organic layer was washed with 100 ml of water thrice and dried over anhydrous magnesium sulfate. After the solvent was distilled off under a reduced pressure, the concentrated residue thus obtained was subjected to silica gel column chromatography (eluent: toluene) and further recrystallized from heptane to obtain 19.9 g (64.7 mmol) of 4-(trans-4-propylcyclohexyl)butylcyclohexylidenemethyl ether. This product and 29.8 g (647 mmol) of formic acid were dissolved in 500 ml of toluene and heated to reflux for 3 hours.

After 100 ml of water was added to the reaction product to terminate the reaction, the organic layer was washed with 200 ml of water thrice and dried over anhydrous magnesium sulfate. The solvent was distilled off under a reduced pressure, and the concentrated residue thus obtained was recrystallized from heptane to obtain 17.3 g (59.1 mmol) of trans-4-(4-(trans-4-propylcyclohexyl)butyl)cyclohexanecarbaldehyde.

Subsequently, this product was dissolved in 200 ml of DMF, and 44.4 g (118 mmol) of PDC was added to the solution and stirred at room temperature for 7 hours. After 1500 ml of water was added to the reaction product to terminate the reaction, the product was extracted with 1000 ml of hexane. The organic layer was washed with 200 ml of water thrice, and the solvent was distilled off under a reduced pressure to obtain 16.9 g of trans-4-(4-(trans-4-propylcyclohexyl)butyl)cyclohexane carboxylic acid.

This product in an amount of 16.9 g (54.9 mmol), 14.3 g (55.0 mmol) of 3,4,5,2',6'-pentafluoro-4'-hydroxybiphenyl, and 2.01 g (16.5 mmol) of DMAP were dissolved in 1000 ml of dichloromethane and stirred on an ice bath under nitrogen gas stream. A solution of 13.6 g (65.9 mmol) of DCC in 100 ml of dichloromethane was added dropwise thereto and stirred at room temperature for 12 hours. Water in an amount of 1000 ml was added thereto to terminate the reaction, precipitated insoluble matters were filtered off, and 500 ml of toluene was added to the filtrate. The organic layer was washed with 300 ml of 2N-aqueous solution of sodium hydroxide twice, with 500 ml of water five times, and with 300 ml of a saturated aqueous solution of ammonium chloride and 500 ml of water twice, in turn, and then dried over anhydrous magnesium sulfate. After the solvent was distilled off under a reduced pressure, the concentrated residue thus obtained was subjected to silica gel column chromatography (eluent: heptane) to obtain a crude trans-4-(4-(trans-4-propylcyclohexyl)butyl)cyclohexyl carboxylic acid 3,4,5,2',6'-pentafluoro-4'-biphenyl ester. This product was recrystallized from heptane to obtain 9.97 g (18.1 mmol) of the subject compound.
MS: m/e= 550 (M⁺)

### Example 6

Preparation of 3,4,5,2',6'-pentafluoro-4'-(2-(trans-4-(4-(trans-4-propylcyclohexyl)butyl)cyclohexyl)ethyl)biphenyl (Compound expressed by the general formula (1) wherein R₁ represents C₃H₇, Z₁ represents (CH₂)₂, Z₂ represents a covalent bond, and all of L₁, L₂, L₃, L₄, and X represent F: Compound No. 197)

Under nitrogen gas stream, 30.3 g (135 mmol) of diethylphosphonoacetic acid ethyl ester was dissolved in 700 ml of THF and cooled down to -50°C. To this solution was added 16.7 g (149 mmol) of t-BuOK and stirred for 1 hour. To this mixture was added dropwise a solution of 30.0 g (108 mmol) of 4-(trans-4-propylcyclohexyl)butylcyclohexanone in 300 ml of THF while maintaining a temperature lower than -50°C. After finishing of the dropping, the reaction temperature was slowly raised up to room temperature, and the mixture was further stirred for 5 hours. After 200 ml of water was added to the reaction product to terminate the reaction, the product was extracted with 1000 ml of toluene. The organic layer was washed with 200 ml of water thrice and dried over anhydrous magnesium sulfate. The solvent was distilled off under a reduced pressure, and the reside was subjected to silica gel column chromatography (eluent: toluene) and further recrystallized from heptane to obtain 16.2 g (46.4 mmol) of 4-(trans-4-propylcyclohexyl)butylcyclohexylidene carboxylic acid ethyl. This product was dissolved in 200 ml of a mixed solvent of toluene/Solmix (1/1), and 0.80 g of palladium/carbon catalyst was added to the solution and stirred at room temperature under a condition of hydrogen gas pressure of 1 to 2 kg/cm². When the amount of consumed hydrogen reached 1050 ml, the stirring was discontinued, and the palladium/carbon catalyst was filtered off. The organic solvent was distilled off under a reduced pressure, and the residue was subjected to silica gel column chromatography (eluent: toluene) and further recrystallized from heptane to obtain 16.0 g (45.5 mmol) of (4-(trans-4-propylcyclohexyl)butylcyclohexyl)acetic acid ethyl ester.

Subsequently, this product was dissolved in 400 ml of THF and cooled down to -50°C under nitrogen gas stream, and then a solution of 1.0 M of DIBAL in 54.6 ml (54.6 mmol) of n-hexane was added dropwise to the solution and stirred at the same temperature for 1 hour. After the reaction product was put in 500 ml of an ice water to terminate the reaction, 40 ml of 2N-hydrochloric acid was added thereto, and the product was extracted with 500 ml of toluene. The organic layer was washed with 100 ml of water thrice and dried over anhydrous magnesium sulfate. The solvent was distilled off under a reduced pressure, and the residue was subjected to silica gel column chromatography (eluent: toluene) and further recrystallized from heptane to obtain 12.6 g (44.1 mmol) of (4-(trans-4-propylcyclohexyl)butylcyclohexyl)acetaldehyde.

Magnesium in an amount of 1.44 g (59.2 mmol) which was heated to dry under nitrogen gas stream for 30 min was suspended in 10 ml of THF, and a solution of 9.51 g (49.3 mmol) of 1-bromo-3,5-difluorobenzene in 20 ml of THF was added dropwise thereto while maintaining a temperature of 50 to 60°C and stirred for 30 min. After allowed to cool, a solution of 12.6 g (44.1 mmol) of (4-(trans-4-propylcyclohexyl)butylcyclohexyl) acetaldehyde in 35 ml of THF was added dropwise to the mixture at room temperature and stirred while maintaining the mixture on a water bath at 60°C for 1.5 hours. After the reaction product was put in 50 ml of an ice water to terminate the reaction, 10 ml of 6N-hydrochloric acid was added thereto, and the product was extracted with 150 ml of toluene. The organic layer was washed with 50 ml of water thrice and dried over anhydrous magnesium sulfate, and then the solvent was distilled off under a reduced pressure. The concentrated residue thus obtained was dissolved in 100 ml of toluene, and 0.63 g of Amberlist was added to the solution and heated to reflux for 2 hours. After allowed to cool down to room temperature, the Amberlist was filtered off, the solvent was distilled off under a reduced pressure, and the residue was subjected to silica gel column chromatography (eluent: heptane) and further recrystallized from heptane to obtain 11.8 g (29.3 mmol) of 1,3-difluoro-5-(2-(4-(4-(trans-4-propylcyclohexyl)butyl)cyclohexyl) ethenyl)benzene. This product was dissolved in a 100 ml of mixed solvent of toluene/Solmix (1/1), and 2.50 g of Raney nickel was added thereto and stirred at room temperature under a condition of hydrogen gas pressure of 1 to 2 kg/cm². When the amount of consumed hydrogen reached 650 ml, the stirring was discontinued, and the Raney nickel was filtered off. The solvent was distilled off under a reduced pressure, and the concentrated residue was subjected to silica gel column chromatography (eluent: heptane) and further recrystallized from heptane to obtain 4.50 g (11.1 mmol) of 1.3-difluoro-5-(2-(trans-4-(4-(trans-4-propylcyclohexyl)butyl) cyclohexyl)ethyl)benzene.

After this product was dissolved in 200 ml of THF and cooled down to -30°C under nitrogen gas stream, a solution of 1.6 M of n-butyl lithium in 8.3 ml (13.3 mmol) of n-hexane was added dropwise thereto while maintaining the same temperature and stirred for 1 hour.

After it was cooled down to -50°C, a solution of 0.5 M of zinc chloride in 26.6 ml (13.3 mmol) of THF was added dropwise thereto while maintaining the same temperature. After the mixture was stirred for 30 min, warmed up to room temperature, and further stirred at room temperature for 1 hour, 0.501 g (0.433 mmol) of tetrakistriphenylphosphine palladium (O) was added thereto, and 2.81 g (13.3 mmol) of 1-bromo-3,4,5-trifluorobenzene was added dropwise. After finishing of the dropping, the mixture was heated to reflux for 4 hours and allowed to cool, and then 200 ml of water was added to the reaction product to terminate the reaction. To the solution of the reaction product was added 15 ml of 2N-hydrochloric acid, and the product was extracted with 150 ml of toluene. The organic layer was treated with 50 ml of a saturated aqueous solution of sodium bicarbonate, washed with 150 ml of water thrice and then dried over anhydrous magnesium sulfate.

The solvent was distilled off under a reduce pressure, and the residue was subjected to silica gel column chromatography (eluent: heptane) to obtain a crude 3,4,5,2',6'-pentafluoro-4'-(2-(trans-4-(4-(trans-4-propylcyclohexyl)butyl)cyclohexyl) ethyl)biphenyl. This product was further recrystallized from heptane to obtain 2.45 g (4.58 mmol) of the subject compound.
MS: m/e= 534 (M⁺)

### Example 7

Preparation of 3,4,5-trifluorophenyl 2,6-difluoro-4-(trans-4-(4-(trans-4-propylcyclohexyl)butyl)cyclohexyl)benzoate (Compound expressed by the general formula (1) wherein R₁ represents C₃H₇, Z₁ represents a covalent bond, Z₂ represents COO, and all of L₁, L₂, L₃, L₄, and X represent F: Compound No. 287)

After 4.50 g (11.1 mmol) of the 1,3-difluoro-5-(trans-4-(4-(trans-4-propylcyclohexyl)butyl)cyclohexyl)benzene which was prepared in the same manner as in Example 3 was dissolved in 200 ml of THF and cooled down to -30°C under nitrogen gas stream, a solution of 1.6 M of n-butyl lithium in 8.3 ml (13.3 mmol) of n-hexane was added dropwise to the solution while maintaining the same temperature and stirred for 1 hour, and 20 g of dry ice was added thereto and stirred for 30 min. After 200 ml of water was added to the reaction product to terminate the reaction, 15 ml of 2N-hydrochloric acid was added thereto, and the product was extracted with 200 ml of diethyl ether. The organic layer was washed with 50 ml of water thrice and dried over anhydrous magnesium sulfate. The solvent was distilled off under a reduced pressure, and the residue was recrystallized from heptane to obtain 4.43 g (9.88 mmol) of 2,6-difluoro-4-(trans-4-(4-(trans-4-propylcyclohexyl)butyl)cyclohexyl)benzoic acid.

This product in an amount of 2.00 g (4.46 mmol), 0.661 g (4.46 mmol)of 3,4,5-trifluorophenol, and 0.163 g (1.34 mmol) of DMAP were dissolved in 50 ml of dichloromethane and stirred on an ice bath under nitrogen gas stream. A solution of 0.921 g (4.46 mmol) of DCC in 10 ml of dichloromethane was added dropwise thereto and stirred at room temperature for 12 hours. The reaction was terminated by adding 100 ml of water to the solution, precipitated insoluble matters were filtered off, and then 50 ml of toluene was added to the filtrate. The organic layer was washed with 300 ml of 2N-aqueous solution of sodium hydroxide twice, with 50 ml of water five times, and with 30 ml of a saturated aqueous solution of ammonium chloride and 50 ml of water twice, in turn, and then dried over anhydrous magnesium sulfate.

The solvent was distilled off under a reduce pressure, and the residue was subjected to silica gel column chromatography (eluent:toluene) to obtain a crude 3,4,5-trifluorophenyl 2,6-difluoro-4-(trans-4-(4-(trans-4-propylcyclohexyl)butyl) cyclohexyl)benzoate. This product was recrystallized from heptane to obtain 0.958 g (1.74 mmol) of the subject compound.
MS: m/e= 550 (M⁺)

### Example 8

Preparation of 1,2,3-trifluoro-5-(2-(2,6-difluoro-4-(trans-4-(4-(trans-4-propylcyclohexyl)butyl)cyclohexyl)phenyl) ethyl)benzene (Compound expressed by the general formula (1) wherein R₁ represents C₃H₇, Z₁ represents a covalent bond, Z₂ represents (CH₂)₂, and all of L₁, L₂, L₃, L₄, and X represent F: Compound No. 377)

In 50 ml of ethanol was dissolved 2.00 g (4.46 mmol) of the 2,6-difluoro-4-(trans-4-(4-(trans-4-propylcyclohexyl)butyl) cyclohexyl)benzoic acid which was prepared in the same manner as in Example 7, and then 0.1 ml of a concentrated sulfuric acid was added and heated to reflux for 3 hours. After the ethanol was distilled off under a reduced pressure, and 50 ml of toluene and 50 ml of water were added to the residual liquid to extract the product, the layer of the extract was washed with 50 ml of a saturated aqueous solution of sodium bicarbonate and then with 50 ml of water five times and dried over anhydrous magnesium sulfate. The solvent was distilled off under a reduce pressure, and the residue was subjected to silica gel column chromatography (eluent: toluene) and further recrystallized from heptane to obtain 1.87 g (3.92 mmol) of 2,6-difluoro-4-(trans-4-(4-(trans-4-propylcyclohexyl)butyl)cyclohexyl)benzoic acid ethyl.

After this product was dissolved in 50 ml of THF and cooled down to -50°C under nitrogen gas stream, a solution of 1.0 M of DIBAL in 4.7 ml (4.7 mmol) of n-hexane was added dropwise to the solution and stirred at the same temperature for 1 hour. After the reaction product was added to 100 ml of an ice water to terminate the reaction, 5 ml of 2N-hydrochloric acid was added, and the product was extracted with 100 ml of toluene. The organic layer was washed with 50 ml of water thrice and dried over anhydrous magnesium sulfate. The solvent was distilled off under a reduced pressure, and the residue was subjected to silica gel column chromatography (eluent: toluene) and recrystallized from heptane to obtain 1.58 g (3.65 mmol) of 2,6-difluoro-4-(trans-4-(4-(trans-4-propylcyclohexyl)butyl) cyclohexyl)benzaldehyde.

Under nitrogen gas stream, 1.50 g (4.38 mmol) of methoxymethyltriphenylphosphonium chloride was dissolved in 50 ml of THF and cooled down to -50°C. To this solution was added 0.59 g (5.26 mmol) of t-BuOK and stirred for 1 hour. A solution of 1.58 g (3.65 mmol) of 2,6-difluoro-4-(trans-4-(4-(trans-4-propylcyclohexyl)butyl)cyclohexyl)benzaldehyde in 15 ml of THF was added dropwise to the mixture while maintaining a temperature lower than -50°C. After finishing of the dropping, the reaction temperature was slowly raised up to room temperature, and the mixture was further stirred for 5 hours. After 10 ml of water was added to the reaction product to terminate the reaction, the product was extracted with 100 ml of toluene. The organic layer was washed with 50 ml of water thrice and dried over anhydrous magnesium sulfate.

The solvent was distilled off under a reduced pressure, and the residue was subjected to silica gel column chromatography (eluent: toluene) and further recrystallized from heptane to obtain 0.947 g (2.19 mmol) of 1-(2-methoxyethenyl)-2,6-difluoro-4-(trans-4-(4-(trans-4-propylcyclohexyl)butyl) cyclohexyl)benzene.

This product was dissolved together with 1.01 g (21.9 mmol) of formic acid in 50 ml of toluene and heated to reflux for 3 hours. After 10 ml of water was added to the reaction product to terminate the reaction, the organic layer was washed with 30 ml of water thrice and dried over anhydrous magnesium sulfate. The solvent was distilled off under a reduced pressure, the residue was recrystallized from heptane to obtain 0.800 g (1.91 mmol) of (2,6-difluoro-4-(trans-4-(4-(trans-4-propylcyclohexyl) butyl)cyclohexyl)phenyl)acetaldehyde.

In 5 ml of THF was suspended 0.0700 g (2.88 mmol) of magnesium which was heated to dry under nitrogen gas stream for 30 min, 0.480 g (2.28 mmol) of 1-bromo-3,4,5-trifluorobenzene was added dropwise to the suspension so that a temperature of 50 to 60°C was maintained and then stirred for 30 min.

After allowed to cool, a solution of 0.800 g (1.91 mmol) of the (2,6-difluoro)-4-(trans-4-(4-(trans-4-propylcyclohexyl) butyl)cyclohexyl)phenyl)acetaldehyde described above in 5 ml of THF was added dropwise to the suspension and stirred for 1.5 hours. The reaction product was put in 10 ml of an ice water to terminate the reaction, 0.5 ml of 6N-hydrochloric acid was added, and then the product was extracted with 30 ml of toluene. After the organic layer was washed with 20 ml of water thrice and dried over anhydrous magnesium sulfate, the solvent was distilled off under a reduced pressure.

After 30 ml of toluene and 0.04 g of Amberlist were added to the residue and heated to reflux for 2 hours, the Amberlist was filtered off. The solvent was distilled of under a reduced pressure, the residue was subjected to silica gel column chromatography (eluent: heptane) and further recrystallized from heptane to obtain 0.712 g (1.34 mmol) of 1,2,3-trifluoro-5-(2-(2,6-difluoro-4-(trans-4-(4-(trans-4-propylcyclohexyl)butyl) cyclohexyl)phenyl)ethenyl)benzene.

Subsequently, this product was dissolved in 20 ml of a mixed solvent of toluene/Solmix (1/1), 0.165 g of Raney nickel was added and stirred at room temperature under a condition of hydrogen gas pressure of 1 to 2 kg/cm². When the amount of consumed hydrogen reached 30 ml, the stirring was discontinued, and the Raney nickel was filtered off. The solvent was distilled off under a reduced pressure, and the residue was subjected to silica gel column chromatography (eluent: heptane) to obtain a crude 1,2,3-trifluoro-5-(2-(2,6-difluoro-4-(trans-4-(4-(trans-4-propylcyclohexyl)butyl)cyclohexyl)phenyl) ethyl)benzene. This product was recrystallized from heptane to obtain 0.412 g (0.771 mmol) of the subject compound.
MS: m/e= 534 (M⁺)

Based on the description in Examples 1 through 8 and the column of BEST MODE FOR CARRYING OUT THE INVENTION in the specification, the following compound No. 1 to No. 838 can be produced (Compound Nos. 11, 18, 25, 49, 107, 197, 287, and 377 are mentioned again). Each of the compounds is indicated by extracting parameters (substituents) R₁, Z₁, Z₂, L₁, L₂, L₃, L₄, and X in the general formula (1). The portions where no parameters are indicated means a covalent bond.

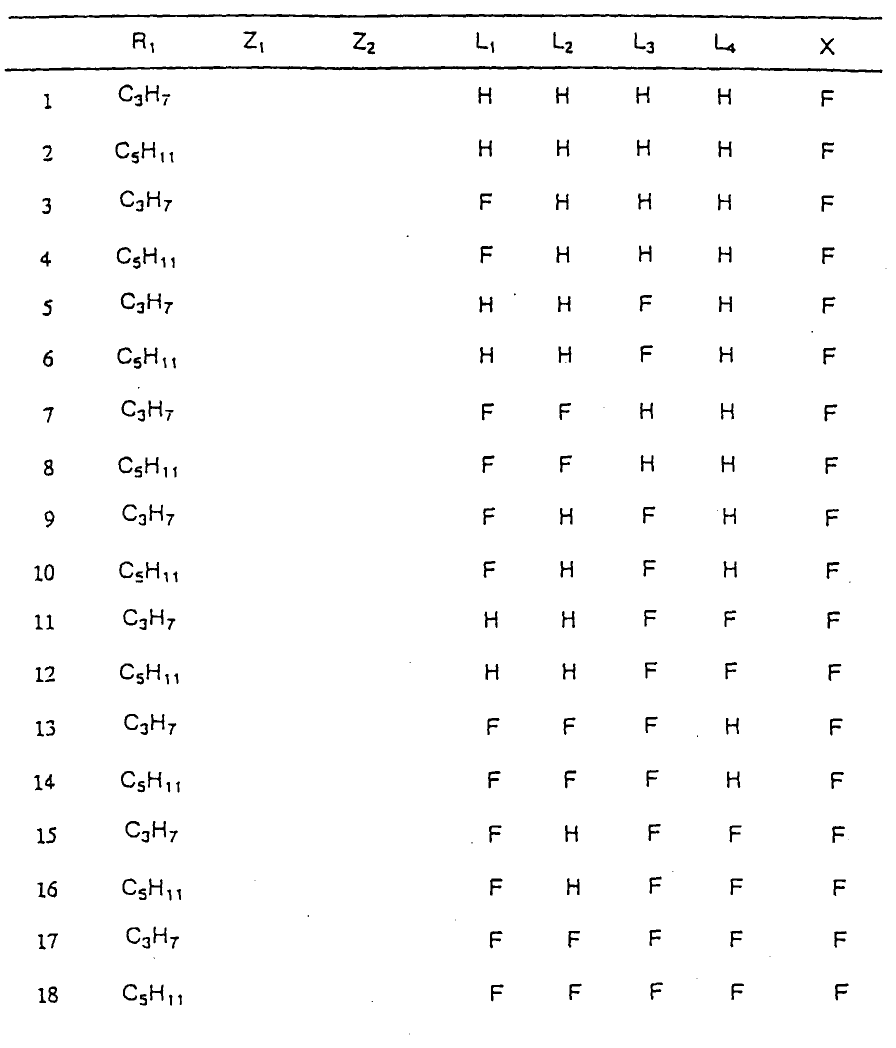

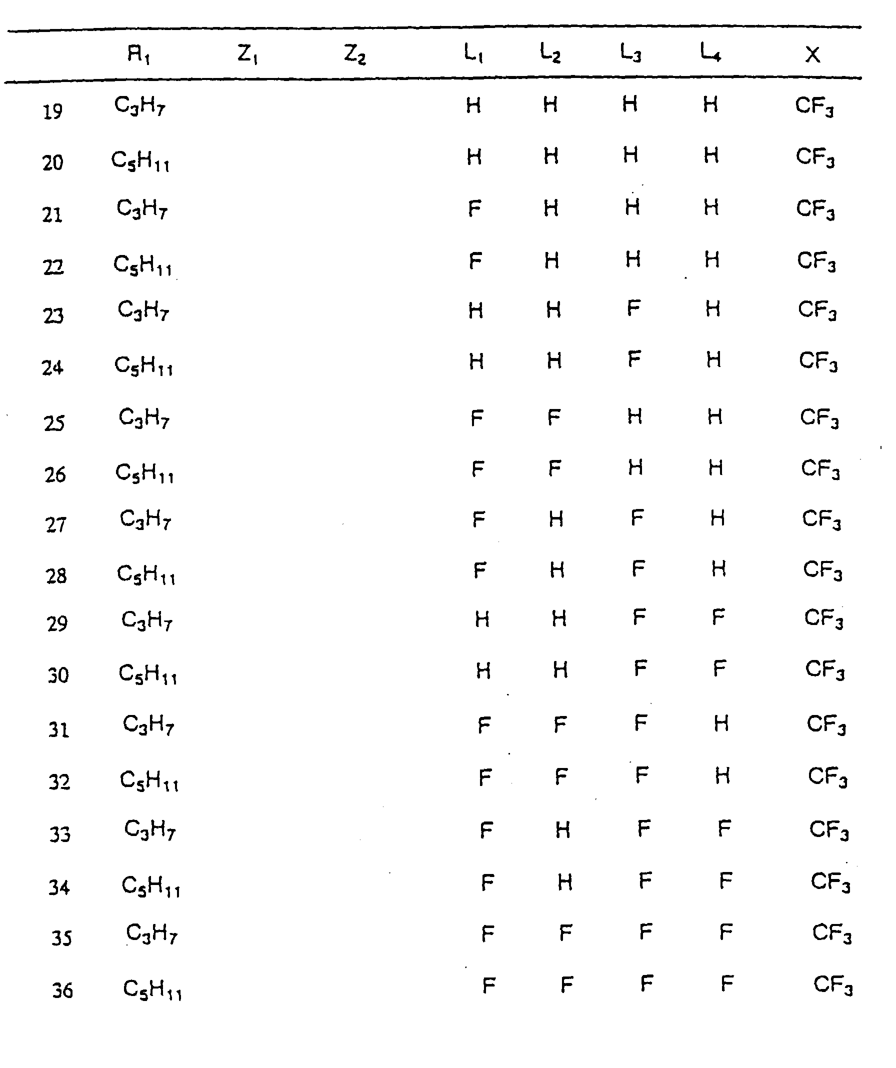

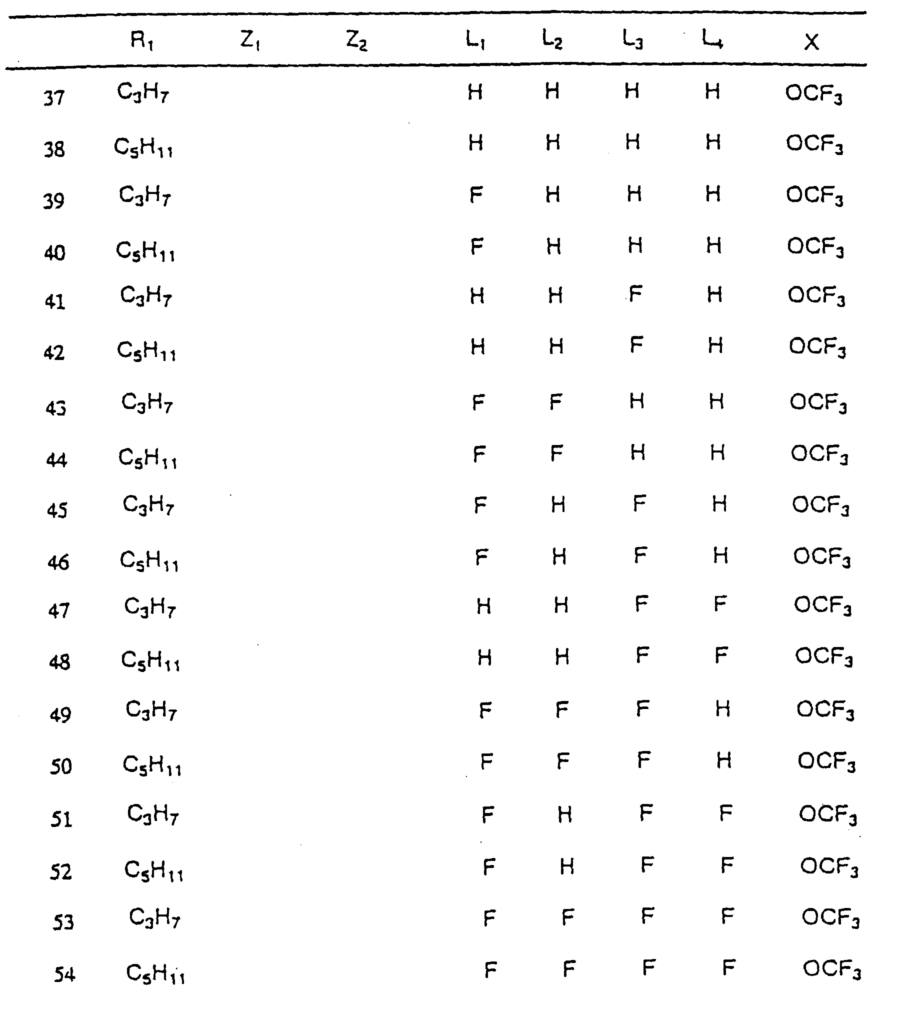

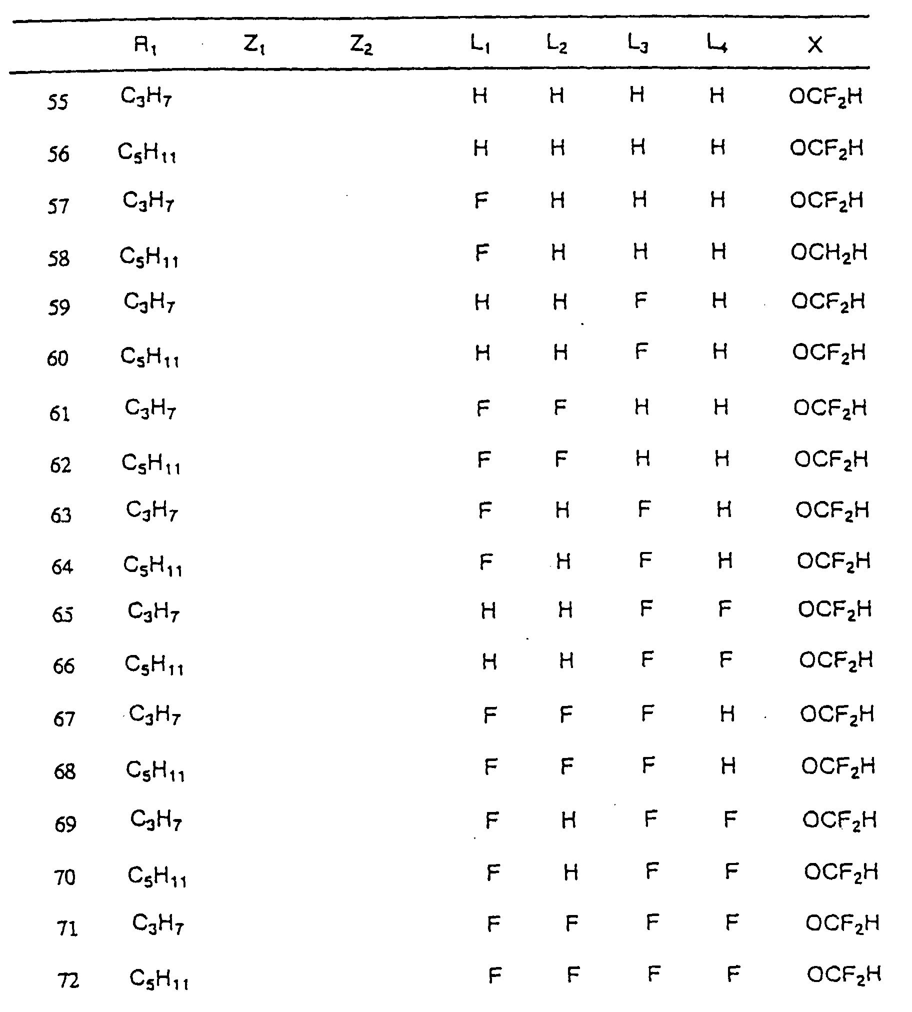

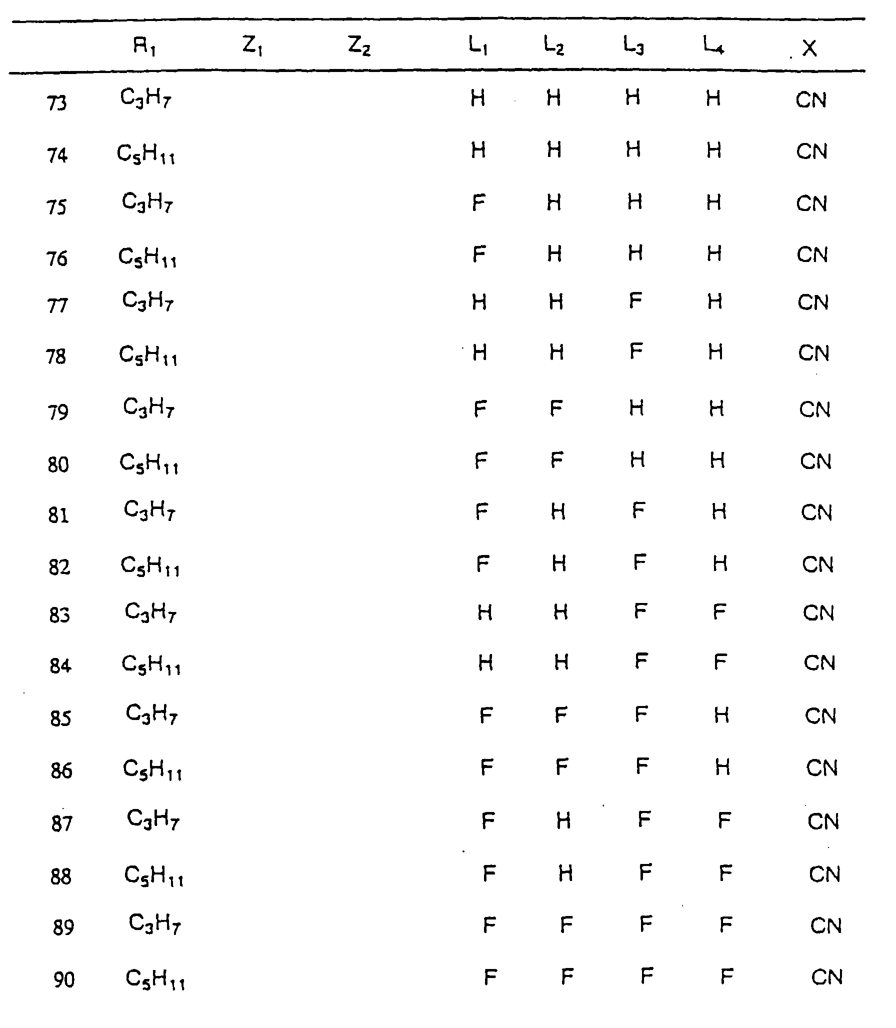

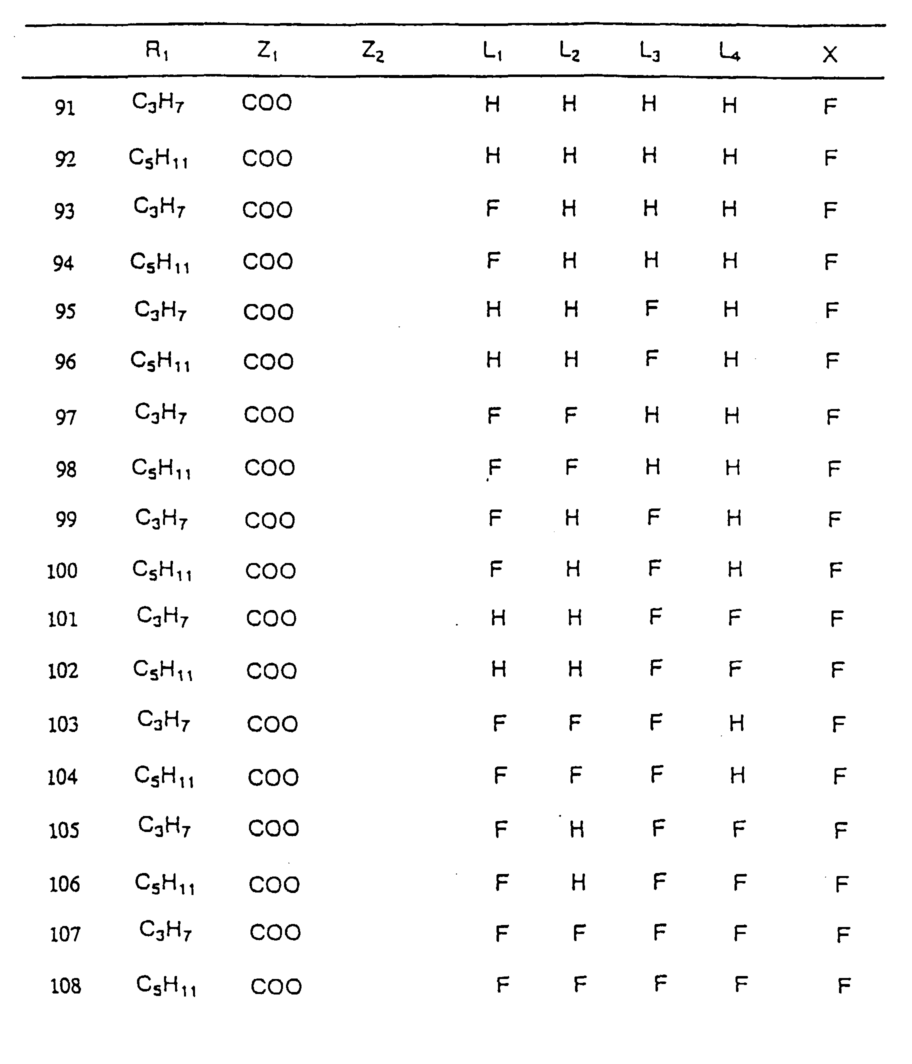

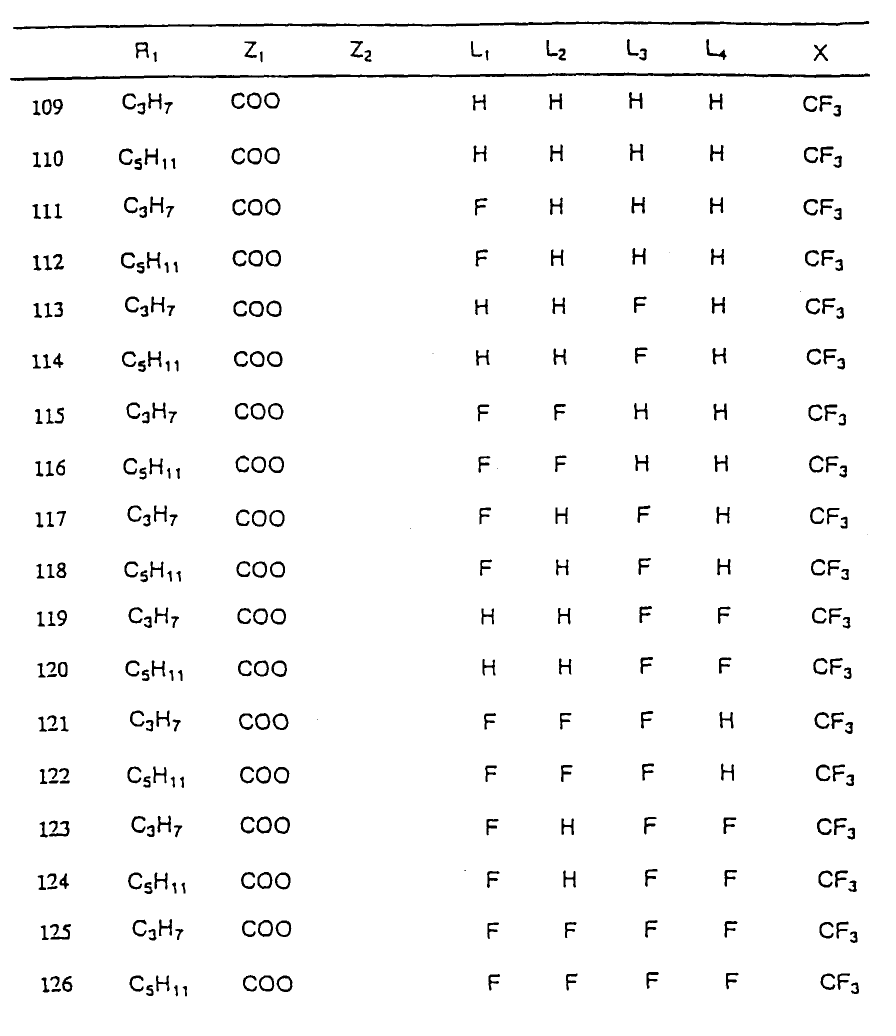

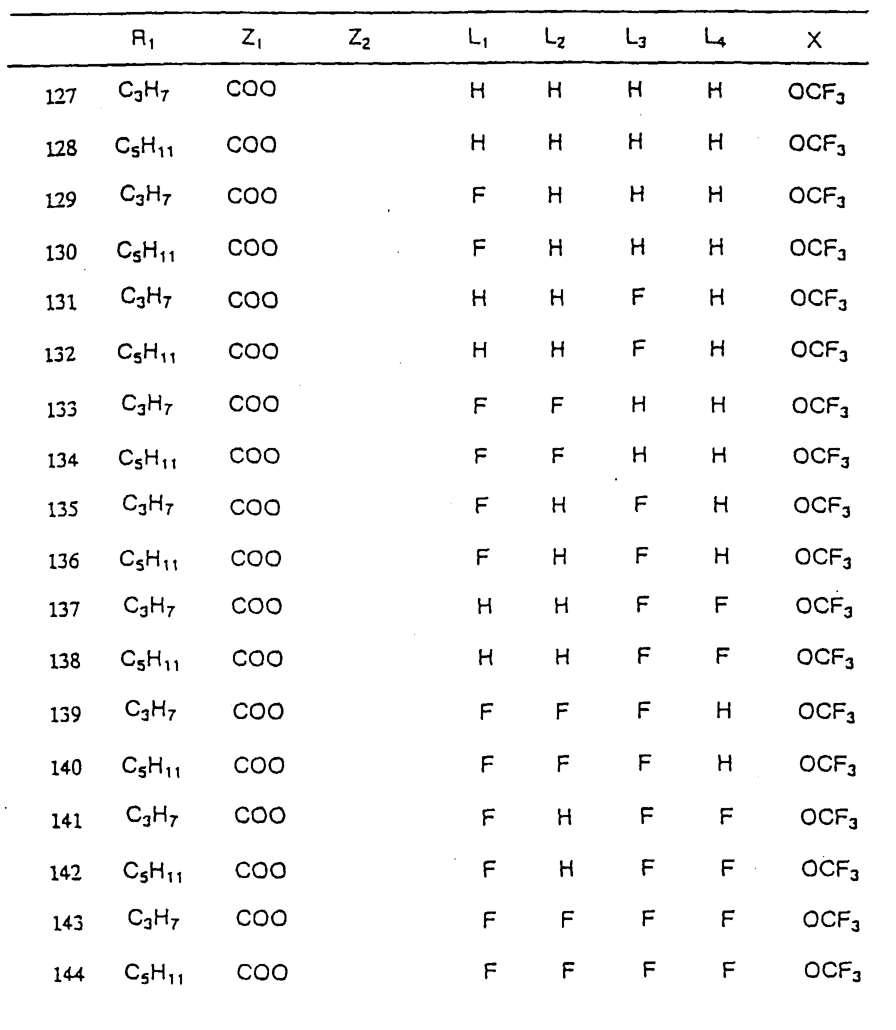

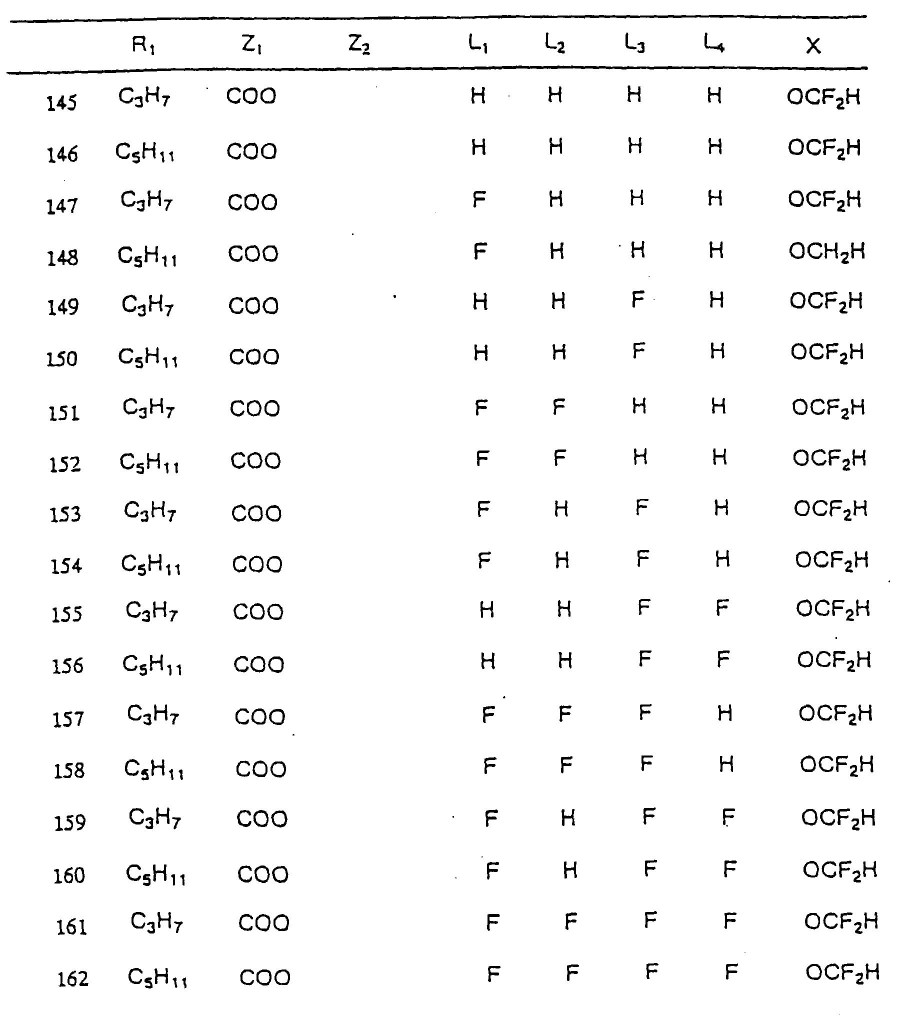

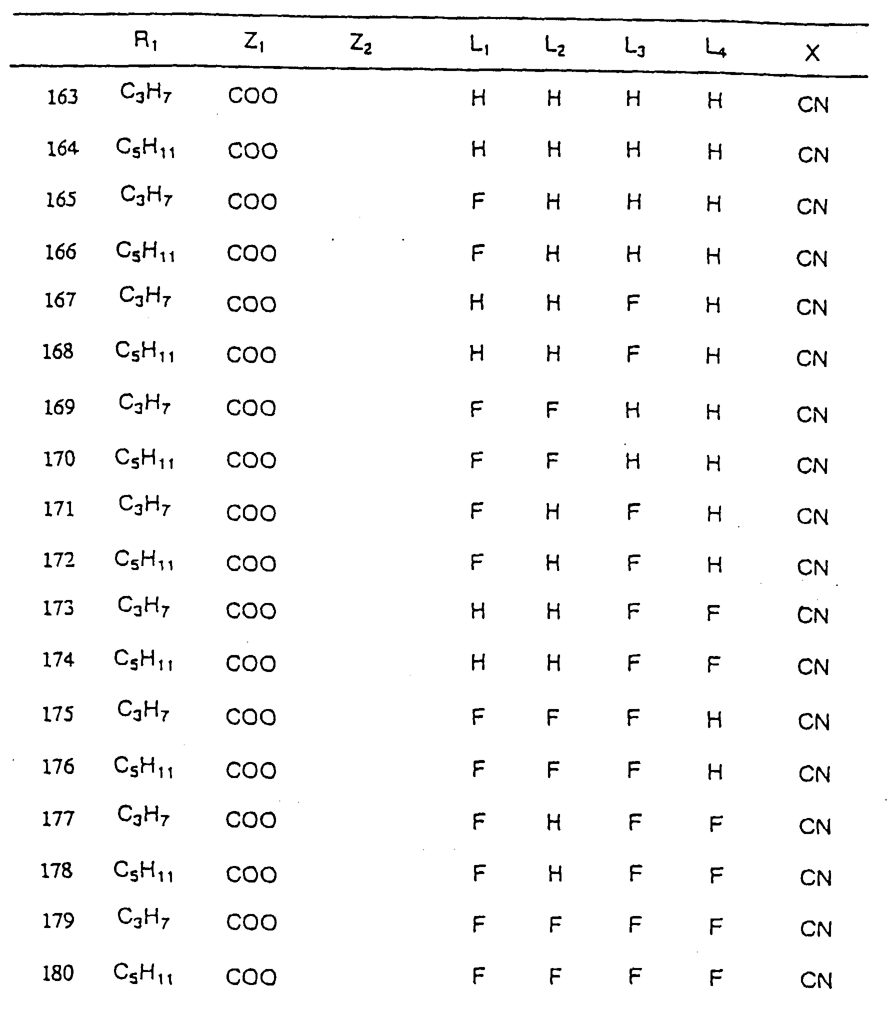

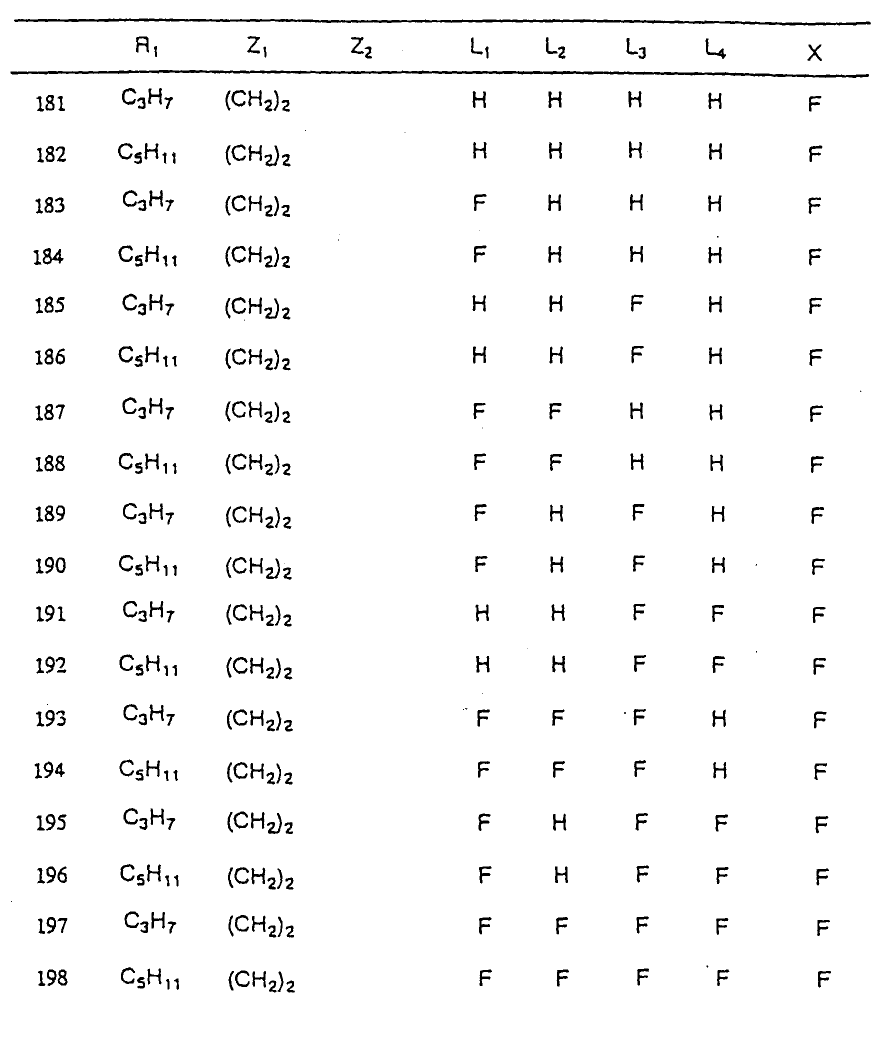

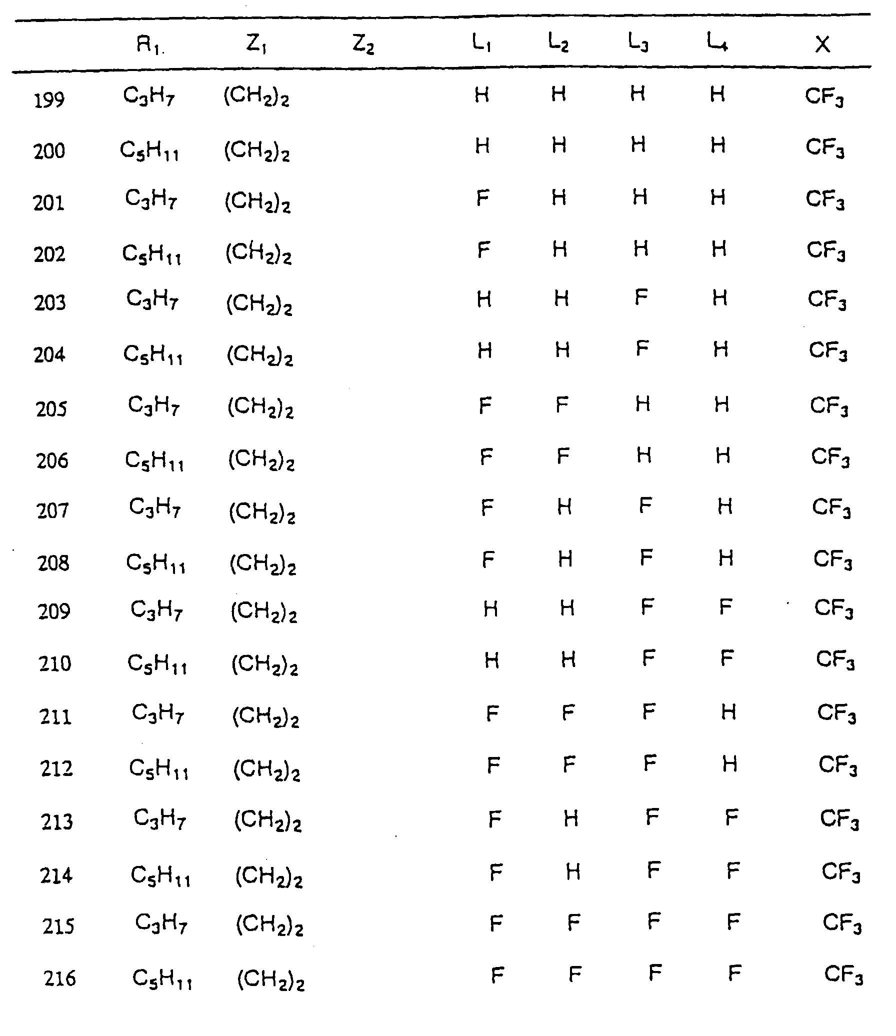

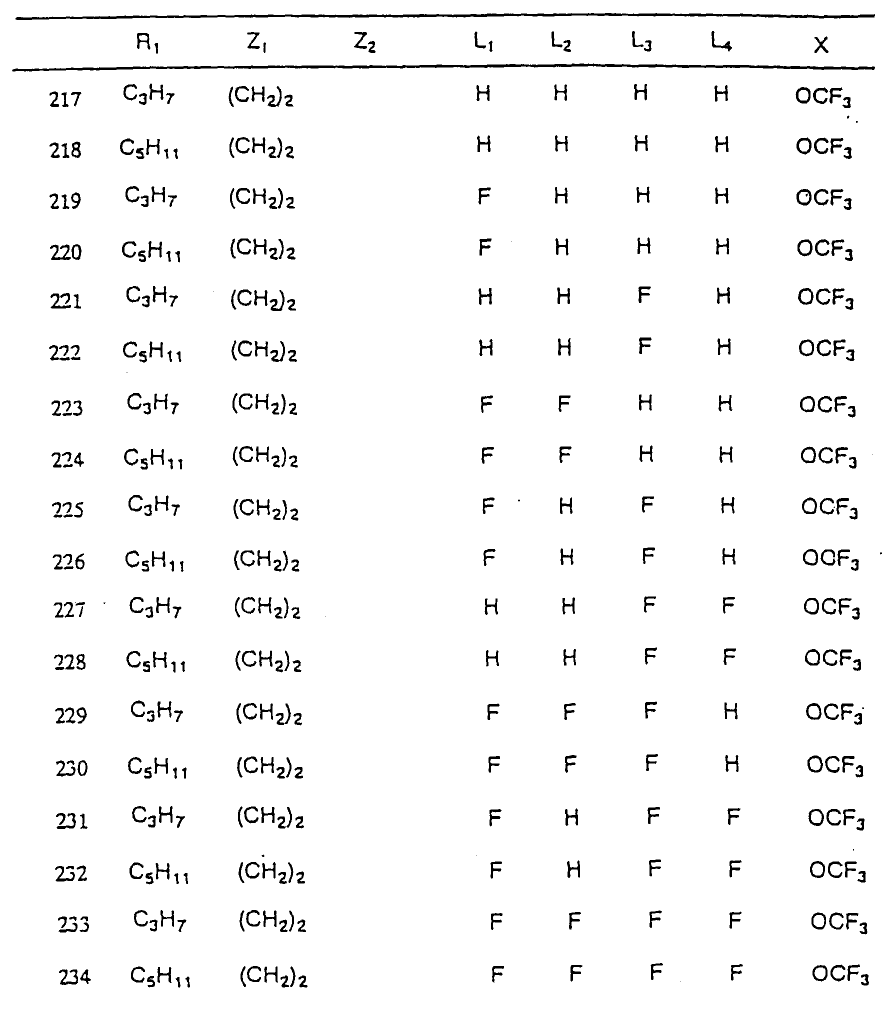

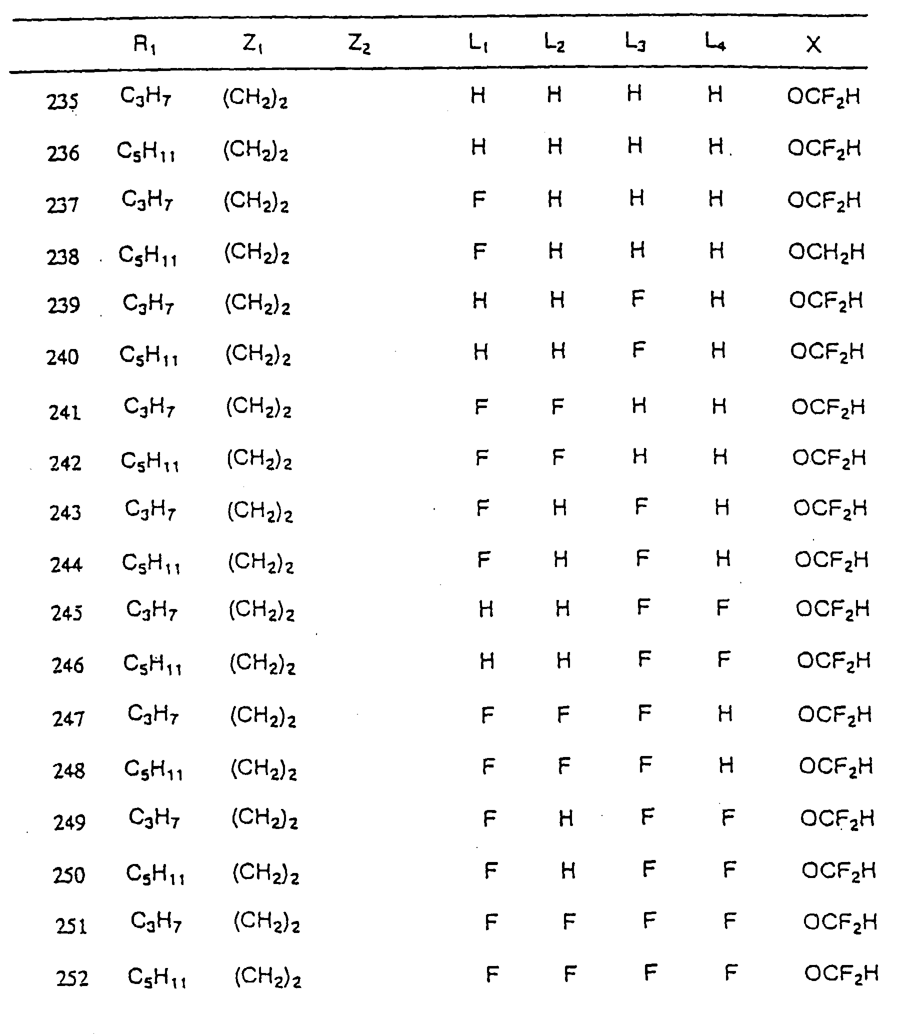

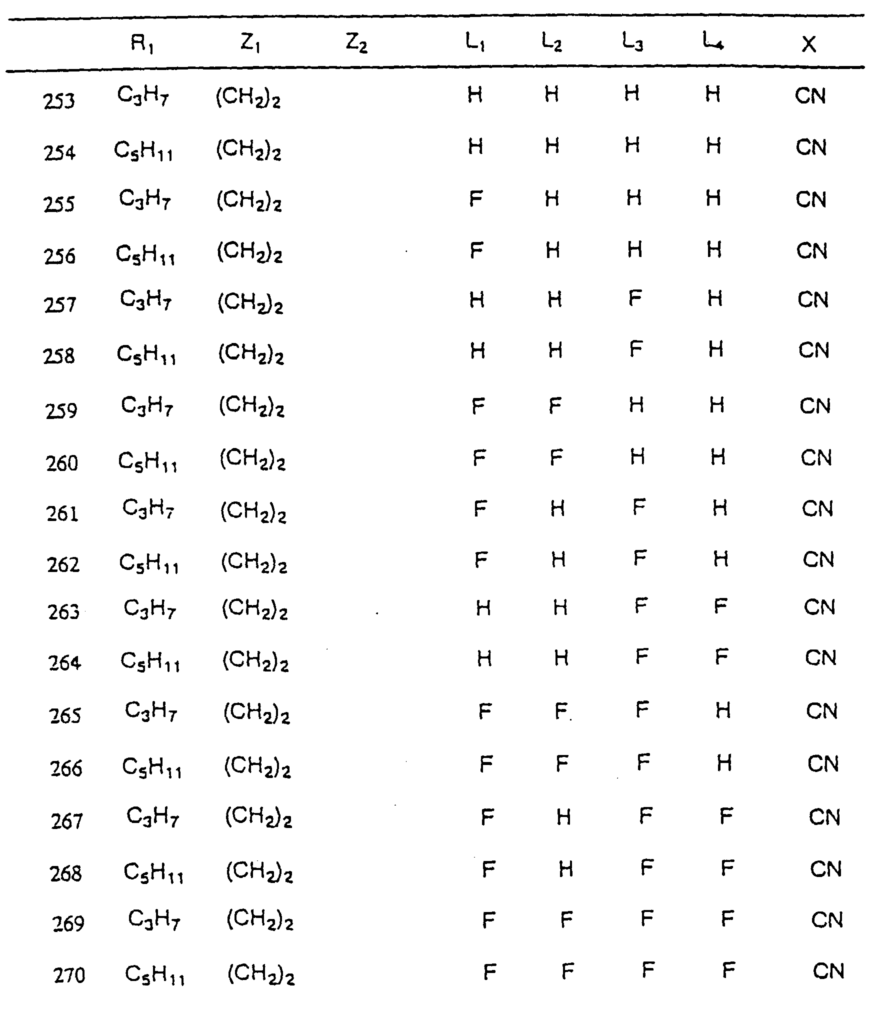

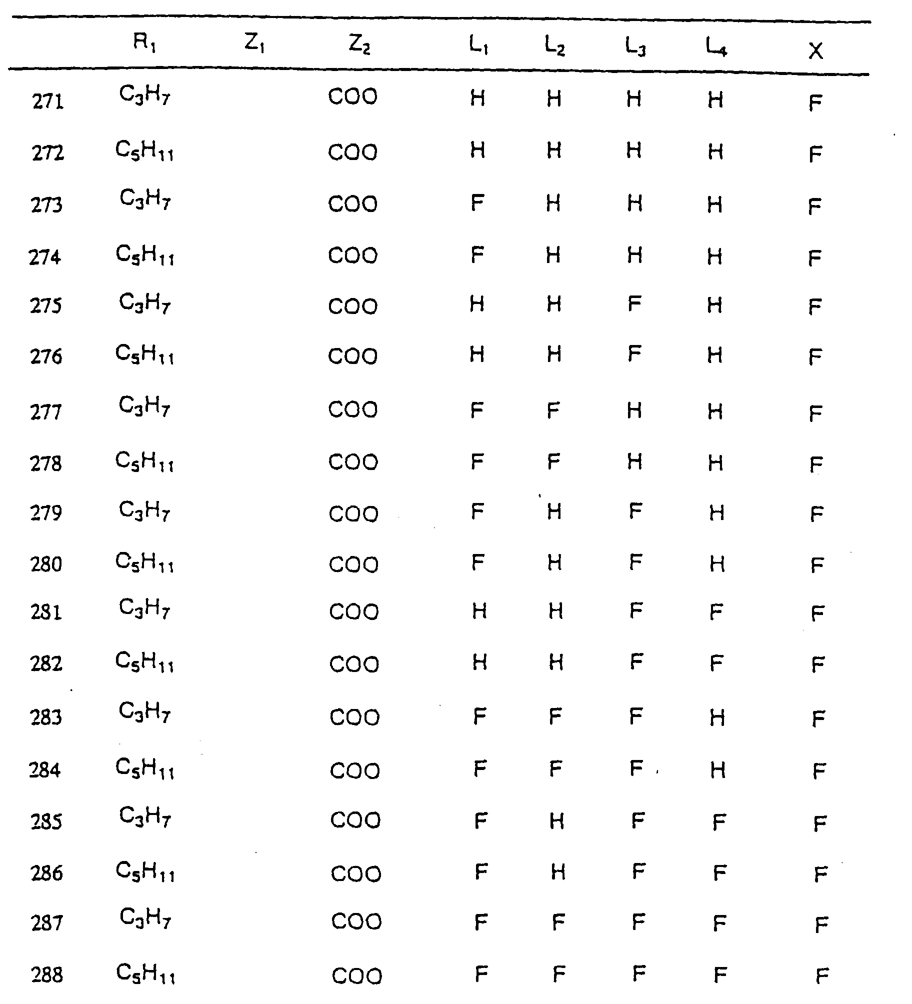

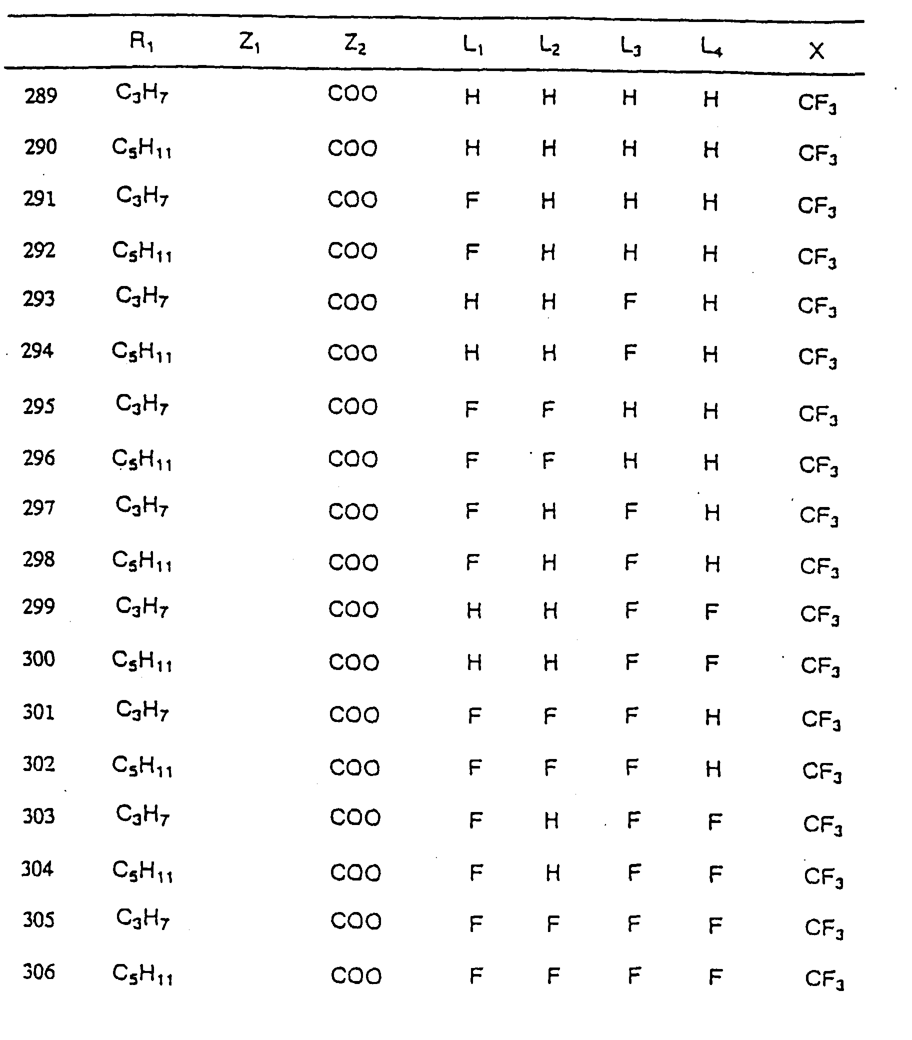

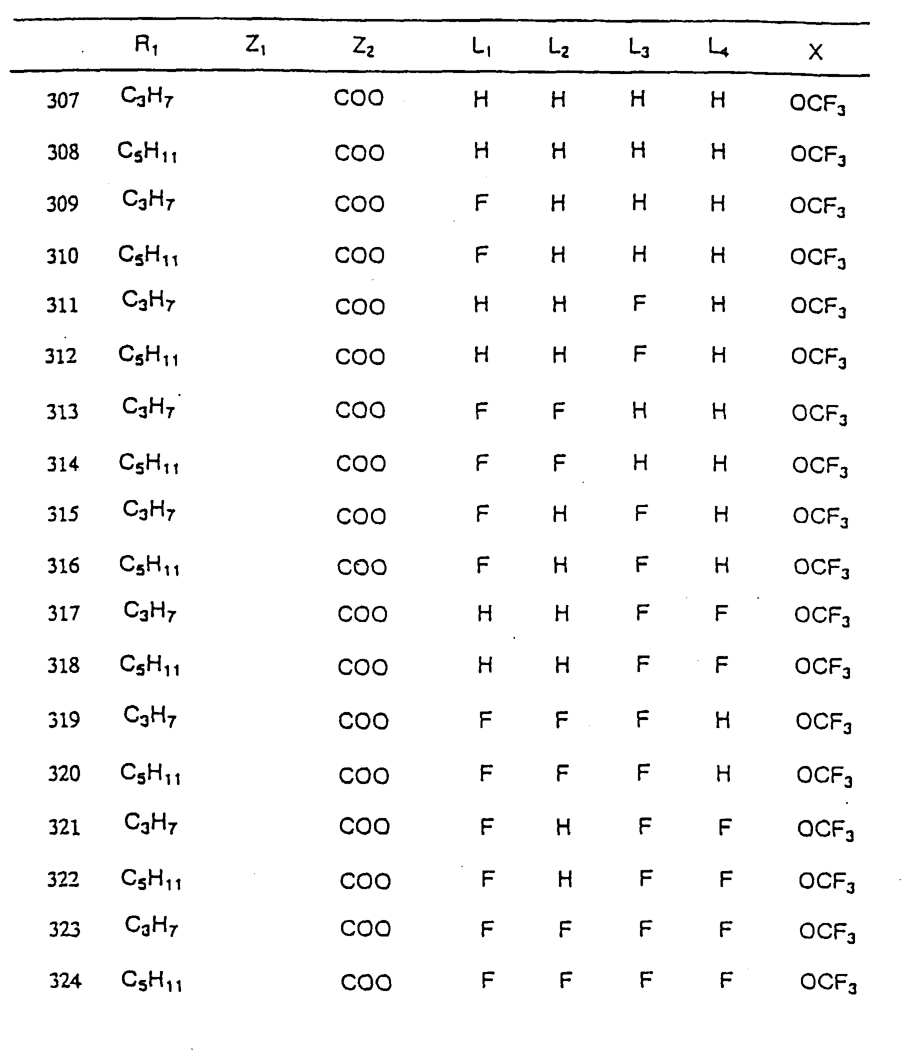

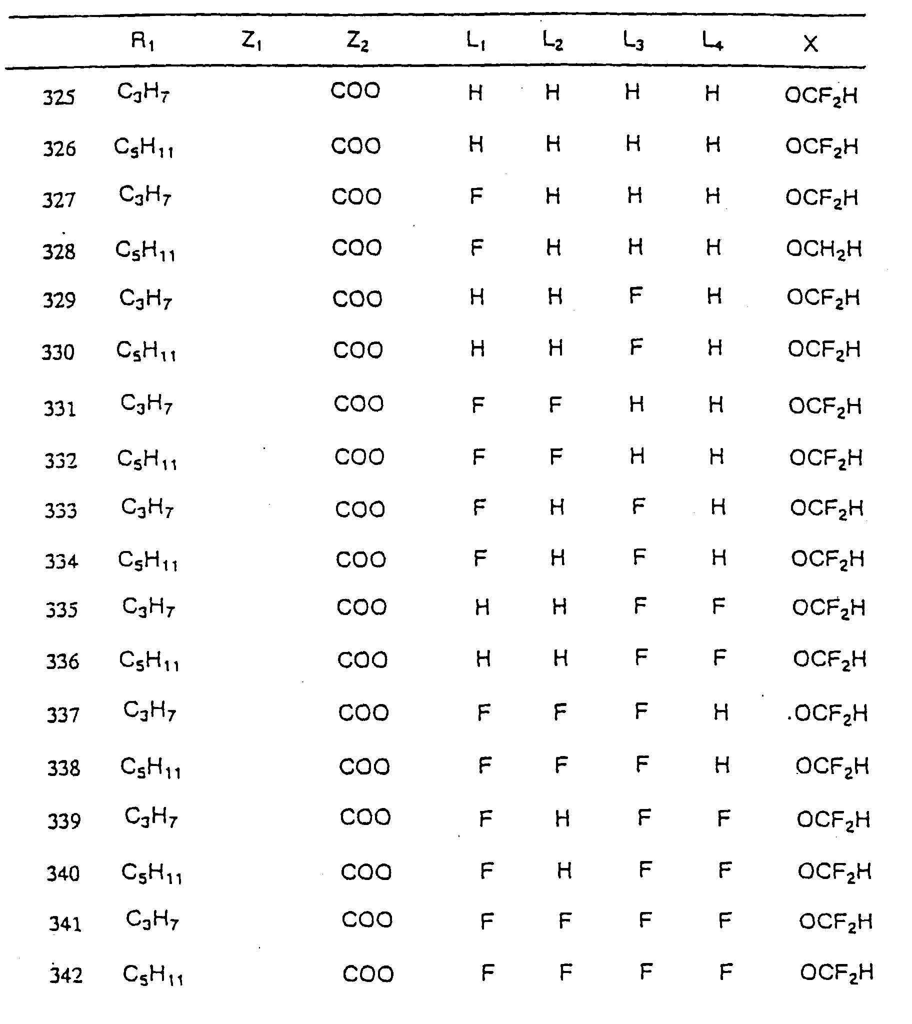

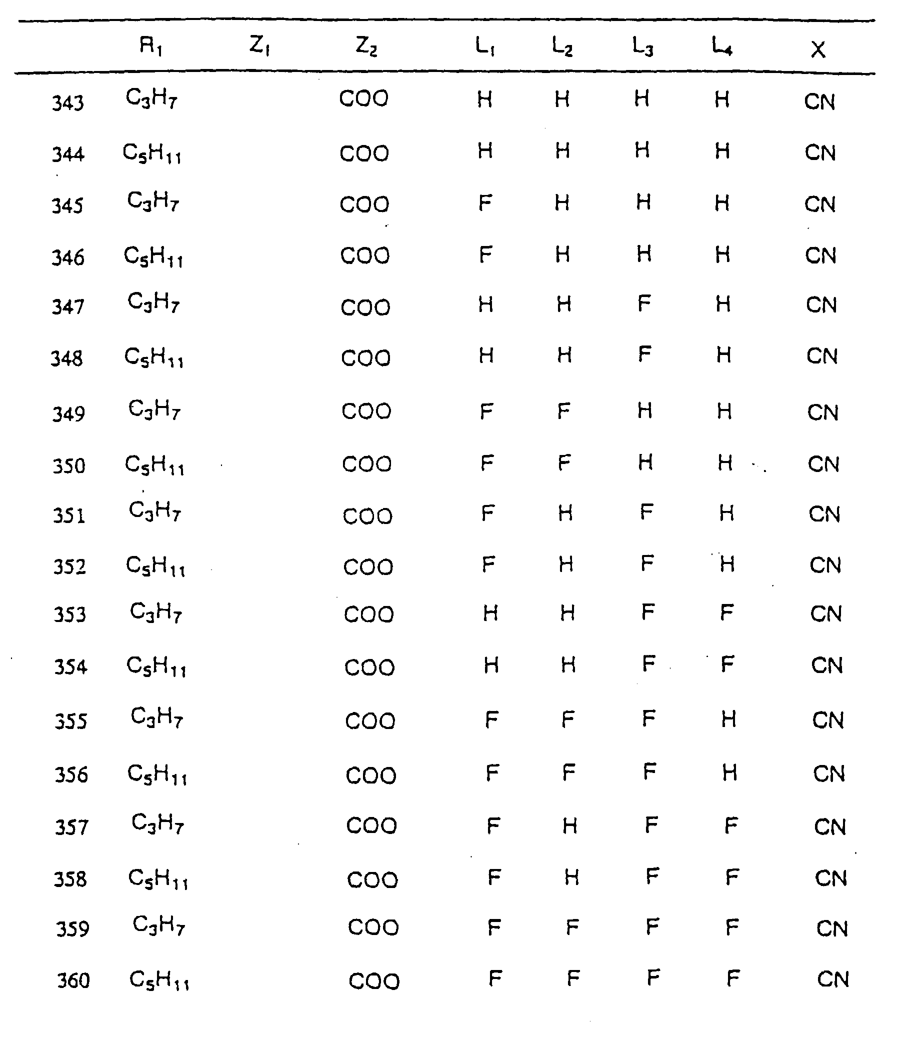

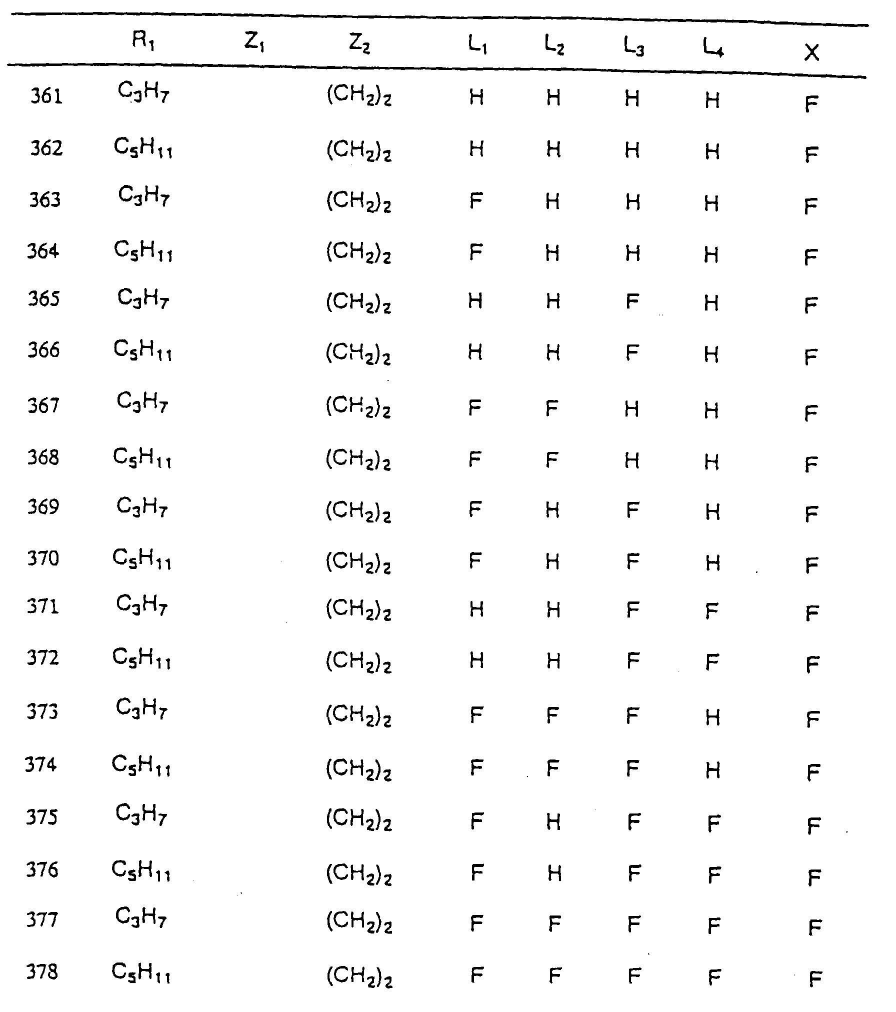

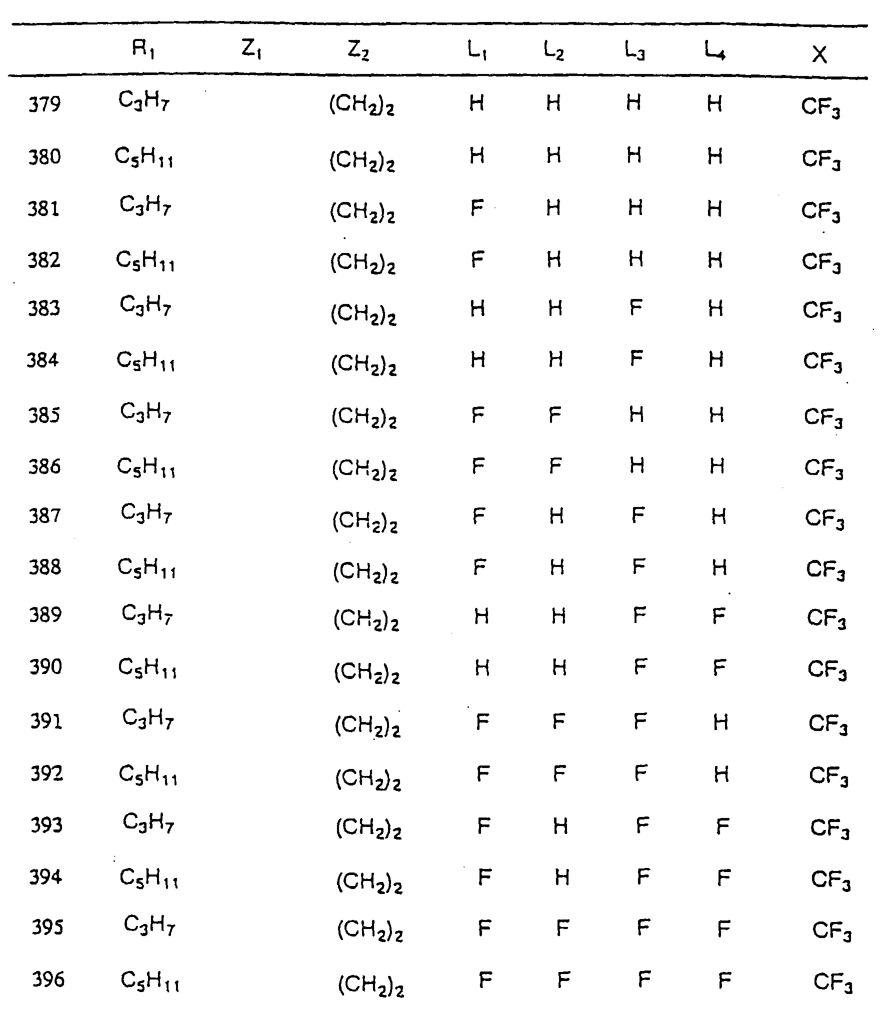

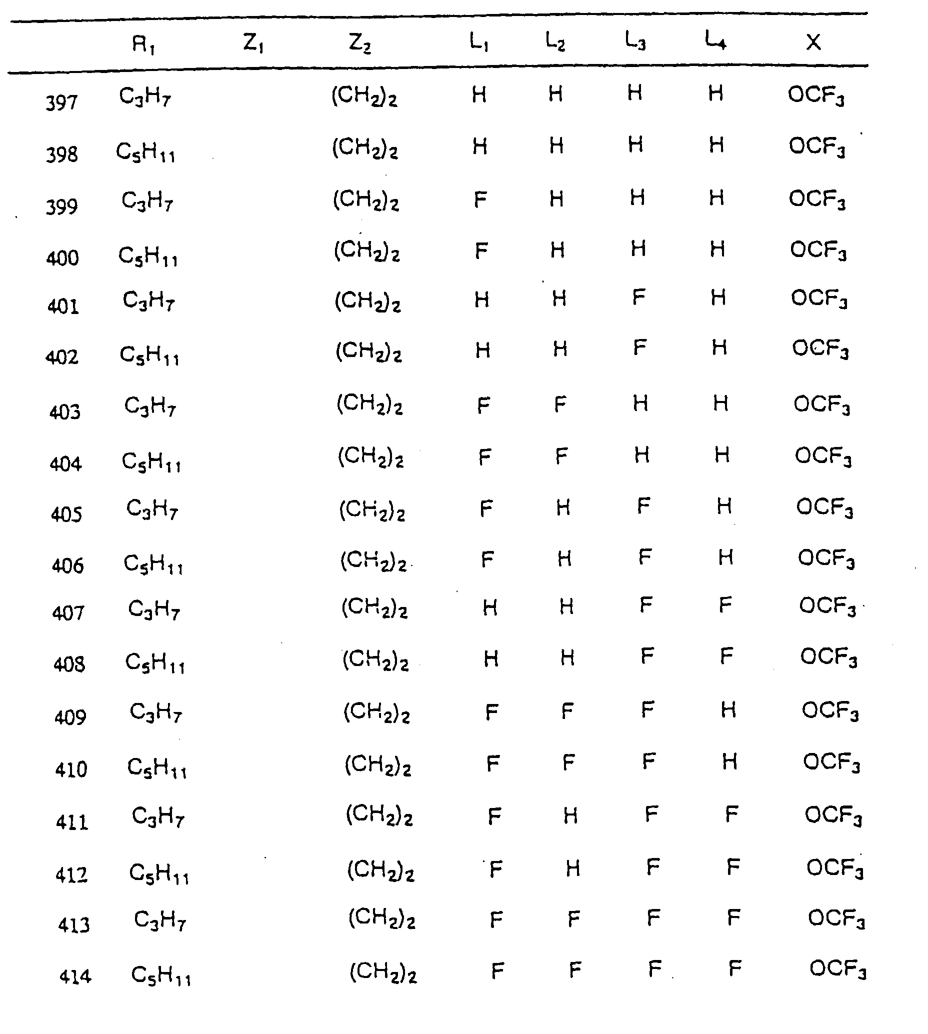

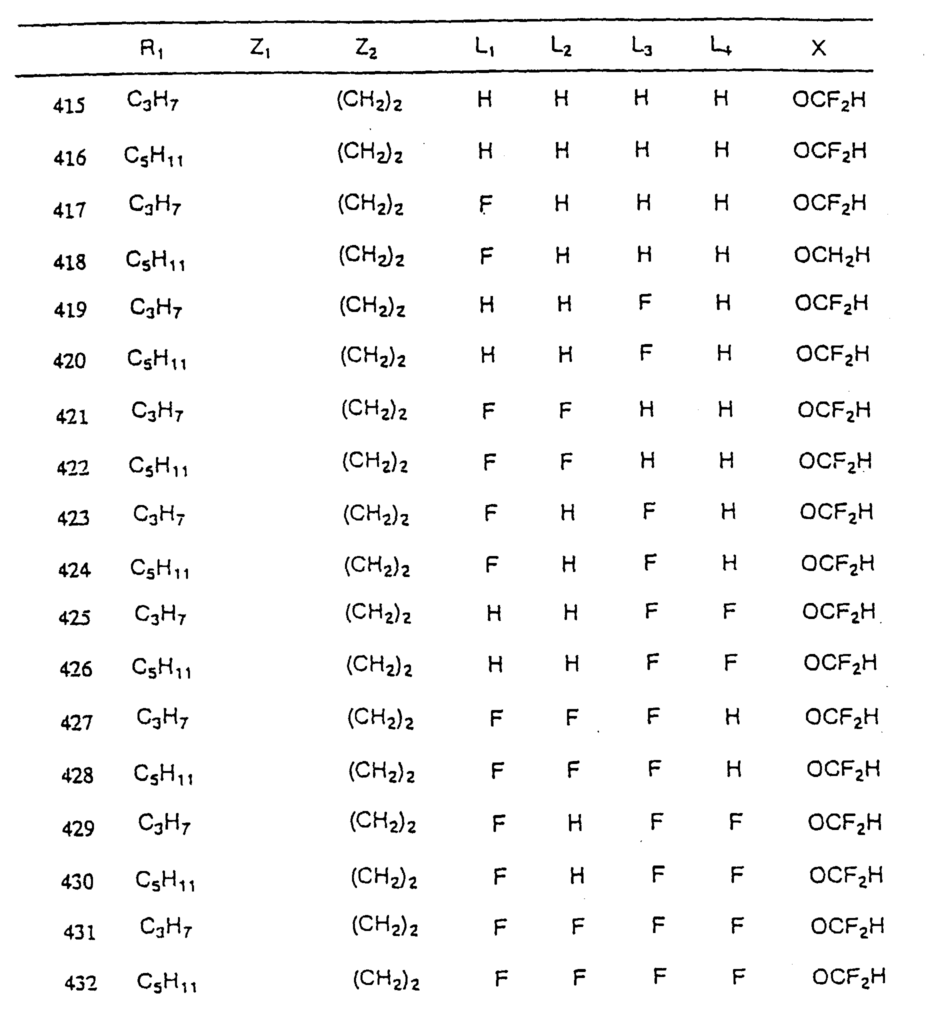

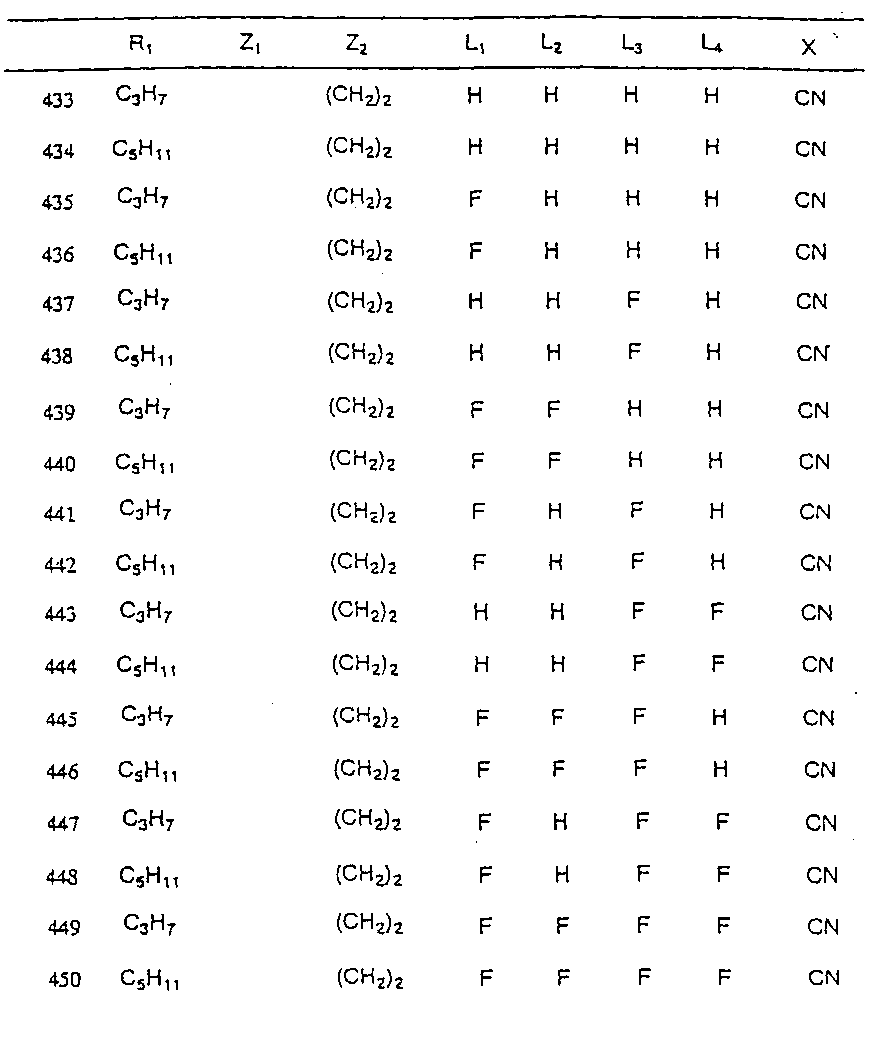

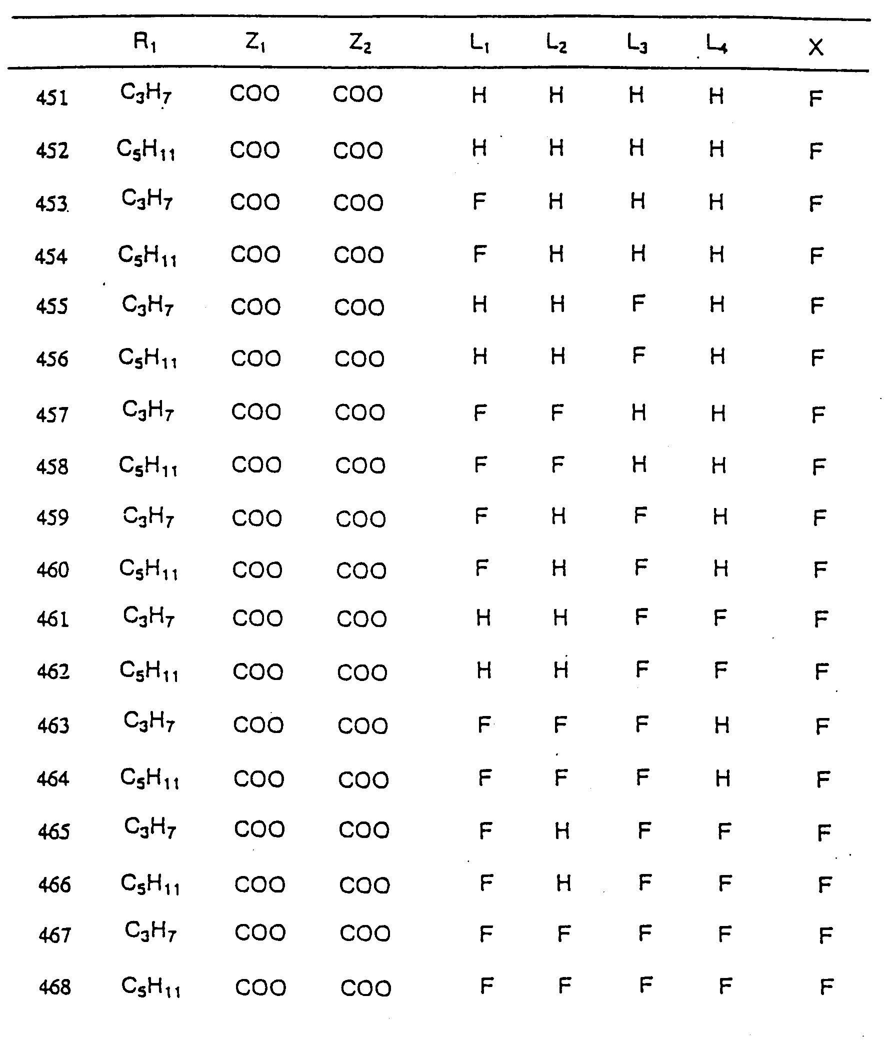

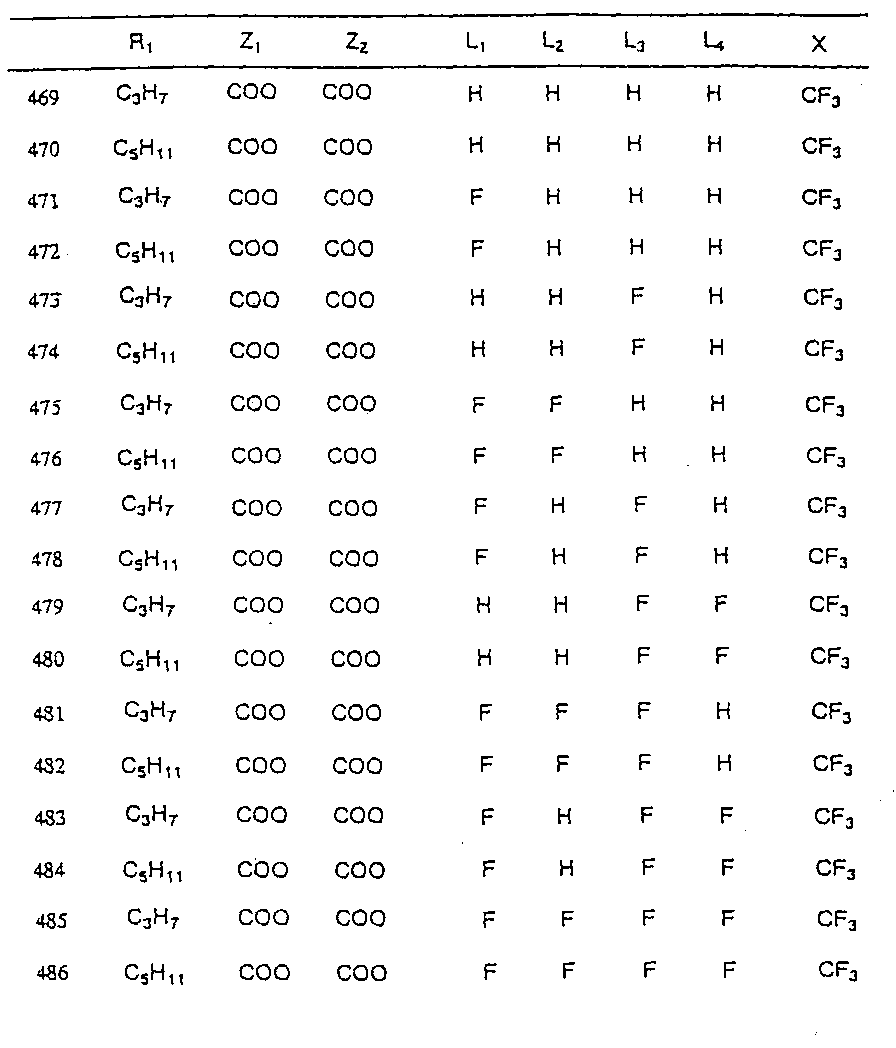

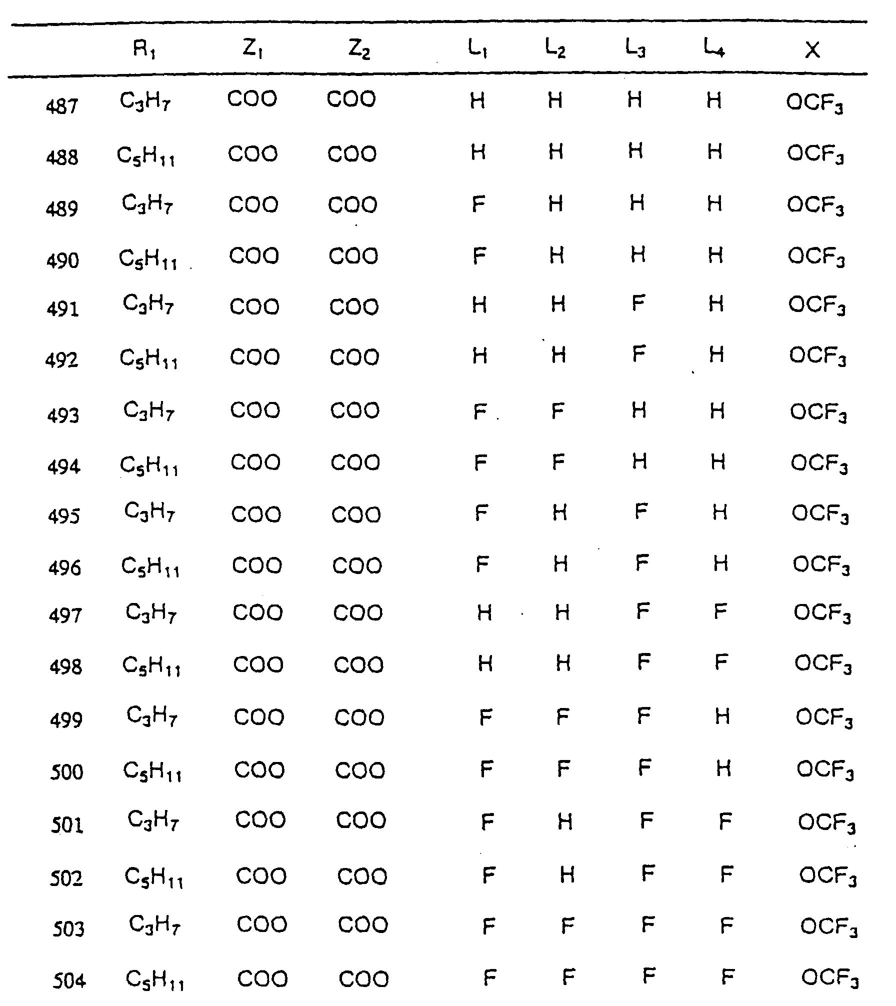

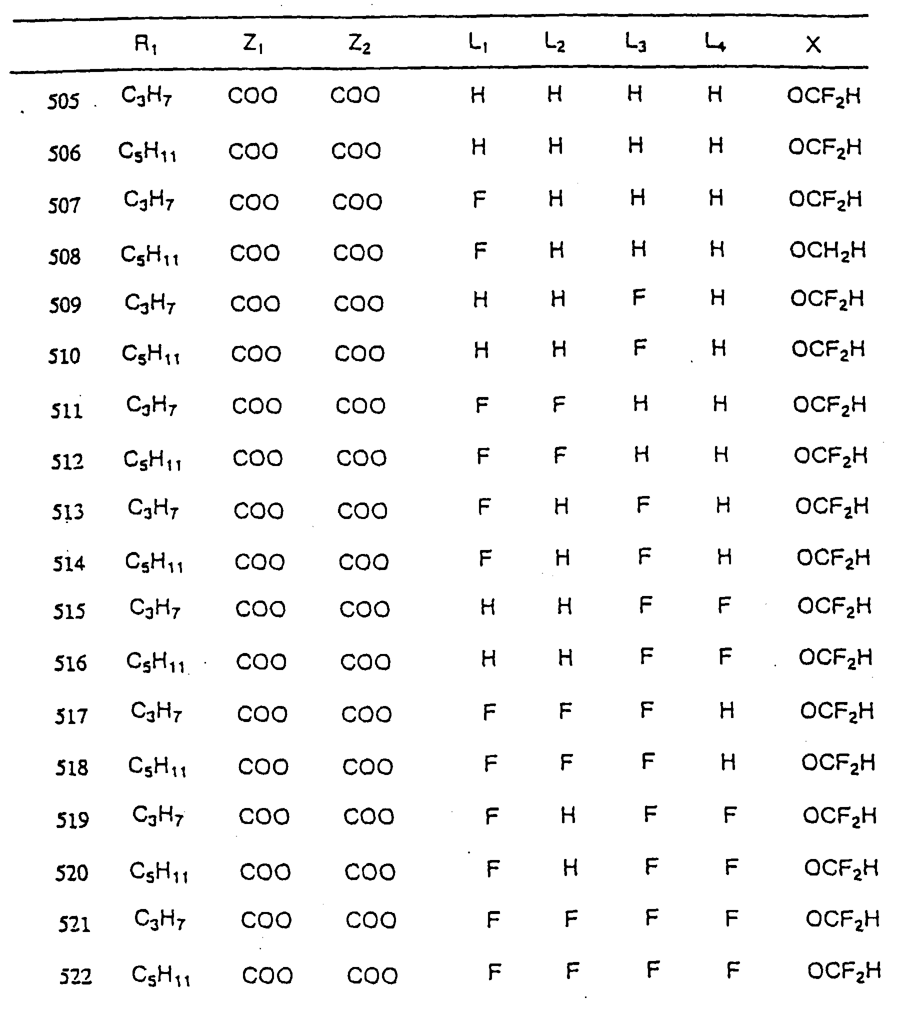

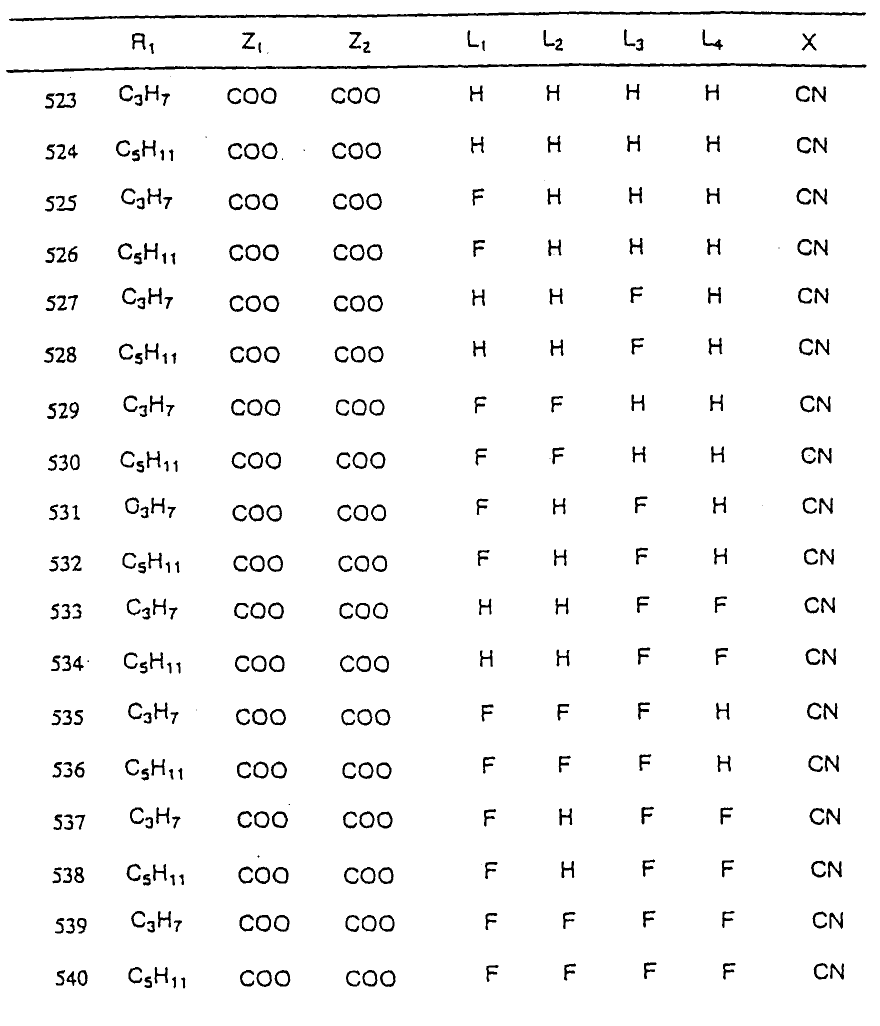

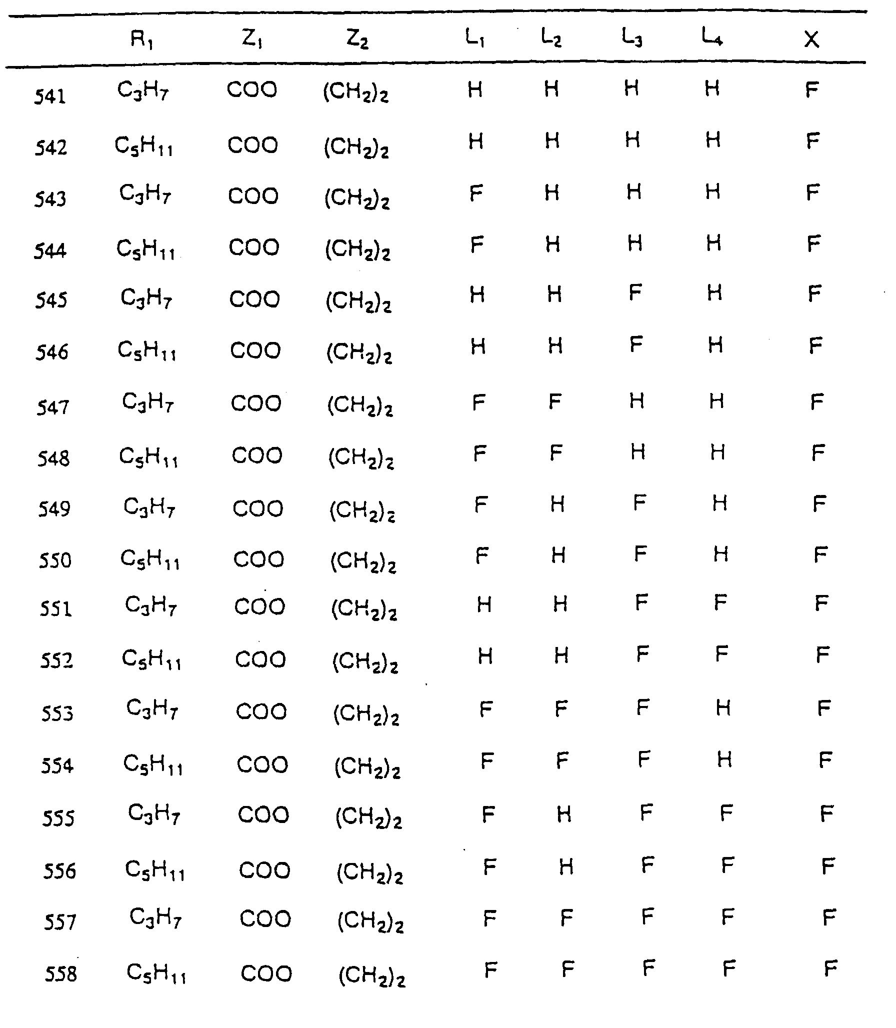

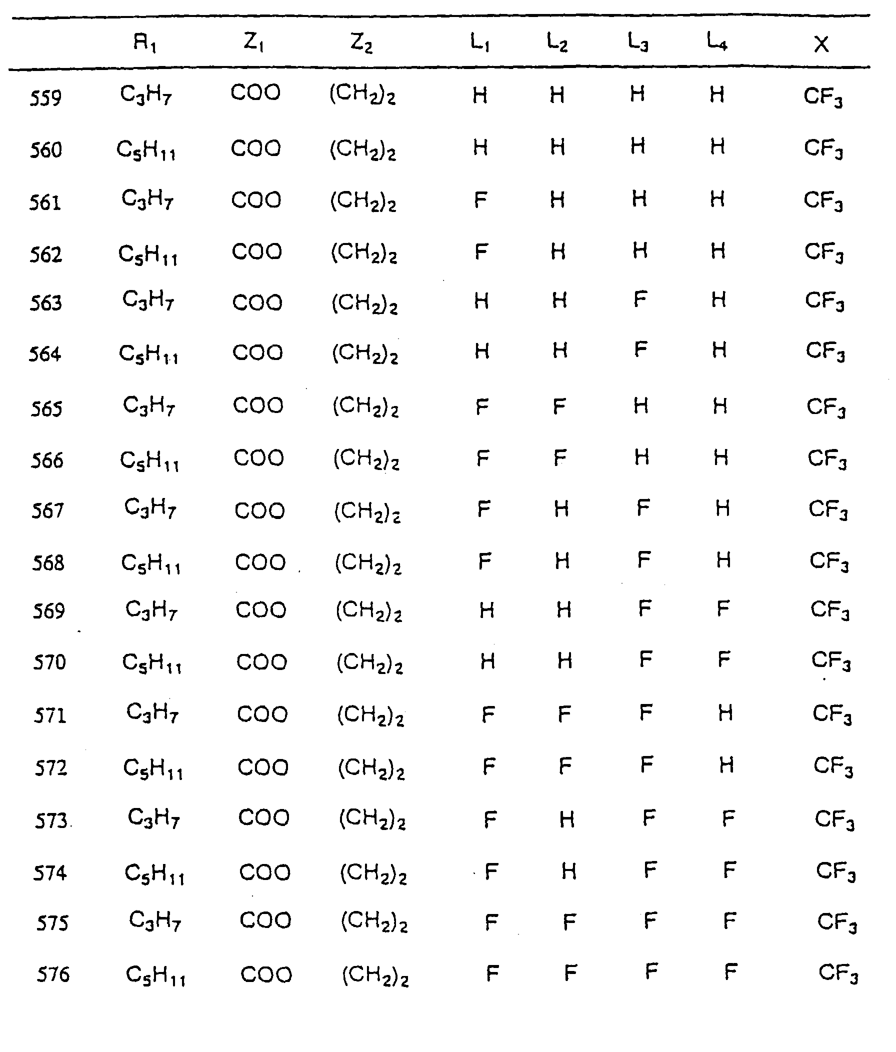

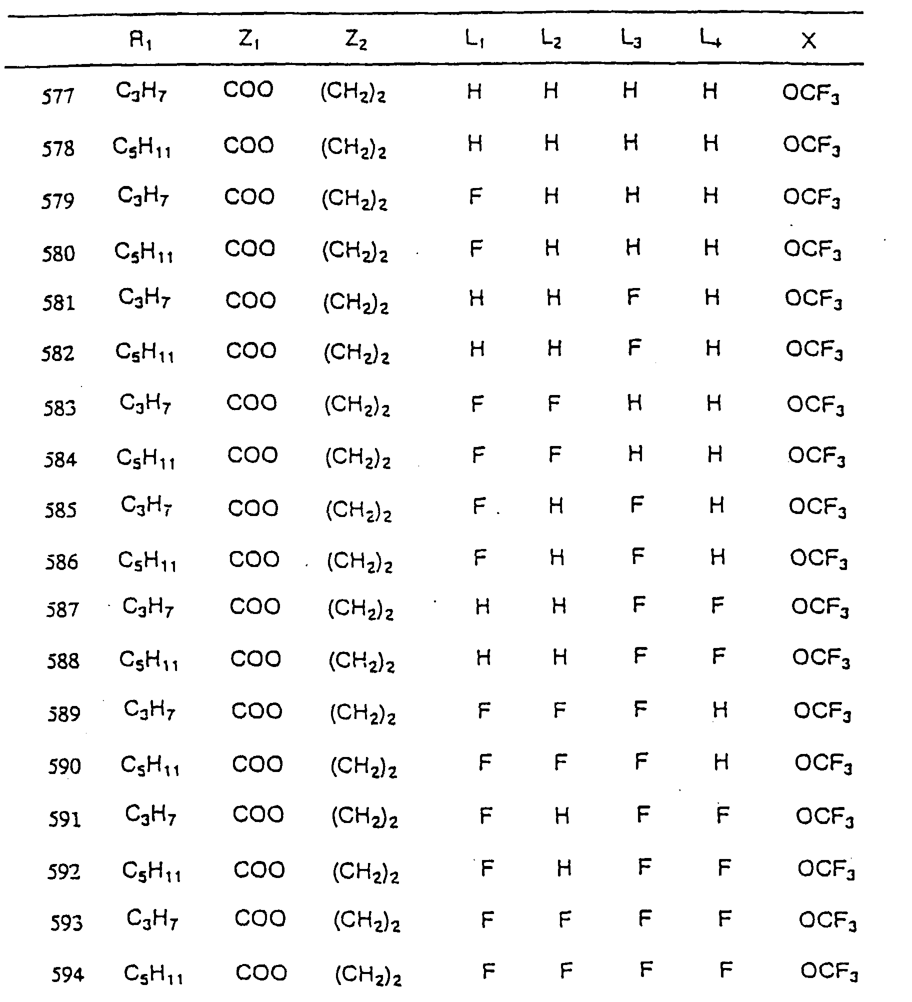

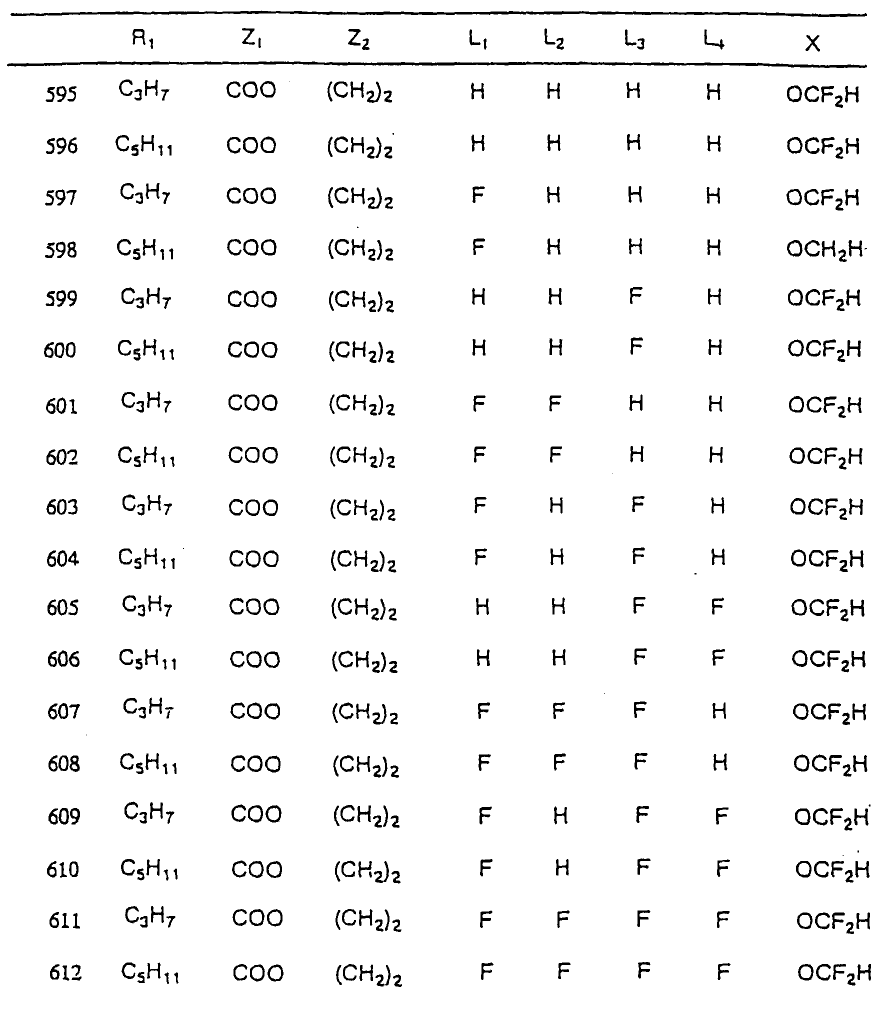

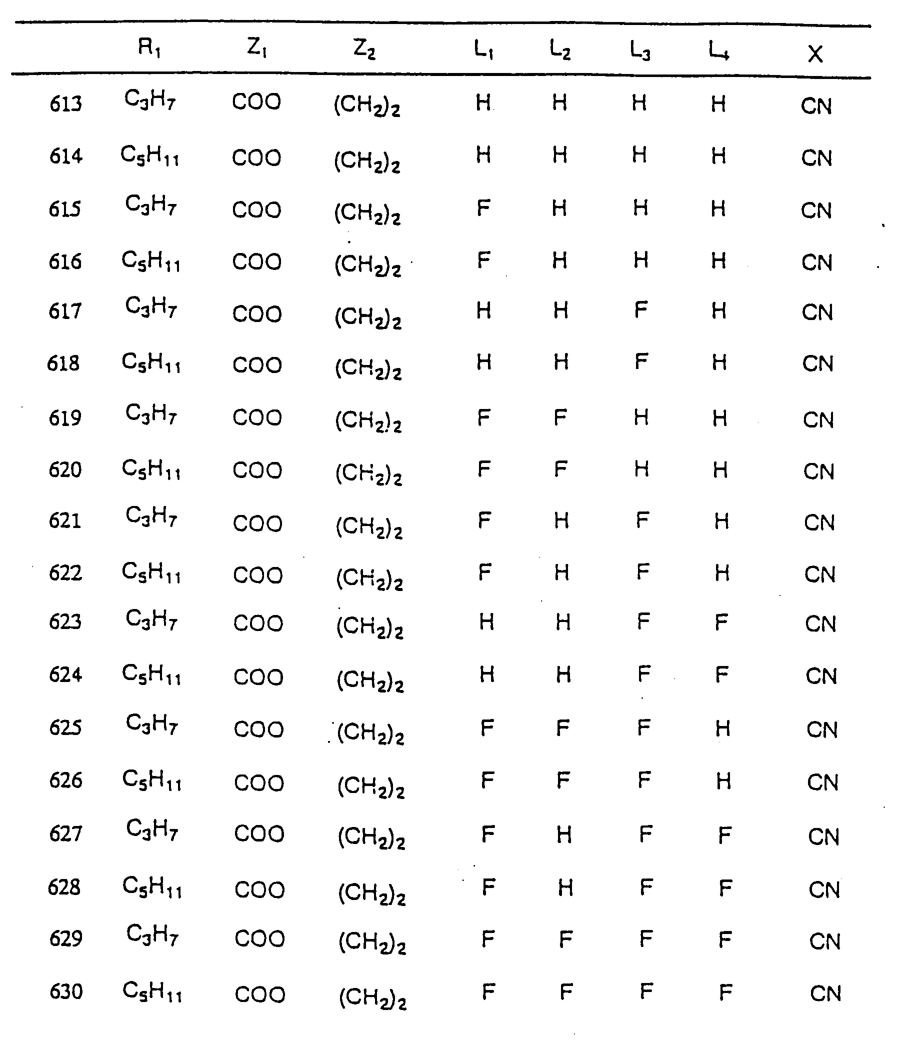

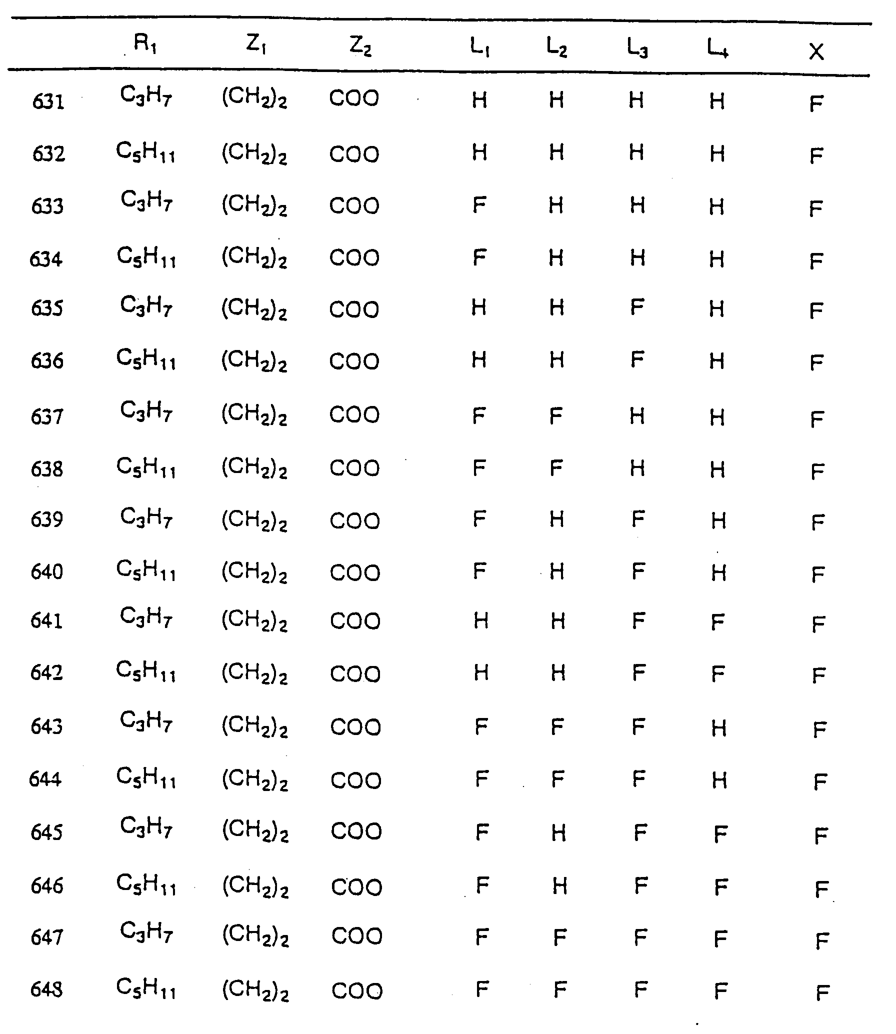

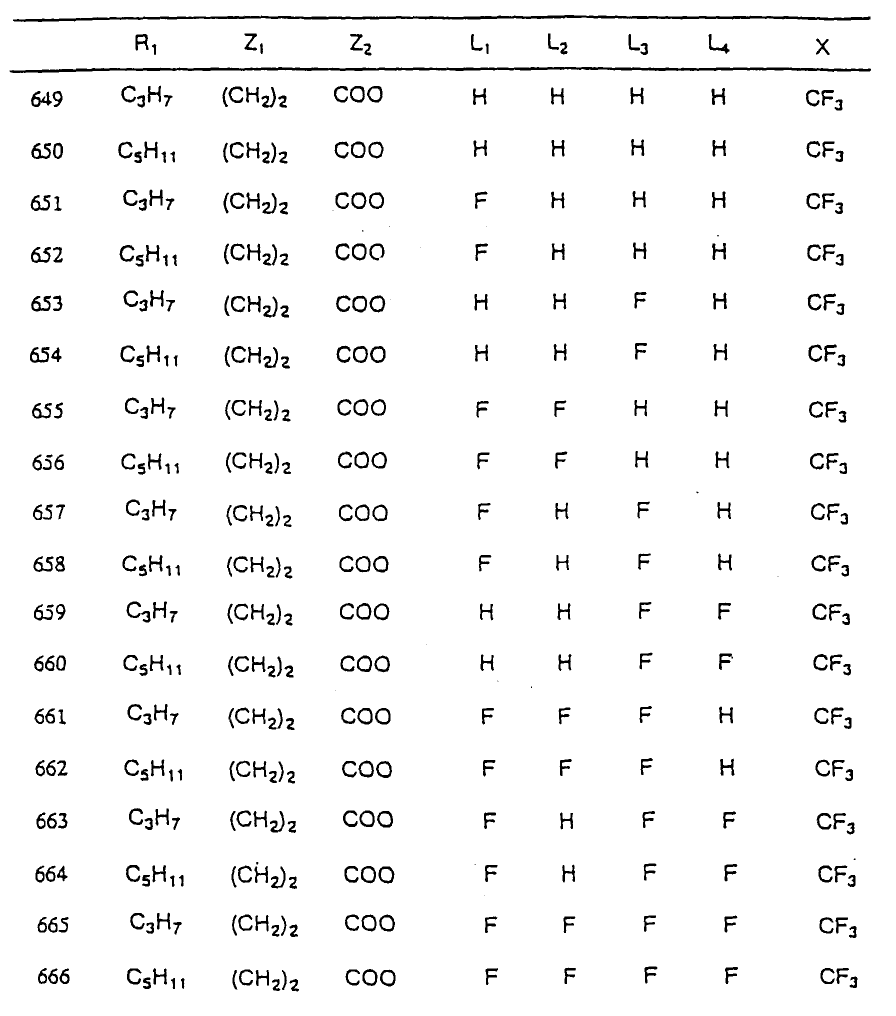

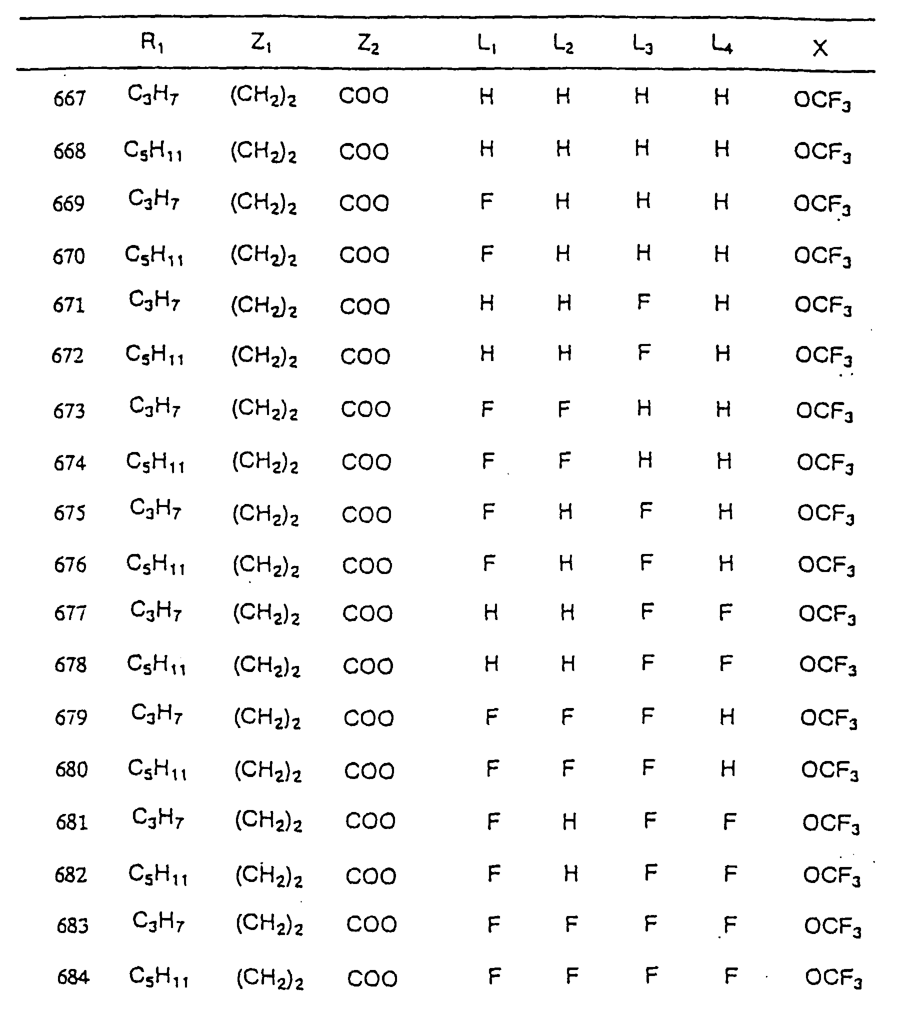

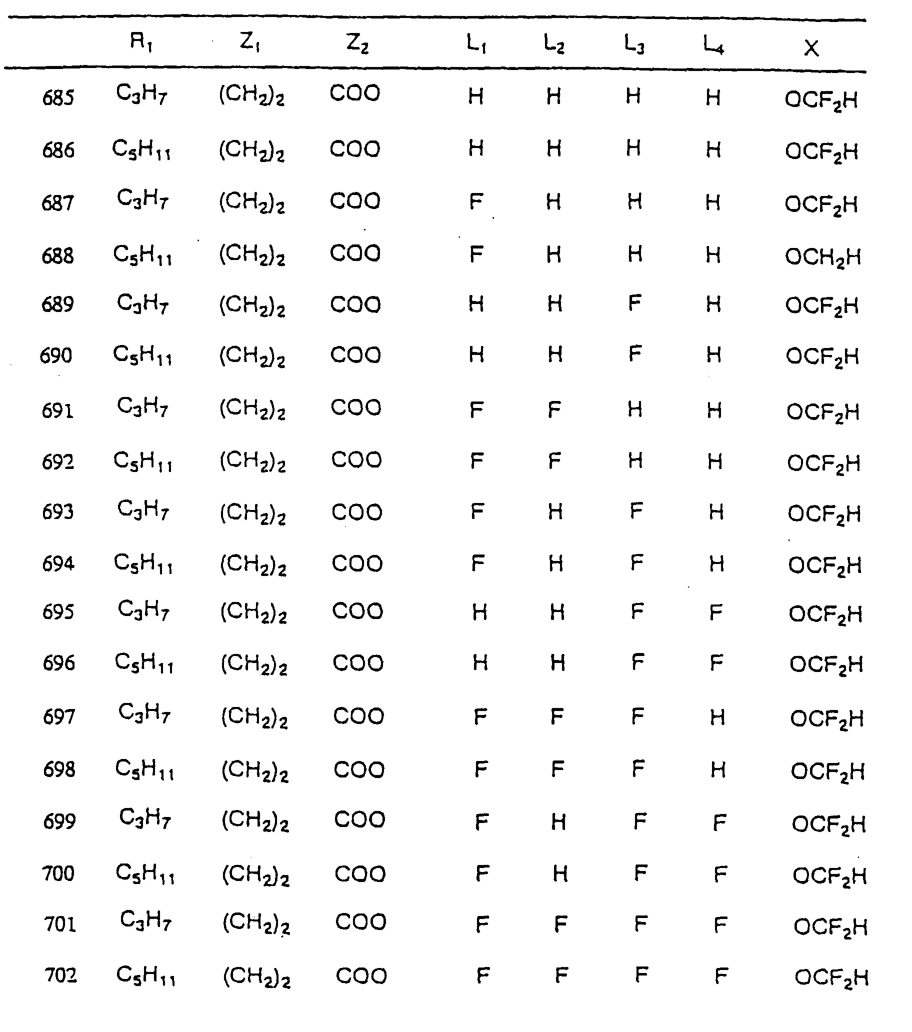

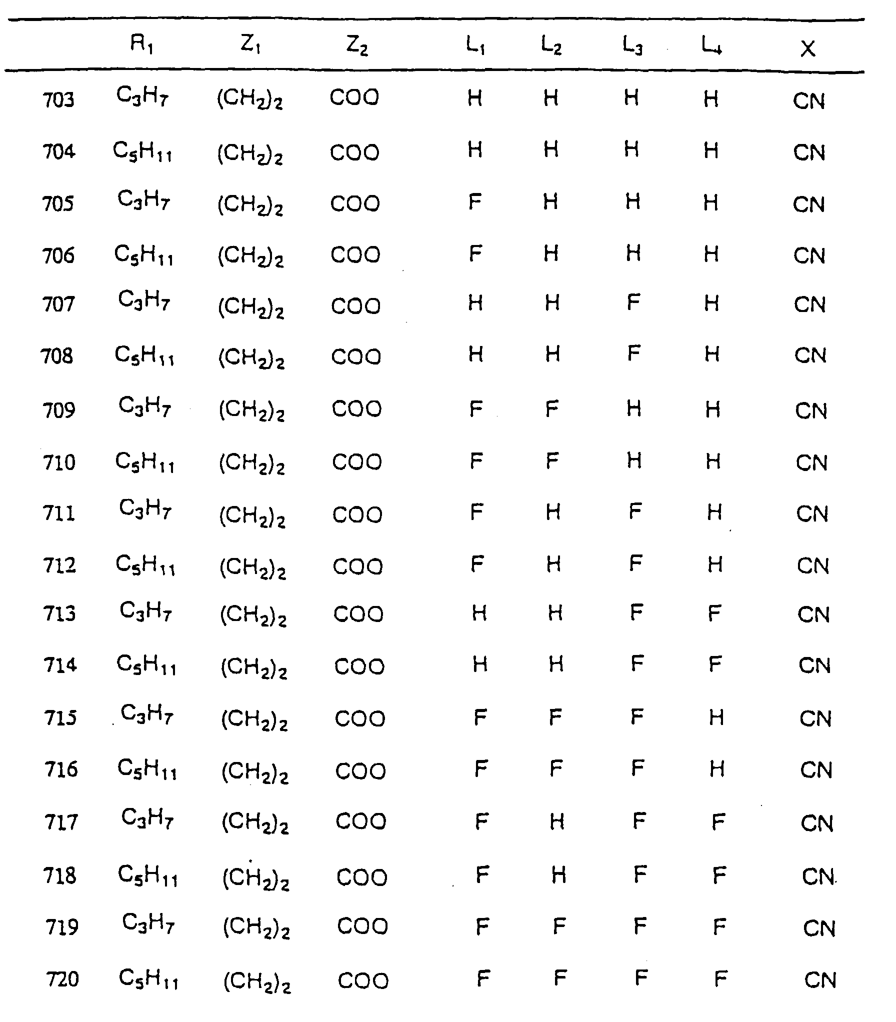

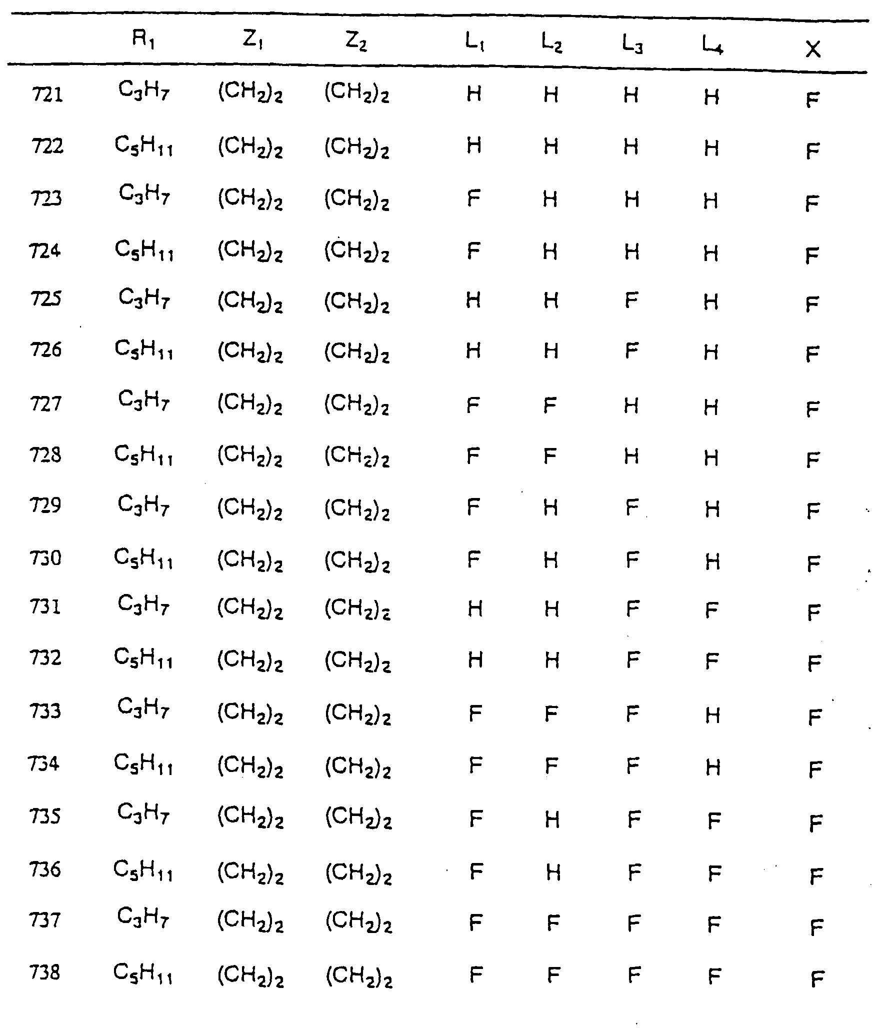

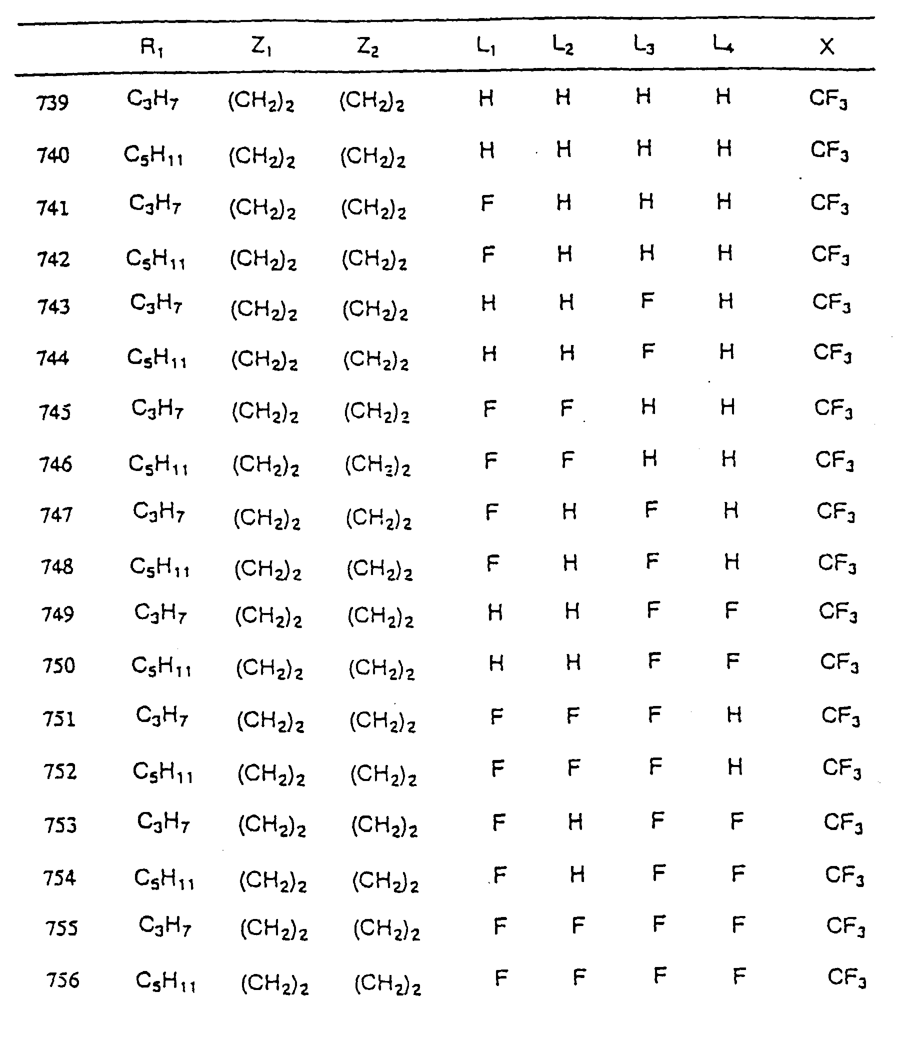

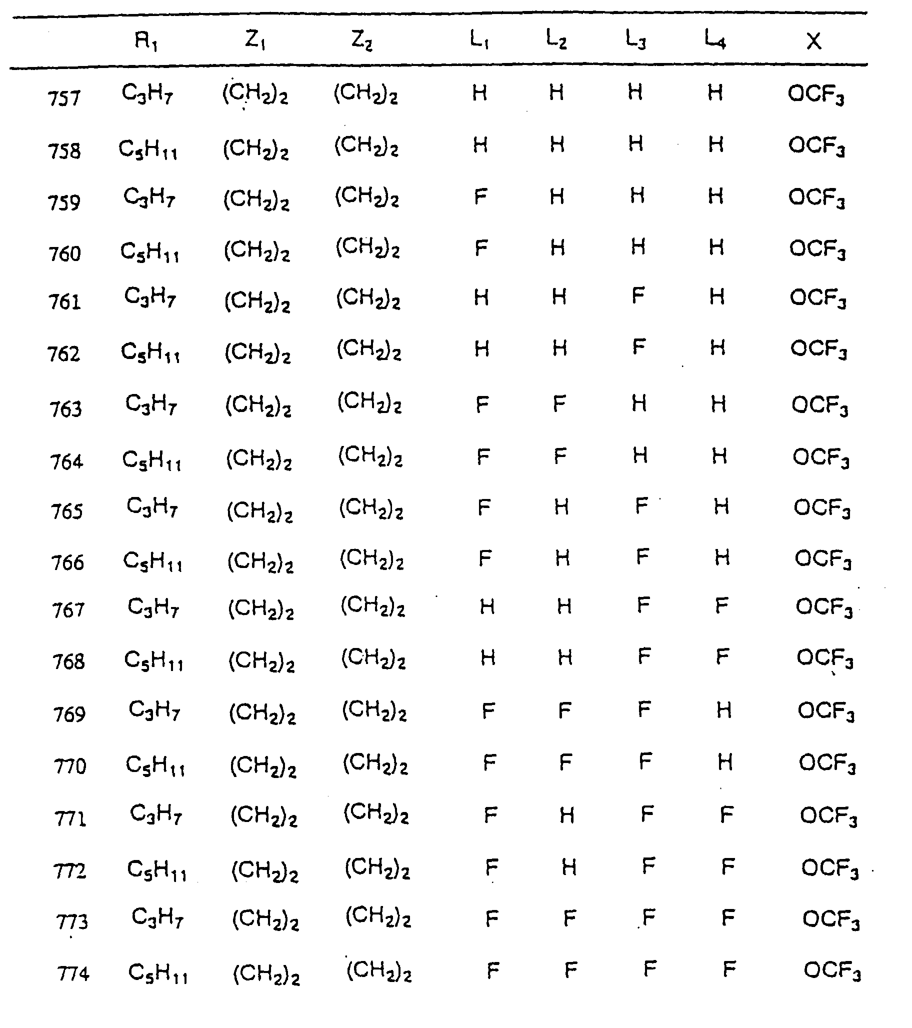

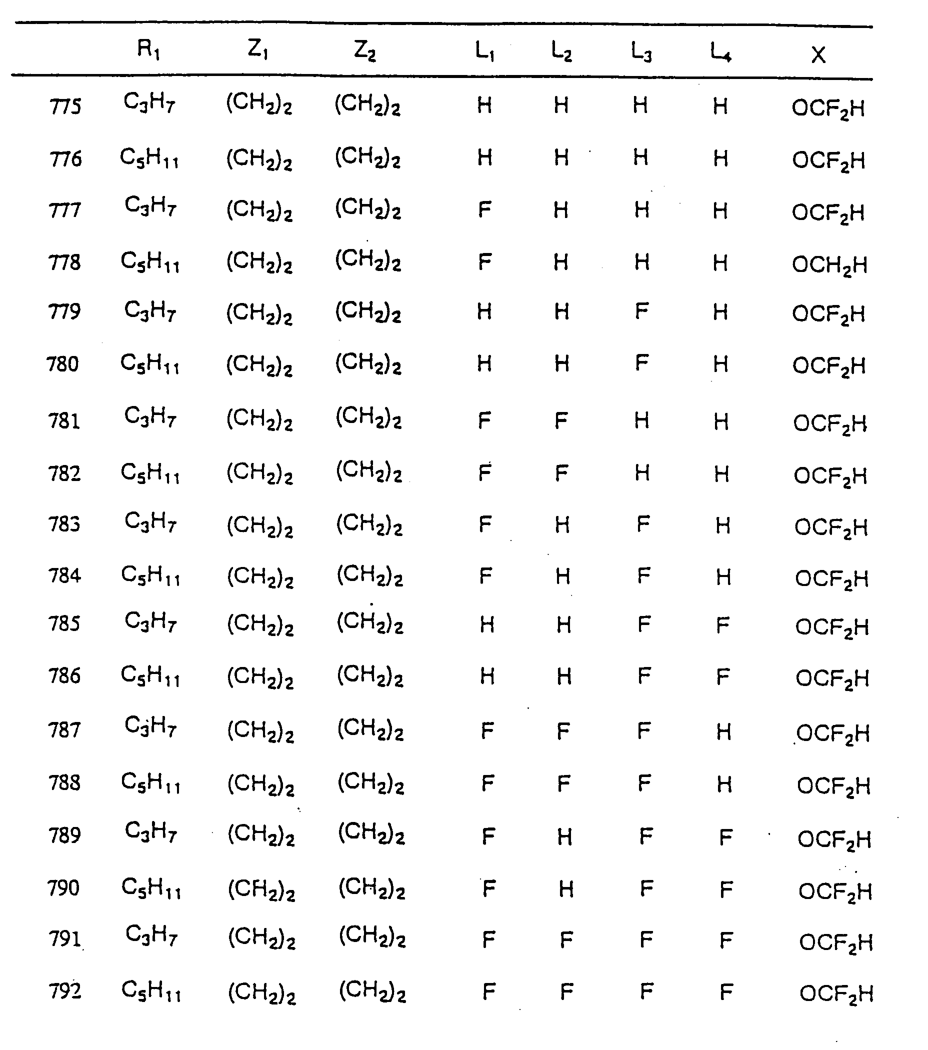

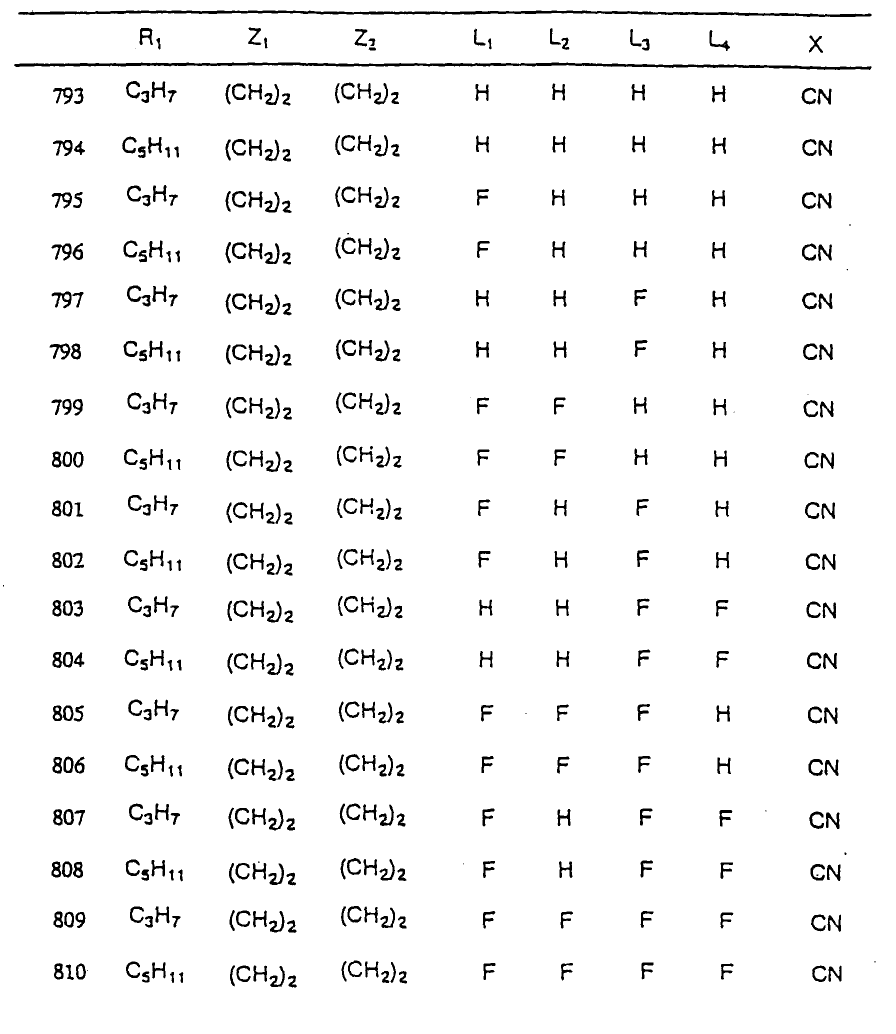

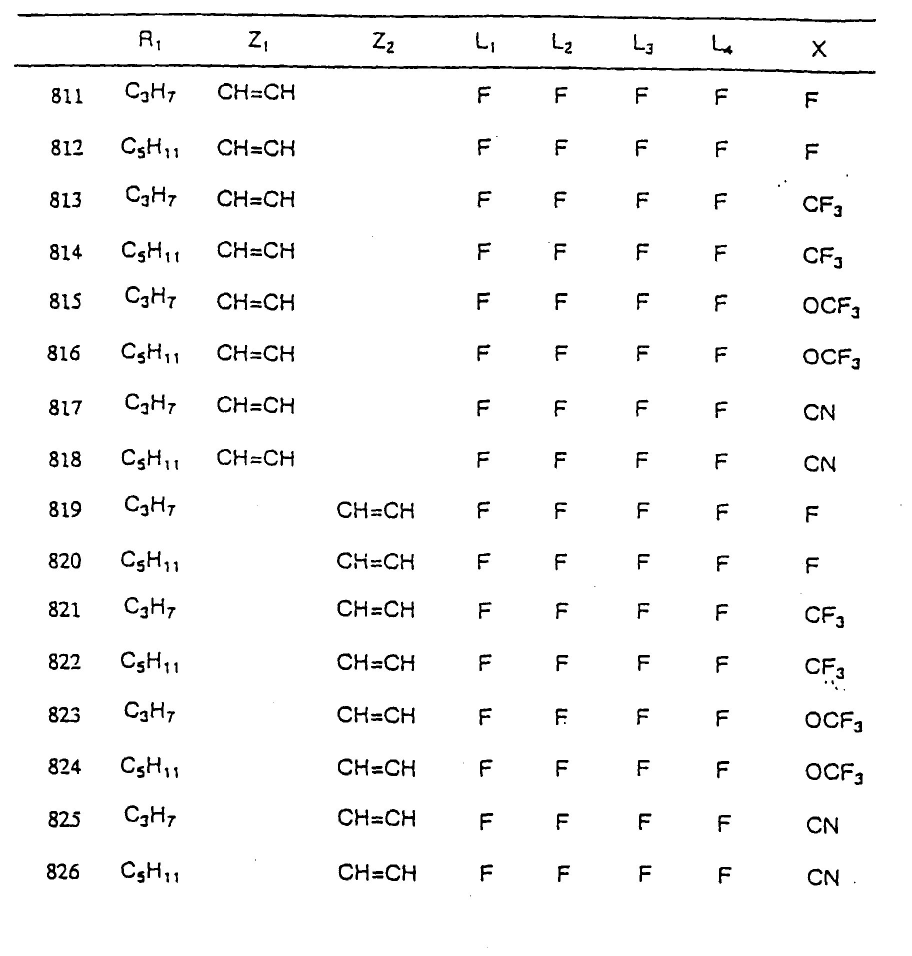

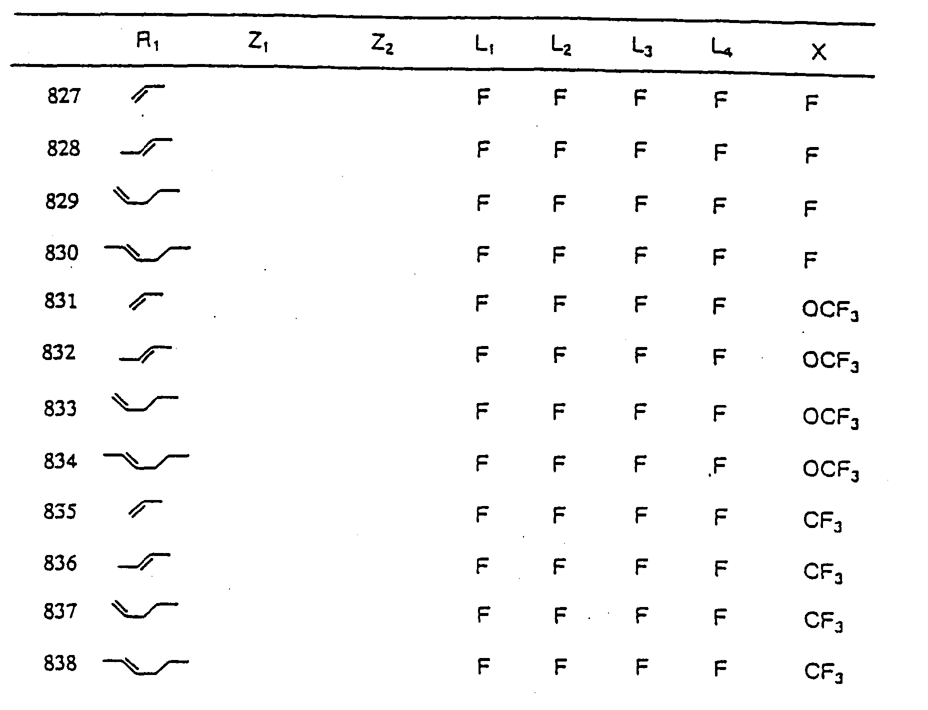

### Example 9 (Use Example 1)

Clearing point (T_{NI}) of a nematic liquid crystal composition comprising the following compounds in the amount shown below was 72.4°C.

| | |
|---|---|
| 4-(trans-4-propylcyclohexyl)benzonitrile | 24 % by weight |
| 4-(trans-4-pentylcyclohexyl)benzonitrile | 36 % by weight |
| 4-(trans-4-heptylcyclohexyl)benzonitrile | 25 % by weight |
| 4-(4-propylphenyl)benzonitrile | 15 % by weight |

This liquid crystal composition filled in a TN cell (twisted nematic cell) having a thickness of 9 µm had a driving threshold voltage of 1.78 V, a dielectric anisotropy value (Δε) of 11.0, an optical anisotropy value (Δn) of 0.137, and a viscosity at 20 °C (η₂₀) of 27.0 mPa·s.

This liquid crystal composition was assumed to be Mother Liquid Crystal A, 85 parts by weight of the Mother Liquid Crystal A was mixed with 15 parts by weight of Compound No. 11, 3,4,5-trifluoro-4'-(trans-4-(4-(trans-4-propylcyclohexyl)butyl) cyclohexyl)biphenyl, obtained by Example 1, and its physical properties were determined. The results were as follows:
T_{NI}: 162.4°C, Δε: 14.3, Vₜₕ: 1.65 V, Δn: 0.170, and η₂₀: 74.8 mPa·s (all values excepting Vₜₕ are extrapolated ones).

While this composition was left in a freezer at -20°C for 40 days, separation of crystals or development of a smectic phase was not observed.

### Example 10 (Use Example 2)

Mother Liquid Crystal A used in Example 9 in an amount of 85 parts by weight was mixed with 15 parts by weight of Compound No. 18, 3,4,5,2',6'-pentafluoro-4'-(trans-4-(4-(trans-4-pentylcyclohexyl)butyl)cyclohexyl)biphenyl, obtained by Example 2, and its physical properties were determined. The results were as follows:
T_{NI}: 114.4°C, Δε: 19.0, Vₜₕ: 1.63 V, Δn: 0.137, and η₂₀: 71.4 mPa·s (all values excepting Vₜₕ are extrapolated ones).

While this composition was left in a freezer at -20°C for 40 days, separation of crystals or development of a smectic phase was not observed.

### Example 11 (Use Example 3)

Mother Liquid Crystal A used in Example 9 in an amount of 85 parts by weight was mixed with 15 parts by weight of Compound No. 25, 4-trifluoromethyl-2',6'-difluoro-4'-(trans-4-(4-(trans-4-propylcyclohexyl)butyl)cyclohexyl)biphenyl, obtained by Example 3, and its physical properties were determined. The results were as follows:
T_{NI}: 132.4°C, Δε: 13.0, Vₜₕ: 1.64 V, Δn: 0.135, and η₂₀: 75.2 mPa·s (all values excepting Vₜₕ are extrapolated ones).

While this composition was left in a freezer at -20°C for 40 days, separation of crystals or development of a smectic phase was not observed.

### Example 12 (Use Example 4)

Mother Liquid Crystal A used in Example 9 in an amount of 85 parts by weight was mixed with 15 parts by weight of Compound No. 49, 3,2',6'-trifluoro-4-trifluoromethoxy-4'-(trans-4-(4-(trans-4-propylcyclohexyl)butyl)cyclohexyl)biphenyl, obtained by Example 4, and its physical properties were determined. The results were as follows:
T_{NI}: 130.4°C, Δε: 13.7, Vₜₕ: 1.66 V, Δn: 0.128, and η₂₀: 76.7 mPa·s (all values excepting Vₜₕ are extrapolated ones).

While this composition was left in a freezer at -20°C for 40 days, separation of crystals or development of a smectic phase was not observed.

### Comparative Example 1

New liquid crystal composition was prepared by the same manner as in Example 9 with the exception that the comparative compound expressed by the formula (a) illustrated above wherein R represents C₃H₇ was used in place of Compound No. 11 of the present invention as the compound to be mixed with Mother Liquid Crystal A, and physical properties of the new composition were determined. As the results, Δε was 12.3 and Vₜₕ was 1.77 V.

When this composition was left in a freezer at -20°C a little longer, separation of crystals was observed 18 days after the composition was put in the freezer.

From the results in Example 9 and Comparative Example 1, it can be understood that Compound No. 11 of the present invention used in the former Example have a higher Δε by about 16 % compared with the conventional compound used in the latter Example whereas both compounds fall within four-ring system ones, and Compound No. 11 exhibits a clearly lower Vₜₕ (by about 7 %) based on the higher Δε. Further, it can be seen that Compound No. 11 is sharply better even in mutual solubility. Example 13 (Use Example 5)

New liquid crystal composition was prepared by the same manner as in Example 9 with the exception that Mother Liquid Crystal B prepared by the following method was used in place of Mother Liquid Crystal A, and voltage holding ratio (VHR) of the composition at different temperatures were determined:

### Preparation of Mother Liquid Crystal B:

Following Mother Liquid Crystals B₁, B₂, and B₃ were mixed in the ratio of 1:1:2
Mother Liquid Crystal B₁:
   Mixture of the same amount of
   1,2-difluoro-4-(trans-4-(trans-4-ethylcyclohexyl)cyclohexyl) benzene,
   1,2-difluoro-4-(trans-4-(trans-4-propylcyclohexyl)cyclohexyl) benzene, and
   1,2-difluoro-4-(trans-4-(trans-4-pentylcyclohexyl)cyclohexyl) benzene.
Mother Liquid Crystal B₂:
   Mixture of
   1,2-difluoro-4-(trans-4-(2-(trans-4-ethylcyclohexyl)ethyl) cyclohexyl)benzene,
   1,2-difluoro-4-(trans-4-(2-(trans-4-proplcyclohexyl)ethyl) cyclohexyl)benzene, and
   1,2-difluoro-4-(trans-4-(2-(trans-4-pentylcyclohexyl)ethyl) cyclohexyl)benzene
   in the ratio of 2:1:2.
Mother Liquid Crystal B₃:
   Mixture of
   3,4-difluoro-4'-(trans-4-ethylcyclohexyl)biphenyl,
   3,4-difluoro-4'-(trans-4-propylcyclohexyl)biphenyl, and
   3,4-difluoro-4'-(trans-4-pentylcyclohexyl)biphenyl
   in the ratio of 1:1:2.

Results are shown by line x in Fig. 1.

### Comparative Example 2

New liquid crystal was prepared by the same manner as in Example 13 with the exception that a comparative compound expressed by the formula (a) illustrated above wherein R represents C₃H₇ was used in place of Compound No. 11 of the present invention as a compound to be mixed with Mother Liquid Crystal B, and VHR of the mother liquid crystal at different temperatures were determined.

Results were shown by line y in Fig. 1.

As will be clear from Fig. 1, whereas VHR begins to sharply lower at about 75°C in Comparative Example 2 (reference is made to line y), such sharp lowering of VHR is not observed in the case of Example 13 of the present invention (reference is made to line x) and it shows a higher value than Comparative Example 2 by about 4 % even at 120°C. Accordingly, it can be understood that the compossition in the latter case (Example 13 of the present invention) is smaller in the temperature dependency of VHR.

Further Use Examples (examples of compositions) are shown below in which compounds are designated according to the definition shown in the following Table 1. In Table 1, particular groups or structures written in each of columns of left hand side terminal group, bonding group, ring structure, and right hand side terminal group correspond to the symbols written in the same line in the same column (In Table 1, m, n, and k represent an integer). Further, the content of compounds means % by weight unless otherwise specified.

Among the characteristics shown in the examples of compositions, Δn, Δε, and Vₜₕ are values determined at 25°C, respectively.

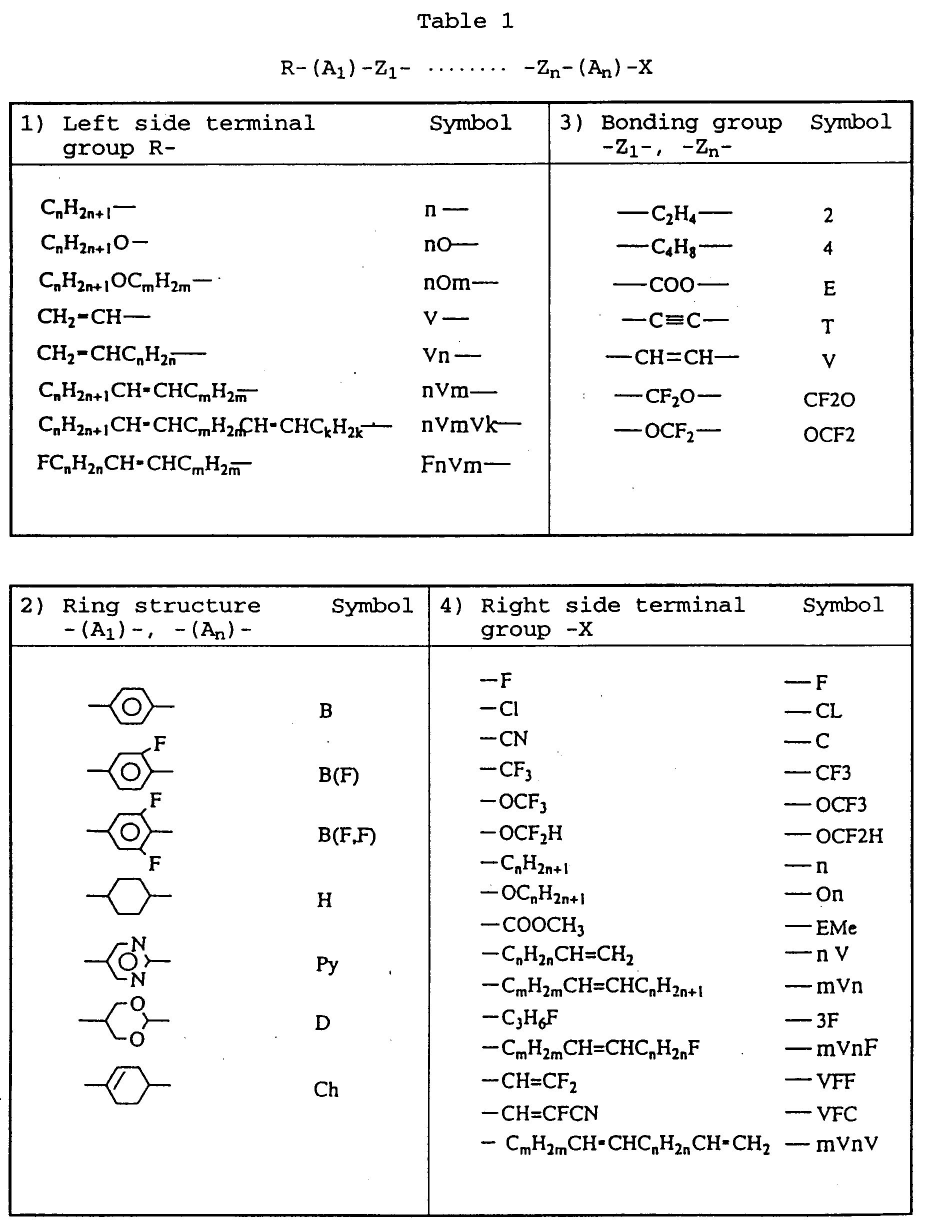

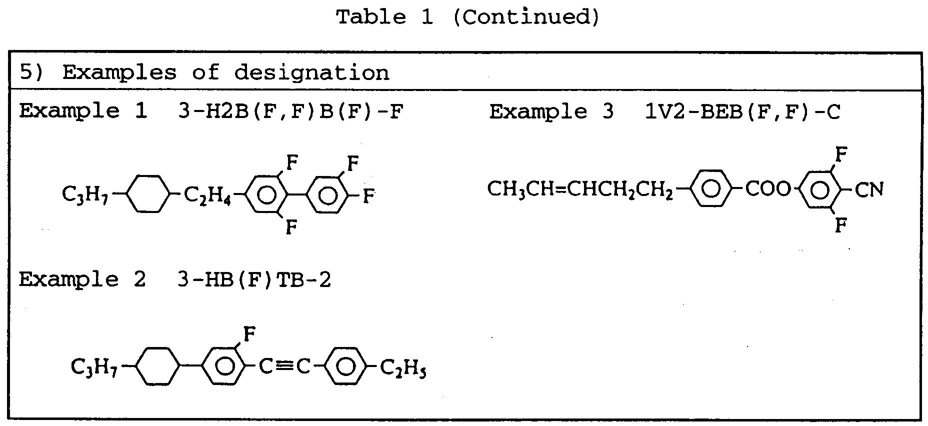

### Example 14 (Use Example 6)

Liquid crystal composition comprising the following compounds in the content shown below was prepared:

| | |
|---|---|
| 3-H4HBB(F,F)-F | 3.0 % |
| 5-H4HB(F,F)B(F,F)-F | 4.0 % |
| 1V2-BEB(F,F)-C | 5.0 % |
| 3-HB-C | 18.0 % |
| 1-BTB-3 | 5.0 % |
| 2-BTB-1 | 10.0 % |
| 3-HH-4 | 11.0 % |
| 3-HHB-1 | 11.0 % |
| 3-HHB-3 | 9.0 % |
| 3-H2BTB-2 | 4.0 % |
| 3-H2BTB-3 | 4.0 % |
| 3-H2BTB-4 | 4.0 % |
| 3-HB(F)TB-2 | 6.0 % |
| 3-HB(F)TB-3 | 6.0 % |

Properties of this liquid crystal composition were determined and the results were as follows:
- T_{NI}=: 87.0°C
- η₂₀=: 16.4 mPa·s
- Δn=: 0.157
- Δε=: 7.1
- Vₜₕ=: 2.16 V

### Example 15 (Use Example 7)

Liquid crystal composition comprising the following compounds in the content shown below was prepared:

| | |
|---|---|
| 3-H4HBB(F,F)-F | 2.0 % |
| 5-H4HB(F,F)B(F,F)-F | 2.0 % |
| 3-H4HB(F,F)B(F)-OCF3 | 3.0 % |
| 3-H4HB(F,F)B-CF3 | 3.0 % |
| V2-HB-C | 12.0 % |
| 1V2-HB-C | 12.0 % |
| 3-HB-C | 14.0 % |
| 3-HB(F)-C | 5.0 % |
| 2-BTB-1 | 2.0 % |
| 3-HH-4 | 8.0 % |
| 3-HB-O2 | 10.0 % |
| 2-HHB-C | 3.0 % |
| 3-HHB-C | 6.0 % |
| 3-HB(F)TB-2 | 8.0 % |
| 3-H2BTB-2 | 5.0 % |
| 3-H2BTB-3 | 5.0 % |

Properties of this liquid crystal composition were determined and the results were as follows:
- T_{NI}=: 89.4°C
- η₂₀=: 22.4 mPa·s
- Δn=: 0.148
- Δε=: 8.5
- Vₜₕ=: 2.21 V

### Example 16 (Use Example 8)

Liquid crystal composition comprising the following compounds in the content shown below was prepared:

| | |
|---|---|
| 3-H4HB(F,F)B(F)-OCF3 | 8.0 % |
| 2O1-BEB(F)-C | 5.0 % |
| 3O1-BEB(F)-C | 13.0 % |
| 4O1-BEB(F)-C | 10.0 % |
| 5O1-BEB(F)-C | 10.0 % |
| 2-HHB(F)-C | 15.0 % |
| 3-HHB(F)-C | 15.0 % |
| 3-HB(F)TB-2 | 4.0 % |
| 3-HB(F)TB-3 | 4.0 % |
| 3-HB(F)TB-4 | 4.0 % |
| 3-HH4 | 8.0 % |
| 3-HHB-1 | 2.0 % |
| 3-HHB-O1 | 2.0 % |

Properties of this liquid crystal composition were determined and the results were as follows:
- T_{NI}=: 95.3°C
- η₂₀=: 85.1 mPa·s
- Δn=: 0.143
- Δε =: 29.6
- Vₜₕ=: 0.94 V

### Example 17 (Use Example 9)

Liquid crystal composition comprising the following compounds in the content shown below was prepared:

| | |
|---|---|
| 3-H4HB(F,F)B-CF3 | 2.0 % |
| 5-PyB-F | 4.0 % |
| 3-PyB(F)-F | 4.0 % |
| 2-BB-C | 5.0 % |
| 4-BB-C | 4.0 % |
| 5-BB-C | 3.0 % |
| 2-PyB-2 | 2.0 % |
| 3-PyB-2 | 2.0 % |
| 4-PyB-2 | 2.0 % |
| 6-PyB-O5 | 3.0 % |
| 6-PyB-O6 | 3.0 % |
| 6-PyB-O7 | 3.0 % |
| 6-PyB-O8 | 3.0 % |
| 3-PyBB-F | 6.0 % |
| 4-PyBB-F | 6.0 % |
| 5-PyBB-F | 6.0 % |
| 3-HHB-1 | 6.0 % |
| 3-HHB-3 | 8.0 % |
| 2-H2BTB-2 | 4.0 % |
| 2-H2BTB-3 | 4.0 % |
| 2-H2BTB-4 | 5.0 % |
| 3-H2BTB-2 | 5.0 % |
| 3-H2BTB-3 | 5.0 % |
| 3-H2BTB-4 | 5.0 % |

Properties of this liquid crystal composition were determined and the results were as follows:
- T_{NI}=: 95.7°C
- η₂₀=: 35.2 mPa·s
- Δn=: 0.200
- Δε =: 6.4
- Vₜₕ=: 2.30 V

### Example 18 (Use Example 10)

Liquid crystal composition comprising the following compounds in the content shown below was prepared:

| | |
|---|---|
| 5-H4HB(F,F)B(F,F)-F | 3.0 % |
| 3-DB-C | 10.0 % |
| 4-DB-C | 10.0 % |
| 2-BEB-C | 12.0 % |
| 3-BEB-C | 4.0 % |
| 3-PyB(F)-F | 6.0 % |
| 3-HEB-O4 | 8.0 % |
| 4-HEB-O2 | 6.0 % |
| 5-HEB-O1 | 6.0 % |
| 3-HEB-O2 | 5.0 % |
| 5-HEB-O2 | 4.0 % |
| 5-HEB-5 | 5.0 % |
| 4-HEB-5 | 5.0 % |
| 1O-BEB-2 | 4.0 % |
| 3-HHB-1 | 6.0 % |
| 3-HHEBB-C | 3.0 % |
| 3-HBEBB-C | 3.0 % |

Properties of this liquid crystal composition were determined and the results were as follows:
- T_{NI}=: 69.4°C
- η₂₀=: 38.8 mPa·s
- Δn=: 0.119
- Δε =: 11.2
- Vₜₕ=: 1.42 V

### Example 19 (Use Example 11)

Liquid crystal composition comprising the following compounds in the content shown below was prepared:

| | |
|---|---|
| 3-H4HB(F,F)B-CF3 | 6.0 % |
| 3-H4HB(F,F)B(F)-OCF3 | 3.0 % |
| 3-HB-C | 12.0 % |
| 7-HB-C | 3.0 % |
| 1O1-HB-C | 10.0 % |
| 3-HB(F)-C | 7.0 % |
| 2-PyB-2 | 2.0 % |
| 3-PyB-2 | 2.0 % |
| 4-PyB-2 | 2.0 % |
| 1O1-HH-3 | 7.0 % |
| 2-BTB-O1 | 7.0 % |
| 3-HHB-1 | 7.0 % |
| 3-HHB-F | 4.0 % |
| 3-HHB-O1 | 4.0 % |
| 3-HB-O2 | 8.0 % |
| 3-H2BTB-2 | 3.0 % |
| 3-H2BTB-3 | 3.0 % |
| 2-PyBH-3 | 4.0 % |
| 3-PyBH-3 | 3.0 % |
| 3-PyBB-2 | 3.0 % |

Properties of this liquid crystal composition were determined and the results were as follows:
- T_{NI}=: 79.3°C
- η₂₀=: 21.0 mPa·s
- Δn=: 0.136
- Δε =: 7.6
- Vₜₕ=: 1.96 V

### Example 20 (Use Example 12)

Liquid crystal composition comprising the following compounds in the content shown below was prepared:

| | |
|---|---|
| 3-H4HBB(F,F)-F | 2.0 % |
| 2O1-BEB(F)-C | 5.0 % |
| 3O1-BEB(F)-C | 10.0 % |
| 5O1-BEB(F)-C | 4.0 % |
| 1V2-BEB(F,F)-C | 10.0 % |
| 3-HH-EMe | 10.0 % |
| 3-HB-O2 | 18.0 % |
| 7-HEB-F | 2.0 % |
| 3-HHEB-F | 2.0 % |
| 5-HHEB-F | 2.0 % |
| 3-HBEB-F | 4.0 % |
| 2O1-HBEB(F)-C | 2.0 % |
| 3-HBEB(3F,5F)-C | 4.0 % |
| 3-HHB-F | 4.0 % |
| 3-HHB-O1 | 4.0 % |
| 3-HHB-3 | 13.0 % |
| 3-HEBEB-F | 2.0 % |
| 3-HEBEB-1 | 2.0 % |

Properties of this liquid crystal composition were determined and the results were as follows:
- T_{NI}=: 79.6°C
- η₂₀=: 35.4 mPa·s
- Δn=: 0.116
- Δε =: 23.0
- Vₜₕ=: 1.19 V

### Example 21 (Use Example 13)

Liquid crystal composition comprising the following compounds in the content shown below was prepared:

| | |
|---|---|
| 3-H4HBB(F,F)-F | 2.0 % |
| 3-H4HB(F,F)B-CF3 | 4.0 % |
| 2O1-BEB(F)-C | 5.0 % |
| 3O1-BEB(F)-C | 10.0 % |
| 5O1-BEB(F)-C | 4.0 % |
| 1V2-BEB(F,F)-C | 12.0 % |
| 3-HB-O2 | 17.0 % |
| 3-HH-4 | 3.0 % |
| 3-HHB-F | 3.0 % |
| 3-HHB-1 | 8.0 % |
| 3-HHB-O1 | 4.0 % |
| 3-HBEB-F | 4.0 % |
| 3-HHEB-F | 7.0 % |
| 3-H2BTB-2 | 4.0 % |
| 3-H2BTB-3 | 4.0 % |
| 3-H2BTB-4 | 4.0 % |
| 3-HB(F)TB-2 | 5.0 % |

Properties of this liquid crystal composition were determined and the results were as follows:
- T_{NI}=: 88.4°C
- η₂₀=: 38.8 mPa·s
- Δn=: 0.141
- Δε =: 25.7
- Vₜₕ=: 1.19 V

### Example 22 (Use Example 14)

Liquid crystal composition comprising the following compounds in the content shown below was prepared:

| | |
|---|---|
| 3-H4HB(F,F)B(F)-OCF3 | 6.0 % |
| 2-BEB-C | 12.0 % |
| 3-BEB-C | 4.0 % |
| 4-BEB-C | 6.0 % |
| 3-HB-C | 22.0 % |
| 3-HEB-O4 | 12.0 % |
| 4-HEB-O2 | 8.0 % |
| 5-HEB-O1 | 8.0 % |
| 3-HEB-O2 | 6.0 % |
| 5-HEB-O2 | 5.0 % |
| 3-HB-O2 | 4.0 % |
| 3-HHB-1 | 3.0 % |
| 3-HHB-O1 | 4.0 % |

Properties of this liquid crystal composition were determined and the results were as follows:
- T_{NI}=: 62.2°C
- η₂₀=: 28.3 mPa·s
- Δn=: 0.110
- Δε =: 9.8
- Vₜₕ=: 1.39 V

### Example 23 (Use Example 15)

Liquid crystal composition comprising the following compounds in the content shown below was prepared:

| | |
|---|---|
| 5-H4HB(F,F)B(F,F)-F | 3.0 % |
| 3-H4HB(F,F)B(F)-OCF3 | 5.0 % |
| 2-BEB-C | 7.0 % |
| 5-BB-C | 7.0 % |
| 7-BB-C | 7.0 % |
| 1-BTB-3 | 7.0 % |
| 2-BTB-1 | 10.0 % |
| 1O-BEB-2 | 10.0 % |
| 1O-BEB-5 | 12.0 % |
| 3-HB-O2 | 7.0 % |
| 2-HHB-1 | 4.0 % |
| 3-HHB-F | 4.0 % |
| 3-HHB-1 | 7.0 % |
| 3-HHB-O1 | 4.0 % |
| 3-HHB-3 | 6.0 % |

Properties of this liquid crystal composition were determined and the results were as follows:
- T_{NI}=: 65.5°C
- η₂₀=: 21.4 mPa·s
- Δn=: 0.149
- Δε =: 5.9
- Vₜₕ=: 1.91 V

### Example 24 (Use Example 16)

Liquid crystal composition comprising the following compounds in the content shown below was prepared:

| | |
|---|---|
| 3-H4HBB(F,F)-F | 5.0 % |
| 2-HHB(F)-F | 17.0 % |
| 3-HHB(F)-F | 17.0 % |
| 5-HHB(F)-F | 16.0 % |
| 2-H2HB(F)-F | 10.0 % |
| 3-H2HB(F)-F | 5.0 % |
| 5-H2HB(F)-F | 5.0 % |
| 2-HBB(F)-F | 6.0 % |
| 3-HBB(F)-F | 6.0 % |
| 5-HBB(F)-F | 13.0 % |

Properties of this liquid crystal composition were determined and the results were as follows:
- T_{NI}=: 103.5°C
- η₂₀=: 28.7 mPa·s
- Δn=: 0.088
- Δε =: 5.5
- Vₜₕ=: 1.98 V

### Example 25 (Use Example 17)

Liquid crystal composition comprising the following compounds in the content shown below was prepared:

| | |
|---|---|
| 3-H4HBB(F,F)-F | 2.0 % |
| 5-H4HB(F,F)B(F,F)-F | 3.0 % |
| 7-HB(F)-F | 5.0 % |
| 5-H2B(F)-F | 5.0 % |
| 3-HB-O2 | 10.0 % |
| 3-HH-4 | 5.0 % |
| 2-HHB(F)-F | 10.0 % |
| 3-HHB(F)-F | 10.0 % |
| 5-HHB(F)-F | 10.0 % |
| 3-H2HB(F)-F | 3.0 % |
| 2-HBB(F)-F | 3.0 % |
| 3-HBB(F)-F | 3.0 % |
| 5-HBB(F)-F | 3.0 % |
| 2-H2BB(F)-F | 5.0 % |
| 3-H2BB(F)-F | 6.0 % |
| 3-HHB-1 | 8.0 % |
| 3-HHB-O1 | 5.0 % |
| 3-HHB-3 | 4.0 % |

Properties of this liquid crystal composition were determined and the results were as follows:
- T_{NI}=: 90.9°C
- η₂₀=: 20.0 mPa·s
- Δn=: 0.095
- Δε =: 3.9
- Vₜₕ=: 2.50 V

### Example 26 (Use Example 18)

Liquid crystal composition comprising the following compounds in the content shown below was prepared:

| | |
|---|---|
| 3-H4HB(F,F)B-CF3 | 4.0 % |
| 3-H4HB(F,F)B(F)-OCF3 | 5.0 % |
| 7-HB(F,F)-F | 3.0 % |
| 3-HB-O2 | 7.0 % |
| 2-HHB(F)-F | 10.0 % |
| 3-HHB(F)-F | 10.0 % |
| 5-HHB(F)-F | 10.0 % |
| 2-HBB(F)-F | 9.0 % |
| 3-HBB(F)-F | 9.0 % |
| 5-HBB(F)-F | 12.0 % |
| 2-HBB-F | 4.0 % |
| 3-HBB-F | 4.0 % |
| 5-HBB-F | 3.0 % |
| 3-HBB(F,F)-F | 5.0 % |
| 5-HBB(F,F)-F | 5.0 % |

Properties of this liquid crystal composition were determined and the results were as follows:
- T_{NI}=: 88.5°C
- η₂₀=: 26.5 mPa·s
- Δn=: 0.115
- Δε =: 6.1
- Vₜₕ=: 1.76 V

### Example 27 (Use Example 19)

Liquid crystal composition comprising the following compounds in the content shown below was prepared:

| | |
|---|---|
| 3-H4HB(F,F)B-CF3 | 3.0 % |
| 7-HB(F,F)-F | 4.0 % |
| 3-H2HB(F,F)-F | 9.0 % |
| 4-H2HB(F,F)-F | 10.0 % |
| 5-H2HB(F,F)-F | 10.0 % |
| 3-HHB(F,F)-F | 10.0 % |
| 4-HHB(F,F)-F | 5.0 % |
| 3-HH2B(F,F)-F | 15.0 % |
| 5-HH2B(F,F)-F | 10.0 % |
| 3-HBB(F,F)-F | 12.0 % |
| 5-HBB(F,F)-F | 12.0 % |

Properties of this liquid crystal composition were determined and the results were as follows:
- T_{NI}=: 75.4°C
- η₂₀=: 29.2 mPa·s
- Δn=: 0.087
- Δε =: 8.8
- Vₜₕ=: 1.50 V

### Example 28 (Use Example 20)

Liquid crystal composition comprising the following compounds in the content shown below was prepared:

| | |
|---|---|
| 5-H4HB(F,F)B(F,F)-F | 4.0 % |
| 3-HB-CL | 10.0 % |
| 5-HB-CL | 4.0 % |
| 7-HB-CL | 4.0 % |
| 1O1-HH-5 | 5.0 % |
| 2-HBB(F)-F | 8.0 % |
| 3-HBB(F)-F | 8.0 % |
| 5-HBB(F)-F | 10.0 % |
| 4-HHB-CL | 8.0 % |
| 5-HHB-CL | 8.0 % |
| 3-H2HB(F)-CL | 4.0 % |
| 3-HBB(F,F)-F | 10.0 % |
| 5-H2BB(F,F)-F | 9.0 % |
| 3-HB(F)VB-2 | 4.0 % |
| 3-HB(F)VB-3 | 4.0 % |

Properties of this liquid crystal composition were determined and the results were as follows:
- T_{NI}=: 92.4°C
- η₂₀=: 21.6 mPa·s
- Δn=: 0.129
- Δε =: 5.5
- Vₜₕ=: 2.17 V

### Example 29 (Use Example 21)

Liquid crystal composition comprising the following compounds in the content shown below was prepared:

| | |
|---|---|
| 3-H4HB(F,F)B-CF3 | 2.0 % |
| 3-H4HBB(F,F)-F | 2.0 % |
| 5-H4HB(F,F)B(F,F)-F | 2.0 % |
| 3-H4HB(F,F)B(F)-OCF3 | 2.0 % |
| 3-HHB(F,F)-F | 9.0 % |
| 3-H2HB(F,F)-F | 8.0 % |
| 4-H2HB(F,F)-F | 8.0 % |
| 5-H2HB(F,F)-F | 8.0 % |
| 3-HBB(F,F)-F | 21.0 % |
| 5-HBB(F,F)-F | 12.0 % |
| 3-H2BB(F,F)-F | 10.0 % |
| 5-HHBB(F,F)-F | 3.0 % |
| 3-HH2BB(F,F)-F | 3.0 % |
| 5-HHEBB-F | 2.0 % |
| 1O1-HBBH-4 | 4.0 % |
| 1O1-HBBH-5 | 4.0 % |

Properties of this liquid crystal composition were determined and the results were as follows:
- T_{NI}=: 104.0°C
- η₂₀=: 37.7 mPa·s
- Δn=: 0.118
- Δε =: 9.4
- Vₜₕ=: 1.58 V

### Example 30 (Use Example 22)

Liquid crystal composition comprising the following compounds in the content shown below was prepared:

| | |
|---|---|
| 3-H4HB(F,F)B(F)-OCF3 | 4.0 % |
| 5-HB-F | 12.0 % |
| 6-HB-F | 9.0 % |
| 7-HB-F | 7.0 % |
| 2-HHB-OCF3 | 7.0 % |
| 3-HHB-OCF3 | 7.0 % |
| 4-HHB-OCF3 | 7.0 % |
| 5-HHB-OCF3 | 5.0 % |
| 3-HH2B-OCF3 | 4.0 % |
| 5-HH2B-OCF3 | 4.0 % |
| 3-HHB(F,F)-OCF3 | 5.0 % |
| 3-HBB(F)-F | 10.0 % |
| 5-HBB(F)-F | 10.0 % |
| 3-HH2B(F)-F | 3.0 % |
| 3-HB(F)BH-3 | 3.0 % |
| 5-HBBH-3 | 3.0 % |

Properties of this liquid crystal composition were determined and the results were as follows:
- T_{NI}=: 86.4°C
- η₂₀=: 16.0 mPa·s
- Δn=: 0.093
- Δε =: 4.8
- Vₜₕ=: 2.25 V

### Example 31 (Use Example 23)

Liquid crystal composition comprising the following compounds in the content shown below was prepared:

| | |
|---|---|
| 3-H4HB(F,F)B(F)-OCF3 | 5.0 % |
| 2-HHB(F)-F | 2.0 % |
| 3-HHB(F)-F | 2.0 % |
| 5-HHB(F)-F | 2.0 % |
| 2-HBB(F)-F | 6.0 % |
| 3-HBB(F)-F | 6.0 % |
| 5-HBB(F)-F | 10.0 % |
| 2-H2BB(F)-F | 9.0 % |
| 3-H2BB(F)-F | 9.0 % |
| 3-HBB(F,F)-F | 25.0 % |
| 5-HBB(F,F)-F | 14.0 % |
| 1O1-HBBH-4 | 5.0 % |
| 1O1-HBBH-5 | 5.0 % |

Properties of this liquid crystal composition were determined and the results were as follows:
- T_{NI}=: 99.6°C
- η₂₀=: 37.0 mPa·s
- Δn=: 0.136
- Δε =: 7.5
- Vₜₕ=: 1.82 V

### INDUSTRIAL APPLICABILITY

As discussed above, the compounds of the present invention have a structure of four ring system characterized by having butylene group in the central portion of the molecule; have a high Δε based on the structure; can improve temperature dependency of VHR; and further, are excellent in mutual solubility with other liquid crystalline compounds.

Accordingly, when the compounds of the present invention were used as component of liquid crystal compositions, it is possible to provide novel liquid crystal compositions which have both a low Vₜₕ currently considered to be necessary and a high VHR at temperatures in which the compounds are used, in addition to such a characteristic that the solubility with other liquid crystal materials is excellent even at low temperatures; and further, it is possible to provide novel liquid crystal compositions having desired physical properties by properly selecting six membered rings, substituents, and/or bonding groups of molecule constituting elements.

## Claims

1. A cyclohexane derivative expressed by the general formula (1) 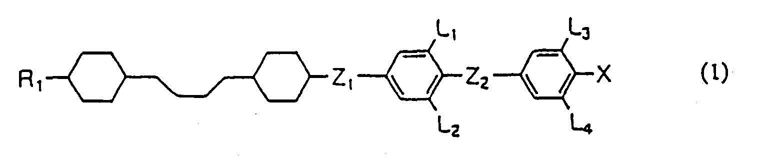 wherein R₁ represents an alkyl group having 1 to 15 carbon atoms, at least one non-adjacent methylene group (-CH₂-) in the alkyl group may be replaced by -O-, -S-, -CH=CH-, or -C≡C-; L₁, L₂, L₃, and L₄ independently represent H or F; X represent a halogen atom, CF₃, OCF₃, OCHF₂, CN, or NCS; and Z₁ and Z₂ independently represent -(CH₂)₂-, -CH=CH-, -COO-, or a covalent bond.

2. The cyclohexane derivative according to claim 1 wherein both L₁ and L₂ are H.

3. The cyclohexane derivative according to claim 1 wherein both L₁ and L₂ are F.

4. The cyclohexane derivative according to claim 2 wherein R₁ is an alkyl group having 1 to 15 carbon atoms.

5. The cyclohexane derivative according to claim 3 wherein R₁ is an alkyl group having 1 to 15 carbon atoms.

6. A liquid crystal composition comprising at least two components and comprising at least one cyclohexane derivative defined in any one of claims 1 to 5.

7. A liquid crystal composition comprising, as a first component, at least one cyclohexane derivative defined in any one of claims 1 to 5, and comprising, as a second component, at least one compound selected from the group consisting of the compounds expressed by any one of the general formulas (2), (3), and (4) 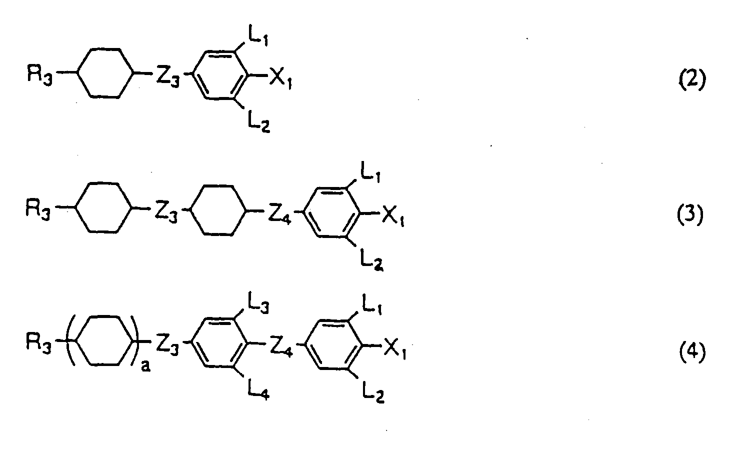 wherein R₃ represents an alkyl group having 1 to 10 carbon atoms; X₁ represents F, Cl, OCF₃, OCF₂H, CF₃, CF₂H, or CFH₂; L₁, L₂, L₃, and L₄ independently represent H or F; Z₃ and Z₄ independently represent -(CH₂)₂-, -CH=CH-, or a covalent bond; and a is 1 or 2.

8. A liquid crystal composition comprising, as a first component, at least one cyclohexane derivative defined in any one of claims 1 to 5, and comprising, as a second component, at least one compound selected from the group consisting of the compounds expressed by any one of the general formulas (5), (6), (7), (8), and (9) 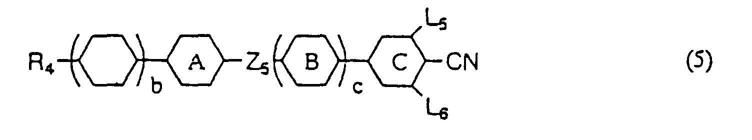 wherein R₄ represents F, an alkyl group having 1 to 10 carbon atoms, or an alkenyl group having 2 to 10 carbon atoms, at least one non-adjacent methylene group (-CH₂-) in the alkyl or alkenyl group may be replaced by oxygen atom (-O-); ring A represents trans-1,4-cyclohexylene group, 1,4-phenylene group, pyrimidine-2,5-diyl group, or 1,3-dioxane-2,5-diyl group; ring B represents trans-1,4-cyclohexylene group, 1,4-phenylene group, or pyrimidine-2,5-diyl group; ring C represents trans-1,4-cyclohexylene group or 1,4-phenylene group; Z₅ represents -(CH₂)₂-, -COO-, or a covalent bond; L₅ and L₆ independently represent H or F; and b and c are independently 0 or 1, 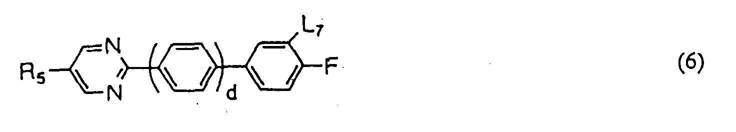 wherein R₅ represents an alkyl group having 1 to 10 carbon atoms; L₇ represents H or F; and d is 0 or 1, 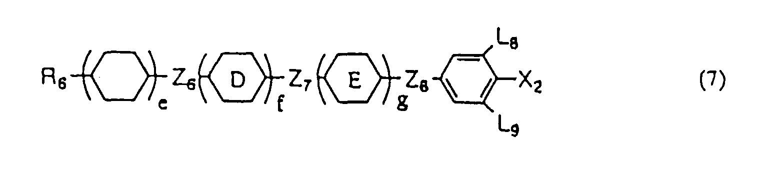 wherein R₆ represents an alkyl group having 1 to 10 carbon atoms; ring D and ring E independently represent trans-1,4-cyclohexylene group or 1,4-phenylene group; Z₆ and Z₇ independently represent -COO- or a covalent bond; Z₈ represents -COO- or -C≡C-; L₈ and L₉ independently represent H or F; X₂ represents F, OCF₃, OCF₂H, CF₃, CF₂H, or CFH₂ provided that when X₂ represents OCF₃, OCF₂H, CF₃, CF₂H, or CFH₂, then both L₈ and L₉ represent H; and e, f, and g are independently 0 or 1, 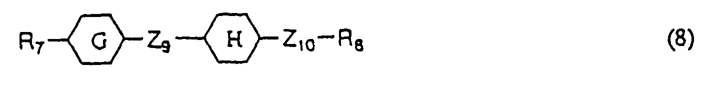 wherein R₇ and R₈ independently represent an alkyl group having 1 to 10 carbon atoms or an alkenyl group having 2 to 10 carbon atoms, at least one non-adjacent methylene group (-CH₂-) in the alkyl or alkenyl group may be replaced by oxygen atom (-O-); ring G represents trans-1,4-cyclohexylene group, 1,4-phenylene group, or pyrimidine-2,5-diyl group; ring H represents trans-1,4-cyclohexylene group or 1,4-phenylene group; Z₉ represents -C≡C-, -COO-, -(CH₂)₂-, -CH=CH-C≡C-, or covalent bond; and Z₁₀ represents -COO- or a covalent bond, 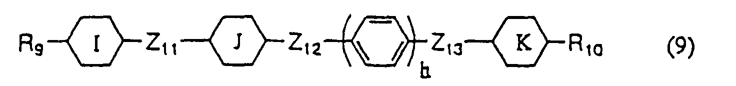 wherein R₉ and R₁₀ independently represent an alkyl group having 1 to 10 carbon atoms or an alkenyl group having 2 to 10 carbon atoms, at least one non-adjacent methylene group (-CH₂-) in the alkyl or alkenyl group may be replaced by oxygen atom (-O-); ring I represents trans-1,4-cyclohexylene group, 1,4-phenylene group, or pyrimidine-2,5-diyl group; ring J represents trans-1,4-cyclohexylene group, 1,4-phenylene group at least one hydrogen atom on which ring may be replaced by F, or pyrimidine-2,5-diyl group; ring K represents trans-1,4-cyclohexylene group or 1,4-phenylene group; Z₁₁ and Z₁₃ independently represent -COO-, -(CH₂)₂-, or a covalent bond; Z₁₂ represents -CH=CH-, -C≡C-, -COO-, or a covalent bond; and h is 0 or 1.

9. A liquid crystal composition comprising, as a first component, at least one cyclohexane derivative defined in any one of claims 1 to 5, comprising, as a part of a second component, at least one compound selected from the group consisting of the compounds expressed by any one of the general formulas (2), (3), and (4), 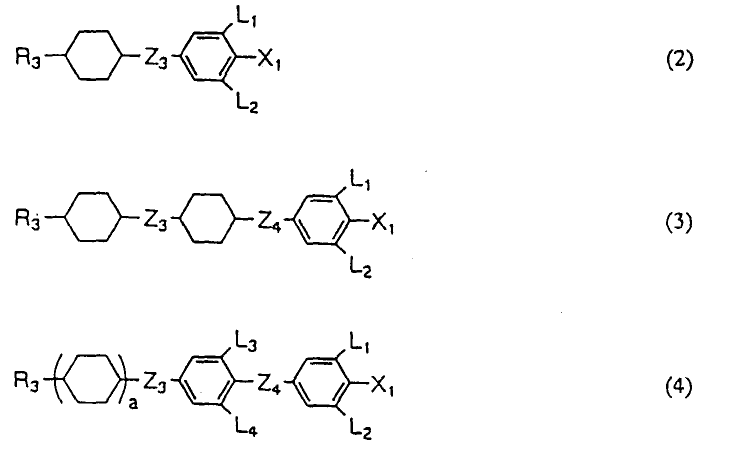 wherein R₃ represents an alkyl group having 1 to 10 carbon atoms; X₁ represents F, Cl, OCF₃, OCF₂H, CF₃, CF₂H, or CFH₂; L₁, L₂, L₃, and L₄ independently represent H or F; Z₃ and Z₄ independently represent -(CH₂)₂-, -CH=CH-, or a covalent bond; and a is 1 or 2, and comprising, as the other part of the second component, at least one compound selected from the group consisting of the compounds expressed by any one of the general formulas (5), (6), (7), (8), and (9) 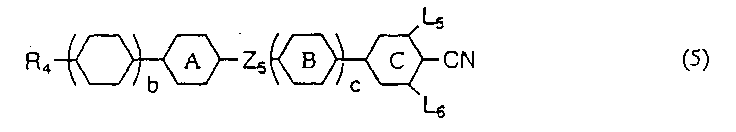 wherein R₄ represents F, an alkyl group having 1 to 10 carbon atoms, or an alkenyl group having 2 to 10 carbon atoms, at least one non-adjacent methylene group (-CH₂-) in the alkyl or alkenyl group may be replaced by oxygen atom (-O-); ring A represents trans-1,4-cyclohexylene group, 1,4-phenylene group, pyrimidine-2,5-diyl group, or 1,3-dioxane-2,5-diyl group; ring B represents trans-1,4-cyclohexylene group, 1,4-phenylene group, or pyrimidine-2,5-diyl group; ring C represents trans-1,4-cyclohexylene group or 1,4-phenylene group; Z₅ represents -(CH₂)₂-, -COO-, or a covalent bond; L₅ and L₆ independently represent H or F; and b and c are independently 0 or 1, 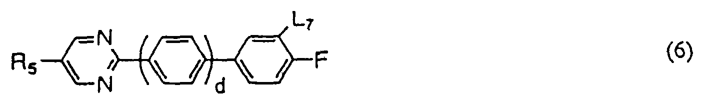 wherein R₅ represents an alkyl group having 1 to 10 carbon atoms; L₇ represents H or F; and d is 0 or 1, 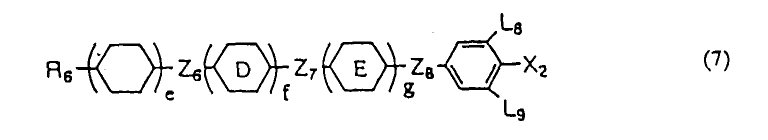 wherein R₆ represents an alkyl group having 1 to 10 carbon atoms; ring D and ring E independently represent trans-1,4-cyclohexylene group or 1,4-phenylene group; Z₆ and Z₇ independently represent -COO- or a covalent bond; Z₈ represents -COO- or -C≡C-; L₈ and L₉ independently represent H or F; X₂ represents F, OCF₃, OCF₂H, CF₃, CF₂H, or CFH₂ provided that when X₂ represents OCF₃, OCF₂H, CF₃, CF₂H, or CFH₂, then both L₈ and L₉ represent H; and e, f, and g are independently 0 or 1, 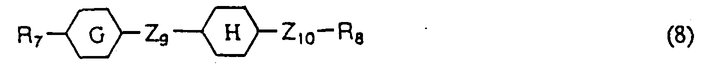 wherein R₇ and R₈ independently represent an alkyl group having 1 to 10 carbon atoms or an alkenyl group having 2 to 10 carbon atoms, at least one non-adjacent methylene group (-CH₂-) in the alkyl or alkenyl group may be replaced by oxygen atom (-O-); ring G represents trans-1,4-cyclohexylene group, 1,4-phenylene group, or pyrimidine-2,5-diyl group; ring H represents trans-1,4-cyclohexylene group or 1,4-phenylene group; Z₉ represents -C≡C-, -COO-, -(CH₂)₂-, -CH=CH-C≡C-, or covalent bond; and Z₁₀ represents -COO- or a covalent bond, 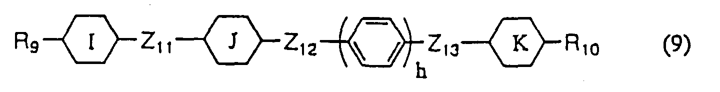 wherein R₉ and R₁₀ independently represent an alkyl group having 1 to 10 carbon atoms or an alkenyl group having 2 to 10 carbon atoms; at least one non-adjacent methylene group (-CH₂-) in the alkyl or alkenyl group may be replaced by oxygen atom (-O-); ring I represents trans-1,4-cyclohexylene group, 1,4-phenylene group, or pyrimidine-2,5-diyl group; ring J represents trans-1,4-cyclohexylene group, 1,4-phenylene group at least one hydrogen atom on which ring may be replaced by F, or pyrimidine-2,5-diyl group; ring K represents trans-1,4-cyclohexylene group or 1,4-phenylene group; Z₁₁ and Z₁₃ independently represent -COO-, -(CH₂)₂-, or a covalent bond; Z₁₂ represents -CH=CH-, -C≡C-, -COO-, or a covalent bond; and h is 0 or 1.

10. A liquid crystal display device comprising a liquid crystal composition defined in claim 6.

11. A liquid crystal display device comprising a liquid crystal composition defined in claim 7.

12. A liquid crystal display device comprising a liquid crystal composition defined in claim 8.

13. A liquid crystal display device comprising a liquid crystal composition defined in claim 9.
